# EUROPEAN PATENT APPLICATION

(11) **EP 3 293 271 A1**
(43) Date of publication of application: **14.03.2018**
(21) Application number: 16188421.8
(22) Date of filing: 12.09.2016
(51) Int. Cl.: C12Q 1/68

(54) **MARKER AND TARGET AS A DIAGNOSTIC VARIABLE AND TARGET FOR THERAPY OF METASTATIC CANCER**

(71) Applicant: The Provost, Fellows, Foundation Scholars, & the other members of Board, of the College of the Holy & Undiv. Trinity of Queen Elizabeth near Dublin, Dublin 2 (IE)
(72) Inventor: O'LEARY, John, Dublin, Dublin 2 (IE); SHEILS, Orla, Dublin, Dublin 2 (IE); SPILLANE, Cathy, Dublin, Dublin 2 (IE); O'TOOLE, Sharon, Dublin, Dublin 2 (IE); CLUXTON, Chris, Dublin, Dublin 2 (IE); GARDINER, Clair, Dublin, Dublin 2 (IE)
(74) Representative: Carmody, Mark

(57) **Abstract**

A method of assessing metastatic potential of a cancer in an individual diagnosed with cancer, the method comprising a step of comparing a level of expression of either CD112 or TIGIT, or both, in a biological sample obtained from the individual to a reference level of CD112 or TIGIT, or both, obtained from a biological sample from an individual who does not have a metastatic cancer, wherein decreased expression levels of either CD112 or TIGIT, or both, when compared to the reference level correlates with increased potential for metastasis of the cancer.

## Description

### Field of the Invention

The invention relates to the novel use of markers CD 112 and TIGIT that have clinical potential as a diagnostic, target and poor-prognostic biomarker for metastatic cancer.

### Background of Invention:

During the metastatic cascade, circulating tumour cells rapidly and efficiently adopt a platelet cloak. Platelet cloaking of tumour cells promotes metastatic disease by promoting cellular proliferation, angiogenesis and epithelial-mesenchymal transition (EMT) while inhibiting autophagy and apoptosis. In addition, the platelet cloak contributes to tumour cell immune evasion by inhibiting Natural Killer (NK) cell-mediated immune surveillance. NK cells are lymphocytes of the innate immune system that monitor cell surfaces of autologous cells for aberrant expression of major histocompatibility complex (MHC) class I molecule and cell stress markers, including MHC class I polypeptide-related sequence A (MICA), MHC class I polypeptide-related sequence B (MICB) and Nectin-2 (CD112).

Immune surveillance by NK cells plays a pivotal role in the elimination of cancer cells from the peripheral circulation in cancer metastasis. NK cells become activated by tumour cells when either the cancer cell lacks the expression of MHC class I molecule, which means inhibitory receptors on the NK cells, such as Killer-cell immunoglobulin-like receptors (KIRs), do not become activated, or when stress ligands expressed on the surface interact with activating NK receptors, such as NKG2D. Once activated NK cells directly kill/target tumour cells through several mechanisms, including release of cytoplasmic granules containing perforin and granzymes leading to the perforation of target cells and subsequent apoptotic death induced by the permeated granzymes. Additionally, upon activation NK cells secret various effector molecules, such as IFNγ, that exert antitumor functions by various methods, including restricting tumour angiogenesis and stimulating adaptive immunity.

Cancer is among the leading causes of morbidity and mortality worldwide, with approximately 14 million new cases and 8.2 million cancer related deaths in 2012. In the next two decades it is estimated that there will be a 70% increase in the incidence of cancer worldwide. In recent years there have been important breakthroughs in cancer research that have had a significant impact on clinical outcome, for example the molecular sub-typing of breast cancers that subsequently has allowed treatment regimens to be tailored to the particular characteristics of the tumour type. However, these advances have primarily related to primary disease and while important, less than 10% of the deaths associated with cancer are related to primary disease and over 90% are associated with metastatic disease. This makes metastasis the deadliest aspect of cancer and the single most significant challenge to the management of the disease.

In order to make significant inroads into the current morbidity and mortality figures associated with cancer novel diagnostic and prognostic markers, as well as treatment/prevention strategies for metastatic disease are urgently required. However, for these new tools to make a significant clinical impact they must be based on an increased understanding of the complex molecular process that is metastasis. Therefore, therapies designed to induce an antitumor response against malignancies by enhancing the immune system is an appealing alternative strategy to control tumour metastasis.

It is an object of the present invention to overcome at least one of the above-mentioned problems.

### Summary of the Invention

It has been shown that platelet cloaking of tumour cells disrupts NK cell immune surveillance of cancer cells by modulation of the NK receptor, NKG2D and its ligands MICA and MICB, and also through the modulation of the NK receptors T cell immunoreceptor with Ig and ITIM domains (TIGIT), Cluster of Differentiation 96 (CD96) and CD226 and their ligands CD155 and CD112. The Applicant has identified markers of cancer cell metastasis (CD112) and TIGIT) that can be detected in a biological sample from the patient, and which function as a both a diagnostic variable and/or a prognostic variable of metastatic cancer (*i.e.* a reduction in the levels of the marker(s) independently correlates with an increased risk of metastasis, and reduced disease free survival, compared with a reference level). The Applicant also provides an additional panel of biomarkers, namely CD96, CD155, CD226, MICA and MICB, one or more of which can be used in conjunction with the biomarkers CD 112 and TIGIT. The Applicant has also discovered that CD226, circulating MICA and circulating MICB are involved in the development and pathology of a metastatic phenotype and that the proteins can therefore be targeted in treatment of a metastatic cancer.

According to the invention, there is provided, as set out in the appended claims, a method of assessing metastatic potential of a cancer, or poor outcome, in an individual diagnosed with cancer, the method comprising a step of comparing a level of expression of either CD112 or TIGIT, or both, in a biological sample obtained from the individual to a reference level of CD 112 or TIGIT, or both, wherein decreased expression levels of either CD 112 or TIGIT, or both, when compared to the reference level correlates with increased potential for metastasis of the cancer and/or poor outcome.

In one embodiment, the method further comprises the step of comparing the expression levels of one or more of CD96, CD155, CD226, circulating MICA and circulating MICB in the biological sample from the individual with cancer to a reference level of CD96, CD155, CD226, circulating MICA and circulating MICB, wherein decreased expression of CD96, CD155 or CD226, or increased expression of circulating MICA or circulating MICB, when compared to the reference level correlates with increased potential for metastasis of the cancer and/or poor outcome for the individual with cancer.

In one embodiment, the biological sample is selected from tumour cells, tumour tissue, conditioned media, blood or blood derivatives, urine, or cerebrospinal fluid.

In one embodiment, there is provided an inhibitor of CD226, circulating MICA or circulating MICB, for use in a method of treatment or prevention of metastatic cancer in a patient, whereby inhibition of CD226 inhibits platelet cloaked cancer cell formation, and inhibition of circulating MICA or circulating MICB increases the ability of NK cells to target cancer cells.

In one embodiment, an inhibitor of CD226, circulating MICA or circulating MICB, for use in a method of treatment or prevention of metastatic cancer in a patient, wherein the inhibitor of CD226, circulating MICA or circulating MICB comprises an antibody or antibody fragment having specific binding affinity to a CD226, circulating MICA or circulating MICB peptide.

In one embodiment, an inhibitor of CD226, circulating MICA or circulating MICB, for use in a method of treatment or prevention of metastatic cancer in a patient, wherein the inhibitor is a nucleic acid capable of inhibiting or reducing the expression of CD226, circulating MICA or circulating MICB.

In one embodiment, an inhibitor of CD226, circulating MICA or circulating MICB, for use in a method of treatment or prevention of metastatic cancer in a patient, wherein the inhibitor is selected from the group consisting of: siRNA; miRNA; ribozyme, anti-sense oligonucleotide.

In one embodiment, an inhibitor of CD226, circulating MICA or circulating MICB, for use in a method of treatment or prevention of metastatic cancer in a patient, wherein the cancer is selected from the list consisting of: breast; non-small cell lung cancer (NSCLC); pancreas; ovarian; colon; kidney; head and neck; stomach; prostate; gliomas; carcinoma; sarcoma; leukaemia; lymphoma; and biologically aggressive forms of uterine cancer.

In one embodiment, an inhibitor of CD226, circulating MICA or circulating MICB, for use in a method of treatment or prevention of metastatic cancer in a patient, wherein the inhibition of CD226, circulating MICA or circulating MICB, or a combination thereof, is administered in combination with a therapeutic drug.

In one embodiment, an inhibitor of CD226, circulating MICA or circulating MICB, for use in a method of treatment or prevention of metastatic cancer in a patient, wherein the inhibitor of CD226, circulating MICA or circulating MICB, or a combination thereof, and the therapeutic drug are administered together or separately.

In one embodiment, an inhibitor of CD226, circulating MICA or circulating MICB, for use in a method of treatment or prevention of metastatic cancer in a patient, wherein the therapeutic drug is selected from the group consisting of: Trastuzumab; Lapatinib; Neratinib; Afatinib; Pertuzumab, Anastrozol, Exemestane, Fulvestrant, Goserelin, Letrozole, Leuprolide, Megestrol acetate, Tamoxifen, Toremifene, Palbociclib, Gemcitabine, Ixabepilone, Liposomal doxorubicin, Methotrexate, Paclitaxel, Vinorelbine, Capecitabine, Carboplatin, Cisplatin, Cyclophosphamide and Docetaxel and variants thereof.

In one embodiment, there is provided an inhibitor of CD226, circulating MICA or circulating MICB, or a combination thereof, for use as a medicament.

In one embodiment, there is provided a pharmaceutical composition comprising an inhibitor of CD226, circulating MICA or circulating MICB, or a combination thereof, and a pharmaceutically acceptable carrier.

In one embodiment, the pharmaceutical composition further includes a therapeutic drug.

In one embodiment, there is provided a method for the treatment or prevention of a metastatic cancer in a patient, the method comprising a step of comparing a level of expression of either CD 112 or TIGIT, or both, in a biological sample obtained from the individual to a reference level of CD112 or TIGIT, or both, obtained from a biological sample from an individual who does not have a metastatic cancer, wherein decreased expression levels of either CD112 or TIGIT, or both, when compared to the reference level indicates that the patient is suitable for treatment with a therapeutically effective amount of an inhibitor of CD226, circulating MICA or circulating MICB, or a combination thereof.

In one embodiment, the method further comprises the step of comparing the expression levels of one or more of CD96, CD155, CD226, circulating MICA and circulating MICB in the biological sample from the patient to a reference level of CD96, CD155, CD226, circulating MICA and circulating MICB, obtained from a biological sample from an individual who does not have a metastatic cancer, wherein decreased expression of CD96, CD155 or CD226, or increased expression of circulating MICA or circulating MICB, when compared to the reference level indicates that the patient is suitable for treatment with a therapeutically effective amount of an inhibitor of CD226, circulating MICA or circulating MICB, or a combination thereof.

The invention provides a method of assessing the metastatic potential of a patient diagnosed with cancer, the method comprising the step of comparing a level of CD112, TIGIT, circulating MICA or circulating MICB, or a combination thereof, in a biological sample obtained from the patient with a reference level of CD 112, TIGIT, circulating MICA or circulating MICB, or a combination thereof, wherein detection of a decreased level of CD 112 or TIGIT, or both, or an increased level of circulating MICA or MICB, or both, when compared to a reference level, indicates that the cancer has greater potential for metastasis than a cancer that does not have a decreased level of CD 112 and/or TIGIT, or an increased level of circulating MICA and/or circulating MICB.

The invention also provides a method of prediction of poor outcome in a patient having a cancer, the method comprising the step of comparing a level of CD112, TIGIT, circulating MICA or circulating MICB, or a combination thereof, in a biological sample obtained from the patient with a reference level of CD 112, TIGIT, circulating MICA or circulating MICB, or a combination thereof, wherein detection of a decreased level of CD112 or TIGIT, or both, or an increased level of circulating MICA or MICB, or both, when compared to a reference level, indicates a poor outcome for the patient.

The invention also provides a method for the treatment or prevention of a cancer in a patient comprising a step of administering to the patient a therapeutically effective amount of an inhibitor of CD226, MICA or MICB, or a combination thereof. In one embodiment, the patient is identified as having a decreased level of CD 112 or TIGIT, or both, and/or an increased level of circulating MICA or MICB, or both. Thus, in one embodiment, the method of the invention involves first identifying the levels of CD 112, TIGIT, circulating MICA or circulating MICB, or a combination thereof, of a patient with a cancer, and wherein the patient is identified as having a decreased level of CD 112 or TIGIT, or both, or an increased level of circulating MICA or MICB, or both, compared to a reference level, then treating the patient with an inhibitor of CD226, MICA or MICB, or a combination thereof.

The invention also provides a method for the treatment or prevention of a cancer in a patient comprising a step of administering to the patient a therapeutically effective amount of an inhibitor of CD226, MICA or MICB, or a combination thereof, and a therapeutic agent. In one embodiment, the patient is identified as having a decreased level of CD 112 or TIGIT, or both, and/or an increased level of circulating MICA or MICB, or both MICA and MICB.

The invention also provides a method of detecting platelet-cloaking of cancer cells, the method comprising the step of comparing a level of one or more of CD96, CD 112, TIGIT, circulating MICA or circulating MICB, in a biological sample with a reference level of CD96, CD 112, TIGIT, circulating MICA or circulating MICB, wherein detection of a level of CD96, CD 112 and/or TIGIT that is decreased compared to the reference level of CD96, CD112 and/or TIGIT wherein no platelet-cloaking is present, or an increased expression of circulating MICA and/or MICB compared to the reference level of circulating MICA and/or circulating MICB wherein no platelet-cloaking is present, indicates that platelet-cloaking of cancer cells is present.

The invention also provides a method of monitoring platelet-cloaking of cancer cells, the method comprising the step of comparing a level of one or more of CD96, CD 112, TIGIT, circulating MICA or circulating MICB, in a biological sample with a reference level of CD96, CD 112, TIGIT, circulating MICA or circulating MICB, wherein detection of a level of CD96, CD112 and/or TIGIT that is decreased compared to the reference level of CD96, CD 112 and/or TIGIT wherein no platelet-cloaking is present, or an increased expression of circulating MICA and/or MICB compared to the reference level of circulating MICA and/or circulating MICB wherein no platelet-cloaking is present, indicates that platelet-cloaking of cancer cells is occurring or has occurred.

Specifically, the method of detecting platelet-cloaking of cancer cells comprises the step of comparing a level of CD96 in a biological sample with a reference level of CD96, wherein detection of a level of CD96 that is decreased compared to the reference level of CD96 wherein no platelet-cloaking is present, indicates that platelet-cloaking of cancer cells is present.

Specifically, the method of monitoring platelet-cloaking of cancer cells comprises the step of comparing a level of CD96 in a biological sample with a reference level of CD96, wherein detection of a level of CD96 that is decreased compared to the reference level of CD96 wherein no platelet-cloaking is present, indicates that platelet-cloaking of cancer cells is occurring or has occurred.

The invention also provides a method of identifying the responsiveness of a metastatic cancer in a patient to cancer treatment, the method comprising the step of comparing a level of one or more of CD 112, TIGIT, circulating MICA or circulating MICB, in a biological sample obtained from the patient with a reference level of CD 112, TIGIT, circulating MICA or circulating MICB obtained from a patient without metastatic cancer, wherein detection of a decreased level of CD112 and/or TIGIT, or an increased expression of circulating MICA and/or circulating MICB when compared to the reference level of CD 112, TIGIT, circulating MICA and/or circulating MICB , indicates that cancer has reduced responsiveness to the cancer treatment. Typically, the cancer treatment is a drug selected from Trastuzumab, Lapatinib, Neratinib, Afatinib, Pertuzumab, Anastrozol, Exemestane, Fulvestrant, Goserelin, Letrozole, Leuprolide, Megestrol acetate, Tamoxifen, Toremifene, Palbociclib, Gemcitabine, Ixabepilone, Liposomal doxorubicin, Methotrexate, Paclitaxel, Vinorelbine, Capecitabine, Carboplatin, Cisplatin, Cyclophosphamide and Docetaxel or an inhibitor of one or more of CD226, MICA and MICB.

The invention also provides an inhibitor for each of CD226, MICA and MICB for use as a medicament.

The invention also provides an inhibitor of CD226, MICA and MICB for use in a method for the treatment or prevention of a metastatic cancer in a patient.

The invention also provides a pharmaceutical composition comprising an inhibitor of CD226, MICA or MICB, or a combination thereof and a pharmaceutically acceptable carrier.

The invention also provides a pharmaceutical composition comprising an inhibitor of CD226, MICA or MICB, or a combination thereof, and a therapeutic drug, and a pharmaceutically acceptable carrier.

In one embodiment, the methods as described above whereby the decreased levels of a protein selected from the group comprising CD96, CD112, CD155, CD226 and TIGIT, or combinations thereof, and or increased levels of circulating MICA and/or MICB, are directly correlated to poor responsiveness to cancer targeting agents.

In one embodiment, the methods as described above whereby the decreased levels of a protein selected from the group comprising CD96, CD112, CD155, CD226 and TIGIT, or combinations thereof, and or increased levels of circulating MICA and/or MICB, are directly correlated to the presence of metastatic cancer.

In one embodiment, the cancer is a cancer type such a breast, bladder, pancreas, NSCLC, ovarian, colon, kidney, head and neck, stomach, prostate, gliomas, epithelial, biologically aggressive forms of uterine cancer, such as uterine serous endometrial carcinoma.

It is an objection of the present invention to provide a biomarker panel for the prediction of cancer metastasis in an individual with cancer. Biomarkers could, at least in part, help to overcome the problem of patients receiving certain therapeutic agents from which they derive no benefit, and also meet the increasing demand for improved patient outcomes. A further objective of the invention is to provide a target for CD226, MICA and/or MICB as a useful therapeutic strategy for treatment of cancer.

### Brief Description of Drawings

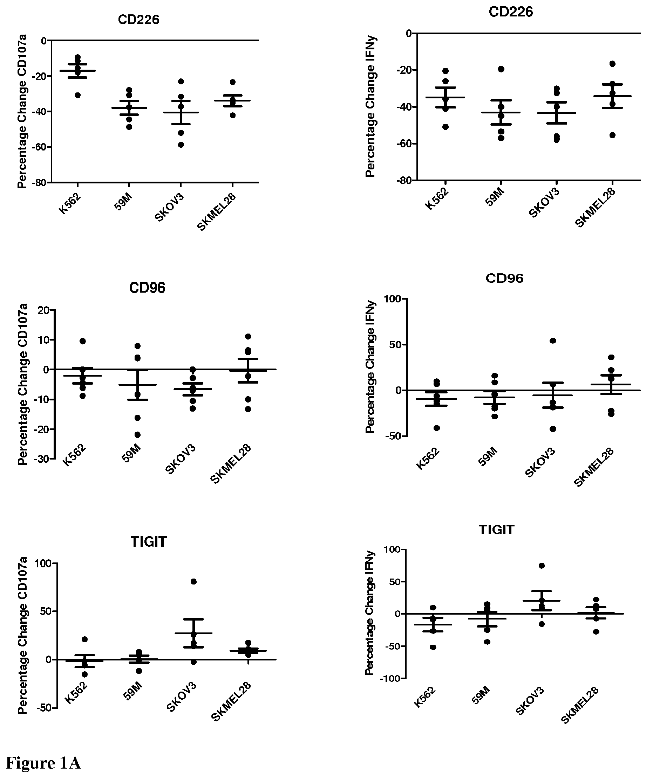

The invention will be more clearly understood from the following description of an embodiment thereof, given by way of example only, with reference to the accompanying drawings, in which:-
**Figures 1A-D** illustrate the percentage change in the ability of NK cells to recognize and kill cancer cells, specifically ovarian (59M and SKOV3), melanoma (Sk-Mel-28) and CML (K562) cell lines, when either CD226, CD96, or Killer cell lectin-like receptor subfamily K, member 1 (NKG2D) on NK cells are blocked or alternative CD155, CD112, MICA or MICB are blocked on the cancer cells. Peripheral blood mononuclear cells (PBMCs) were stimulated with Interleukin-2 (IL-2) and co-incubated with ovarian (59M and SKOV3), melanoma (Sk-Mel-28) and CML (K562) cell lines in the presence of either a blocking antibody towards CD226 (BioLegend, cat#: 338312, clone: 11A8), CD96 (BioLegend, cat#: 338302, clone: NK92.39), CD155 (BioLegend, cat#: 337602, clone: SKII.4), CD112 (BioLegend, cat#: 337402, clone: TX31), NKG2D (R&D, cat#: MAB139, clone: 149810), MICA (R&D, cat#: MAB1300, clone: 159227) MICB (R&D, cat#:MAB1599, clone: 236511). NK cell mediated recognition and 'killing' of cancer cells was quantified by flow cytometry, using both an anti-CD107a specific antibody (BD, cat#: 560664, clone:H4A3) to detect the expression of CD107a on the NK cell surface and an anti-IFNγ specific antibody (BD, cat#: 655933, clone: B27) to detect the release of IFNγ from NK cells. All results represent the mean of n=3 biological replicates ±SEM.

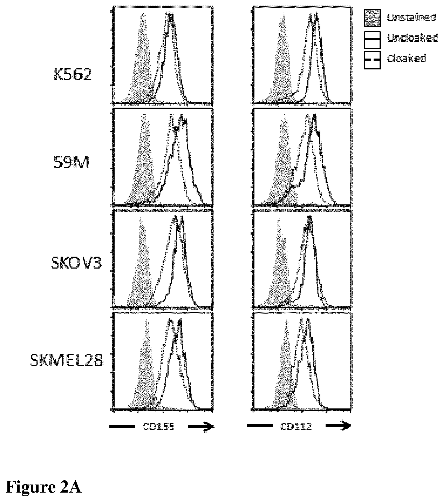

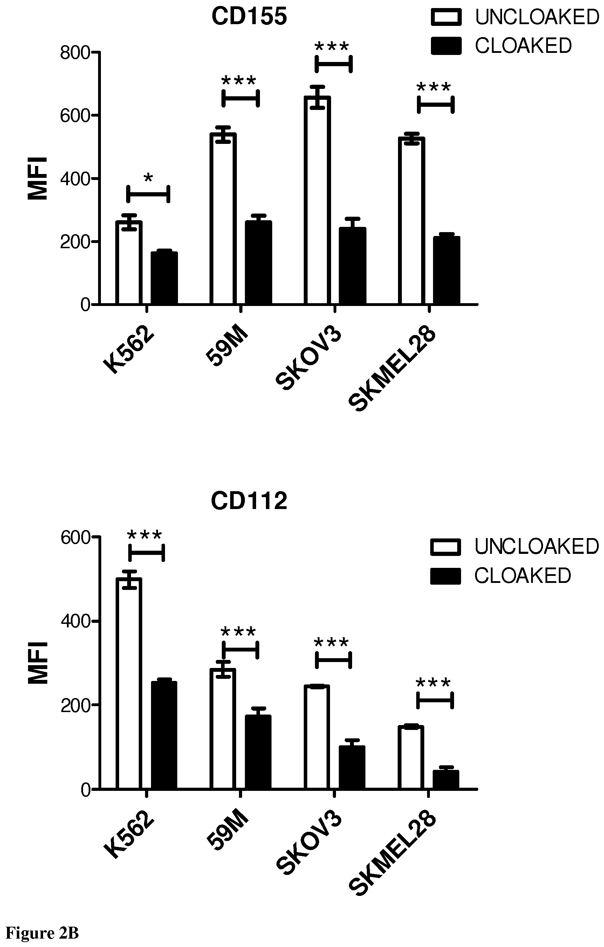

**Figure 2A** and **2B** illustrate graphs showing that platelet cloaking of cancer cells down-regulates cancer cell ligands, CD155 and CD112. Washed platelets were isolated from the whole blood of healthy donors. Ovarian (59M and SKOV3), melanoma (Sk-Mel-28) and CML (K562) cell lines were either left uncloaked or cloaked with washed platelets, at a cancer cell-platelet ratio of 1:1000. The expression of CD155 and CD112 on the cancer cells was quantified by flow cytometry, using anti-CD155 (BioLegend, cat#: 337614, clone: SKII.4) and anti-CD112 (BioLegend, cat#: 337412, clone: TX31) specific antibodies respectively. Results represent the mean of n=3 biological replicates ±SEM. P-values were generated using two-way ANOVA with Bonferroni post-tests, where *=p<0.05, **=p<0.01, ***=p<0.001.

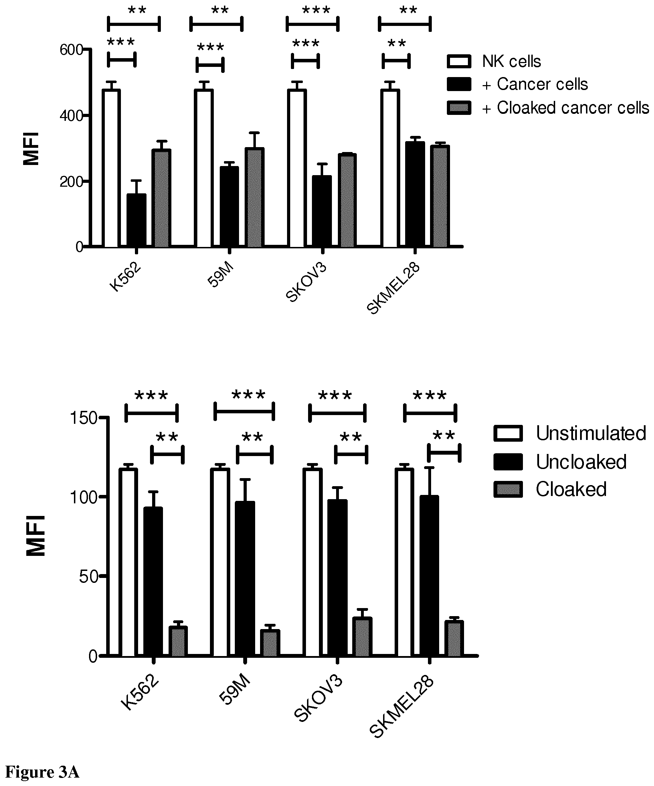

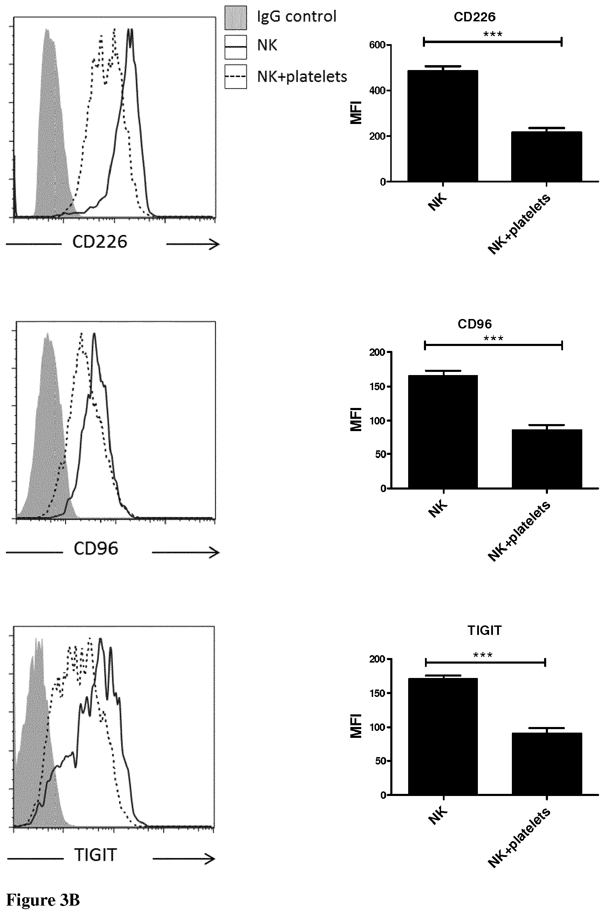

**Figure 3A** shows two graphs, the left-hand graph illustrates that cancer cells and platelet-cloaked cancer cells down-regulate the NK cell receptor CD226. The right-hand graph illustrates that only platelet-cloaked cancers, and not cancer cells alone, have the ability to down-regulate the NK cell receptor CD96. PBMCs were harvested from healthy donors and stimulated for 18 hours with IL-2. PBMCs were co-incubated with ovarian (59M and SKOV3), melanoma (Sk-Mel-28) and CML (K562) cell lines that were either uncloaked or cloaked with washed platelets from healthy donors. The expression of CD226 and CD96 on NK cells post incubation was quantified by flow cytometry, using anti-CD226 (BioLegend, cat#: 338312, clone: 11A8) and anti-CD96 (eBioscience, cat#: 12-0969-42, clone: NK92.39) specific antibodies respectively. **Figure 3B** shows three graphs illustrating that platelets down-regulate NK cell receptors, CD226, CD96 and TIGIT. PBMCs were harvested from healthy donors and stimulated for 18 hours with IL-2. Washed platelets were also isolated from the whole blood of healthy donors. PBMCs were co-incubated with platelets and the expression of CD226, CD96 and TIGIT on NK cells post incubation was quantified by flow cytometry, using anti-CD226 (BioLegend, cat#: 338312, clone: 11A8), anti-CD96 (eBioscience, cat#: 12-0969-42, clone: NK92.39) and anti-TIGIT (eBioscience, cat#: 25-9500-42, clone: MBSA43) specific antibodies respectively. Results represent the mean of n=3 biological replicates ±SEM. P-values for Figure 3A were generated using two-way ANOVA with Bonferroni post-tests and p-values for Figure 3B were generated using Student's t-test, where **=p<0.01, ***=p<0.001.

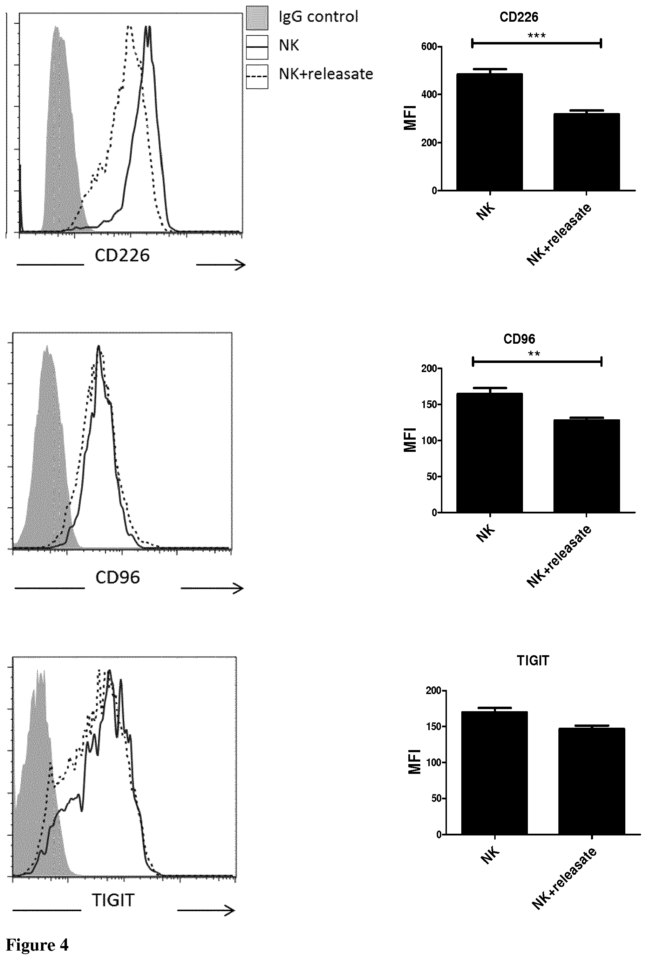

**Figure 4** is a series of graphs illustrating that platelet releasate down-regulates NK cell receptors, CD226, CD96 and TIGIT. PBMCs were harvested from healthy donors and stimulated for 18 hours with IL-2. Washed platelets were also isolated from the whole blood of healthy donors and activated by stimulation with thrombin receptor activating peptide (VWR, cat#: GENSRP10623-1). The platelets were next centrifuged and the platelet releasate (supernatant) removed. PBMCs were co-incubated with the platelet releasate from the activated platelets and the expression of CD226, CD96 and TIGIT on NK cells post incubation was quantified by flow cytometry, using anti-CD226 (BioLegend, cat#: 338312, clone: 11A8), anti-CD96 (eBioscience, cat#: 12-0969-42, clone: NK92.39) and anti-TIGIT (eBioscience, cat#: 25-9500-42, clone: MBSA43) specific antibodies respectivelyResults represent the mean of n=3 biological replicates ±SEM. P-values were generated using Student's t-test, where **=p<0.01, ***=p<0.001.

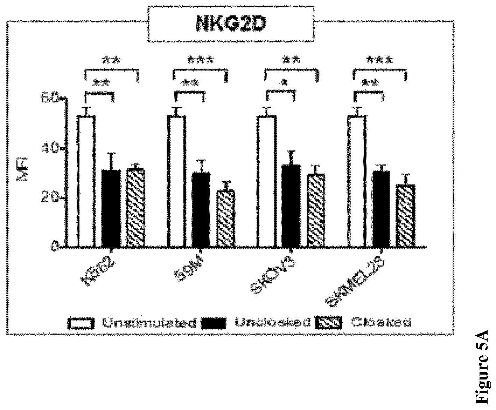

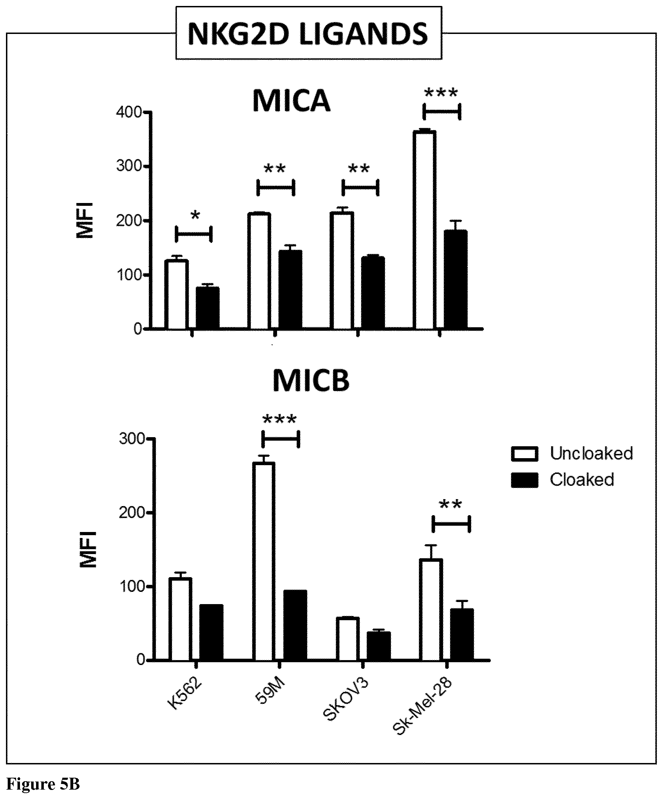

**Figure 5A** illustrates that cancer cells and to a greater degree platelet-cloaking of cancer cells reduces NKG2D on NK cells. **Figure 5B** illustrates that platelet cloaking reduces the level of the NKG2D ligands MHC class I polypeptide-related sequence A (MICA) and MHC class I polypeptide-related sequence B (MICB) on cancer cells. PBMCs were harvested from healthy donors and stimulated for 18 hours with IL-2. PBMCs were either left untreated or co-incubated with ovarian (59M and SKOV3), melanoma (Sk-Mel-28) and CML (K562) cell lines that were either uncloaked or cloaked with washed platelets from healthy donors. The expression of NKG2D on NK cells post incubation was quantified by flow cytometry, using anti-NKG2D (BioLegend, cat#: 32080, clone: IDII) specific antibodies, respectively. The expression of MICA and MICB on cancer cells post incubation was quantified by flow cytometry, using anti-MICA (R&D, cat#: FAB1300A, clone: 159227) or anti-MICB (R&D, cat#: FAB1599A, clone: 236511) specific antibodies, respectively. Results represent the mean of n=3 biological replicates ±SEM. P-values were generated using two-way ANOVA with Bonferroni post-tests, where *=p<0.05, **=p<0.01, ***=p<0.001.

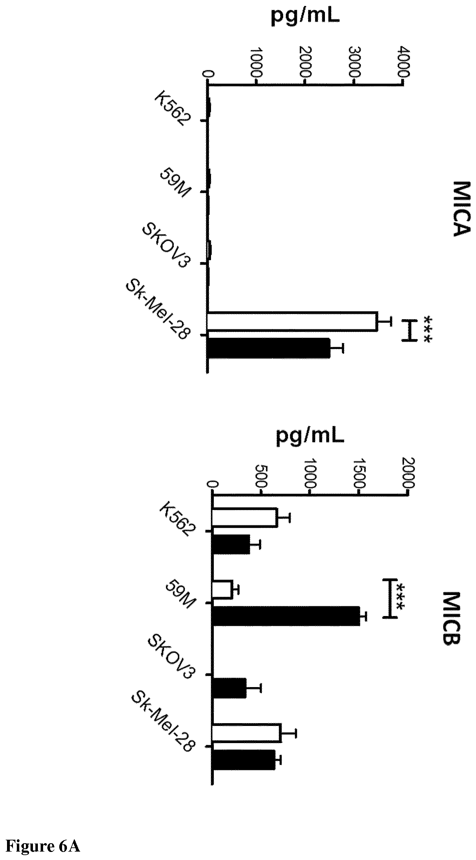

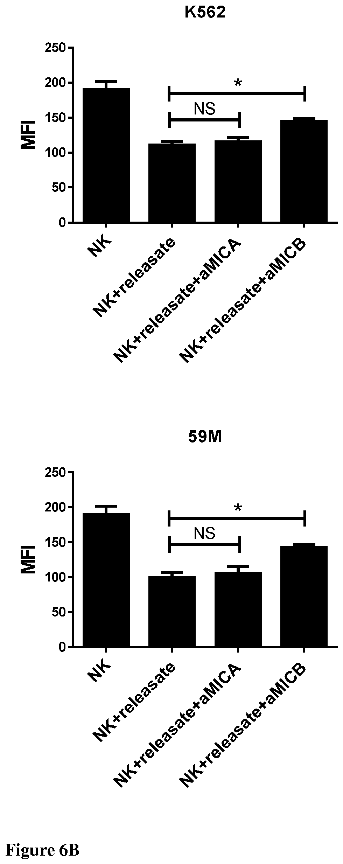

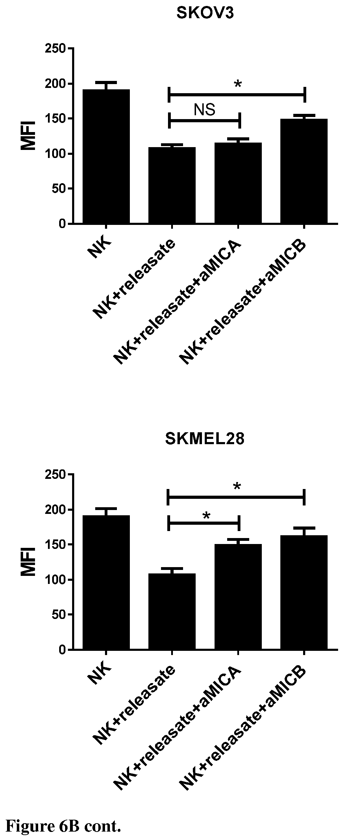

**Figure 6** is a series of graphs illustrating that platelet-cloaking of tumour cells alters the release of soluble MICA and MICB from cancer cells and that soluble MICA and MICB protect cancer cells by down-regulating NKG2D on NK cells. Washed platelets were isolated from healthy donors and co-incubated with ovarian (59M and SKOV3), melanoma (Sk-Mel-28) and CML (K562) cell lines, resulting in platelet cloaking of cancer cells and platelet activation. Next each cancer cell/platelet mix was centrifuged and the platelet releasate (supernatant) was removed. In Figure 6A the expression of MICA and MICB in the platelet releasate was quantified by flow cytometry, using anti-MICA (R&D, cat#: FAB1300A, clone: 159227) or anti-MICB (R&D, cat#: FAB1599A, clone: 236511) specific antibodies respectively. In Figure 6B PBMCs, which were stimulated for 18 hours with IL-2, were either left untreated, co-incubated with platelet releasate in the presence or absence of the MICA specific blocking antibody (R&D, cat#: MAB1300, clone: 159227) or MICB specific blocking antibody (R&D, cat#: MAB1599, clone: 236511). The expression of NKG2D on NK cells post incubation was quantified by flow cytometry, using anti-NKG2D (BioLegend, cat#: 32080, clone: IDII) specific antibodies respectively. Results represent the mean of n=3 biological replicates ±SEM. P-values for Figure 6A were generated using two-way ANOVA with Bonferroni post-tests and for Figure 6B using one-way ANOVA with Tukey's multiple comparison test, where *=p<0.05, **=p<0.01, ***=p<0.001.

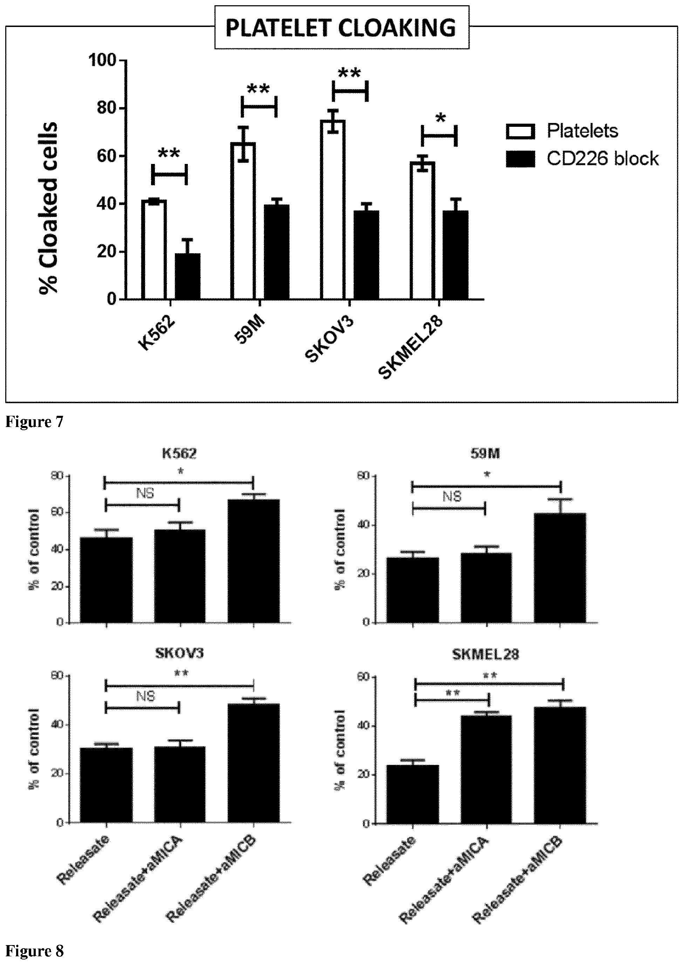

**Figure 7** illustrates that blocking CD226 on platelets reduces platelet cloaking of cancer cells. Washed platelets were isolated from the whole blood of healthy donors and preincubated with a CD226 blocking antibody (BioLegend, cat#: 338302, clone: 11A8). Ovarian (59M and SKOV3), melanoma (Sk-Mel-28) and CML (K562) cell lines were cloaked with washed platelets, at a cancer cell-platelet ratio of 1:1000, in the presence or absence of a CD226 blocking antibody (BioLegend, cat#: 338302, clone: 11A8). Platelet adhesion to cancer cells was measured by flow cytometry. The assay was based on the detection of a platelet specific marker, CD42b (BioLegend, cat#: 303910, clone: HIP1), on the surface of platelets following co-incubation. Results represent the mean of n=3 biological replicates ±SEM. P-values were generated using two-way ANOVA with Bonferroni post-tests, where *=p<0.05, **=p<0.01.
**Figure 8** is a series of graphs illustrating that neutralizing MICA and MICB in platelet releasate from platelet-cloaked cancer cells reverses the inhibition of NK cell function induced by platelet cloaking of cancer cells. PBMCs were harvested from healthy donors and stimulated for 18 hours with IL-2. Washed platelets were isolated from healthy donors and co-incubated with ovarian (59M and SKOV3), melanoma (Sk-Mel-28) and CML (K562) cell lines, resulting in platelet cloaking of cancer cells and platelet activation. Next each cancer cell/platelet mix was centrifuged and the platelet releasate (supernatant) was removed. PBMCs were then co-incubated with each cancer cell line and their corresponding platelet releasate in the presence or absence of the MICA specific blocking antibody (R&D, cat#: MAB1300, clone: 159227) or MICB specific blocking antibody (R&D, cat#: MAB1599, clone: 236511). NK cell mediated recognition and 'killing' of cancer cells was quantified by flow cytometry, using an anti-CD107a specific antibody (BD Pharmigen, cat#: 655933, clone: B27) to detect the expression of CD107a on the NK cell surface. Results represent the mean of n=3 biological replicates ±SEM. P-values were generated using one-way ANOVA with Dunnett's Multiple Comparison Test, where *=p<0.05, **=p<0.01. [

### Detailed Description of the Drawings

### Definitions

In this specification, the term "CD112" or "Poliovirus receptor-related 2 (PVRL2)" or "nectin-2" should be understood to be a human plasma membrane glycoprotein with two Ig-like C2-type domains and an Ig-like V-type domain. The CD112 marker is expressed on the surface of a tumour cell. The nucleotide sequence, the protein sequence and the mRNA sequence encoding the protein are provided in the Annex below:
In this specification, the term "T cell immunoreceptor with Ig and ITIM domains (TIGIT)" should be understood to be a receptor found on the cell surface of NK cells and some T cells that has an affinity for CD112 and CD155. The nucleotide sequence, the protein sequence and the mRNA sequence encoding the protein are provided in the Annex below:
   In this specification, the term "Cluster of Differentiation 155 (CD155)" or "Poliovirus receptor (PVR)" or "Nectin-like protein 5 (Necl-5)" should be understood to be a type I transmembrane glycoprotein in the immunoglobulin superfamily. It is characterised by 3 extracellular Ig-like domains. It is closely related to CD112; with both having identical extracellular domains and differing only over transmembrane and cytoplasmic regions. CD155 is highly expressed in tumour cells of epithelial or neuronal origin. The nucleotide sequence, the protein sequence and the mRNA sequence encoding the protein are provided in the Annex below.

In this specification, the term "Cluster of Differentiation 226 (CD226)" should be understood to be a ∼65 kDa glycoprotein expressed on the surface of natural killer cells, platelets, monocytes and a subset of T cells. It is a member of the immunoglobulin superfamily containing 2 Ig-like domains of the V-set. CD226 mediates cellular adhesion to other cells bearing its ligands, CD112 and CD155. The CD226 marker is expressed on the surface of NK cells. The nucleotide sequence, the protein sequence and the mRNA sequence encoding the protein are provided in the Annex below:
In this specification, the term "Cluster of Differentiation 96 (CD96)" or Tactile (T cell activation, increased late expression) is a T cell-specific receptor and shares sequence similarity with CD226. The protein encoded by this gene belongs to the immunoglobulin superfamily. It is a type I membrane protein. The protein may play a role in the adhesive interactions of activated T and NK cells during the late phase of the immune response. It may also function in antigen presentation. CD96 is a transmembrane glycoprotein that has three extracellular immunoglobulin-like domains and is expressed by NK cells. CD96 main ligand is CD155. The CD96 marker is expressed on the surface of NK cells. The nucleotide sequence, the protein sequence and the mRNA sequence encoding the protein are provided in the Annex below:
   In this specification, the term "MHC class I polypeptide-related sequence A (MICA)" should be understood to be a cell surface glycoprotein related to MHC class I and has similar domain structure, which is made up of external α1α2α3 domain, transmembrane segment and C-terminal cytoplasmic tail. MICA functions as a stress-induced ligand for NKG2D receptor. MICA is broadly recognized by NK cells, γδ T cells, and CD8+ αβ T cells which carry NKG2D receptor on their cell surface. As a result of NKG2D-MICA engagement, effector cytolytic responses of T cells and NK cells against epithelial tumour cells expressing MICA are initiated. Ordinarily, MICA-expressing tumour cells are bound to NK cells via the binding of MICA-NKG2D. When MICA-expressing tumour cells become cloaked with platelets, the NKG2D binds to the platelets and releases MICA from the tumour cell to become a circulating MICA ligand. Hence, this results in a decrease in MICA-expressing tumour cells, but an increase in circulating MICA. The nucleotide sequence, the protein sequence and the mRNA sequence encoding the protein are provided in the Annex below:
      In this specification, the term "MHC class I polypeptide-related sequence B (MICB)" should be understood to be a protein that is related to MHC class I and has similar domain structure, which is made up of external α1α2α3 domain, transmembrane segment and C-terminal cytoplasmic tail. MICB is a stress-induced ligand for NKG2D receptor. The heat shock stress pathway is involved in the regulation of MICB expression as transcription of MICB is regulated by promoter heat shock element. Ordinarily, MICB-expressing tumour cells are bound to NK cells via the binding of MICB-NKG2D. When MICB-expressing tumour cells become cloaked with platelets, the NKG2D binds to the platelets and releases MICB from the tumour cell to become a circulating MICB ligand. Hence, this results in a decrease in MICB-expressing tumour cells, but an increase in circulating MICB. The nucleotide sequence, the protein sequence and the mRNA sequence encoding the protein are provided in the Annex below.

In this specification, the term "NKG2D" should be understood to be a transmembrane protein belonging to the CD94/NKG2 family of C-type lectin-like receptors. In humans, it is expressed by NK cells, γδ T cells and CD8+ αβ T cells. NKG2D recognizes induced-self proteins from MIC and RAET1/ULBP families which appear on the surface of stressed, malignant transformed, and infected cells. The nucleotide sequence, the protein sequence and the mRNA sequence encoding the protein are provided in the Annex below:
The methods of the invention employ a step in which one or more of CD96, CD112, CD155, CD226, TIGIT, MICA and MICB levels in a biological sample from a patient are compared with a reference value/level/abundance. CD96, CD112, CD155, CD226, TIGIT, MICA and MICB levels may be determined at a protein or nucleic acid level. For example, the levels of the protein may be determined using established techniques, or level of the mRNA encoding CD96, CD112, CD155, CD226, TIGIT, MICA and MICB may be determined. Methods for determination are described below, and will be well known to those skilled in the art.

In the specification, the term "platelet cloaking" or "platelet-cloaking" should be understood to mean a potent dynamic interaction between cancer cells and platelets. As circulating tumour cells course through the vascular system they are in constant dynamic interaction with blood cells, including platelets, whereupon circulating tumour cells can become cloaked by platelets. Platelet cloaking occurs maximally under venous shear conditions and is directly influenced by the number of tumour cells, the number of platelets and the activation profile of platelets in the vicinity of the tumour cell. Platelet cloaking of cancer cells is a universal phenomenon occurring across all tumour types, for example, breast, cervix, lung, melanoma, ovary, prostate and thyroid. Not only does the platelet cloaking phenomenon occur but it drives a pro-metastatic phenotype in cancer cells, through the induction of processes associated with EMT, invasion and cellular movement, migration, invasion, adhesion, stem cell plasticity, development, differentiation and inflammation. In addition, the platelet cloak enables the circulating tumour cells to evade natural immune response by disrupting NK cell immune surveillance of cancer cells.

In this specification, the term "biological sample" should be understood to mean tumour cells, tumour tissue, conditioned media, blood or blood derivatives (serum, plasma *etc*), urine, or cerebrospinal fluid.

In this specification, the term "reference level" as applied to CD96, CD112, CD155, CD226, TIGIT, MICA and MICB should be understood to mean a level of CD96, CD112, CD155, CD226, TIGIT, MICA and MICB detected in a patient identified as *not* having a metastatic cancer or any cancer. Typically, the patient is a patient identified as having non-metastatic cancer.

In this specification, the term "poor outcome" should be understood to mean that the chances of disease free survival are low.

In this specification, the term "inhibitor of CD226, MICA or MICB" should be understood to mean an agent that is capable of binding to CD226, MICA or MICB *in vivo*, for example a ligand. The activity may be decreased in a number of different ways which will be apparent to a person skilled in the art, including reducing the expression of the peptide (for example by means of low molecular weight inhibitors such as for example siRNA or shRNA), or by directly inhibiting the activity of the protein by administering a CD226, MICA or MICB inhibitor or an antibody that has specific binding affinity for CD226, MICA or MICB, respectively. In one aspect of the invention, the invention relates to a low molecular weight inhibitor of CD226, MICA or MICB expression, the details of which will be well known to the person skilled in the field of molecular biology, and which include CRISPR knockout sequences, siRNA, shRNA, miRNA, antisense oligonucleotides, and ribozyme molecules. Small inhibitory RNA (siRNA) are small double stranded RNA molecules which induce the degradation of mRNAs. Micro RNA's (miRNAs) are single stranded (∼22nt) non-coding RNAs (ncRNAs) that regulate gene expression at the level of translation. Alternatively, small hairpin RNA (shRNA) molecules are short RNA molecules having a small hairpin loop in their tertiary structure that may be employed to silence genes. The design of miRNA or shRNA molecules capable of silencing CD226, MICA or MICB will be apparent to those skilled in the field of miRNA or shRNA molecule design. As an alternative, the level of tumour CD226, MICA or MICB expression can be modulated using antisense or ribozyme approaches to inhibit or prevent translation of CD226, MICA or MICB mRNA transcripts or triple helix approaches to individually inhibit transcription of the CD226, MICA or MICB genes. Antisense approaches involve the design of oligonucleotides (either DNA or RNA) that are complementary to mRNA of each of CD226, MICA or MICB. The antisense oligonucleotides will bind to the complementary mRNA transcripts and prevent translation. Ribozyme molecules designed to catalytically cleave mRNA transcripts can also be used to prevent translation and expression of CD226, MICA or MICB.

In the specification, the term "Low molecular weight inhibitor" means an inhibitor that has a molecular of less than 50 KDa. In one embodiment, the low molecular weight inhibitor has a molecular weight of less than 20 KDa. In one embodiment, the low molecular weight inhibitor has a molecular weight of less than 10 KDa. In one embodiment, the low molecular weight inhibitor has a molecular weight of less than 5 KDa. In one embodiment, the low molecular weight inhibitor has a molecular weight of less than 4 KDa. In one embodiment, the low molecular weight inhibitor has a molecular weight of less than 3 KDa. In one embodiment, the low molecular weight inhibitor has a molecular weight of less than 2 KDa. In one embodiment, the low molecular weight inhibitor has a molecular weight of less than 1 KDa.

Examples of shRNA molecules for CD226 are commercially available, such as from SIGMA-ALDRICH®:
SEQ ID NO: 67 (3'-CCGGGCTTCCAATAACATGACTCTTCTCGAGAAGAGTCATGTTATTGGAAGCTTTTTG-5');
SEQ ID NO: 68 (3'- CCGGCCGGTCAACCTACCAATCAATCTCGAGATTGATTGGTAGGTTGACCGGTTTTTG -5'); 
SEQ ID NO: 69 (3' – CCGGCCCAAGACAAATAGTGAGCAACTCGAGTTGCTCACTATTTGTCTTGGGTTTTTG
5');
SEQ ID NO: 70 (3' - CCGGGCCGAGAACATGTCTCTAGAACTCGAGTTCTAGAGACATGTTCTCGGCTTTTTG -5');

SEQ ID NO: 71 (3' – CCGGCGCAGACCAAAGACTAGAGTTCTCGAGAACTCTAGTCTTTGGTCTGCGTTTTTG –
5');
SEQ ID NO: 72 (3' – CCGGATGATACAAGAGAGGATATTTCTCGAGAAATATCCTCTCTTGTATCATTTTTTG
5');
SEQ ID NO: 73 (3 – CCGGGCTTTGGGCAAGGGCTATTTACTCGAGTAAATAGCCCTTGCCCAAAGCTTTTTG -
5');
SEQ ID NO: 74 (3'–CCGGGTACTCATGCATGGATCTTTACTCGAGTAAAGATCCATGCATGAGTACTTTTTG –
5');
SEQ ID NO: 75 (3'–CCGGATCCATCAATGGGCATCTTAACTCGAGTTAAGATGCCCATTGATGGATTTTTTG –
5').

Examples of shRNA molecules for MICA are commercially available, such as from GE Healthcare Dharmacon Inc.
Clone ID: TRCN0000061288 (3' - ATCCAGGCAGGGAATTGAATC - 5') SEQ ID 76
Clone ID: TRCN0000061289 (3' - TATTCCAGGGATAGAAGCCAG - 5') SEQ ID 77
Clone ID: TRCN0000061290 (3' - TTTCTGGTCCTTGATATGAGC - 5') SEQ ID 78
Clone ID: TRCN0000061291 (3' - TTCTTACAACAACGGACATAG - 5') SEQ ID 79
Clone ID: TRCN0000061292 (3' - ATTTCCCAGGACATCTTCTGC - 5') SEQ ID 80

Examples of shRNA molecules for MICB are commercially available, such as from:
Clone ID: NM_005931.2-484s1c1 SEQ ID 81 (3' - CCGGCCTTGGCTATGAACGTCACAACTCGAGTTGTGACGTTCATAGCCAAGGTTTTTG - 5')
Clone ID: NM_005931.2-915s1c1 SEQ ID 82 (3' - CCGGCAGAGTCAACGGACAGACTTTCTCGAGAAAGTCTGTCCGTTGACTCTGTTTTTG - 5')
Clone ID: NM_005931.2-983s1c1 SEQ ID 83 (3' - CCGGCTGTGTCCCTTGTTGCAAGAACTCGAGTTCTTGCAACAAGGGACACAGTTTTTG - 5')
Clone ID: NM_005931.2-924s1c1 SEQ ID 84 (3' - CCGGCGGACAGACTTTCCATATGTTCTCGAGAACATATGGAAAGTCTGTCCGTTTTTG - 5')
Clone ID: NM_005931.2-261s1c1 SEQ ID 85 (3' - CCGGACCGAGGACTTGACAGAGAATCTCGAGATTCTCTGTCAAGTCCTCGGTTTTTTG - 5')
Clone ID: NM_005931.3-1094s21c1 SEQ ID 86 (3' - CCGGCTGTGTCCCTTGTTGCAAGAACTCGAGTTCTTGCAACAAGGGACACAGTTTTTG - 5')
Clone ID: NM_005931.3-681s21c1 SEQ ID 87 (3' - CCGGCAGAAACTACAGCGATATCTGCTCGAGCAGATATCGCTGTAGTTTCTGTTTTTG - 5')
Clone ID: NM_005931.3-1692s21c1 SEQ ID 88 (3' - CCGGGCTATCTCCTATACCAATAAACTCGAGTTTATTGGTATAGGAGATAGCTTTTTG - 5')
Clone ID:NM_005931.3-1035s21c1 SEQ ID 89 (3' - CCGGCGGACAGACTTTCCATATGTTCTCGAGAACATATGGAAAGTCTGTCCGTTTTTG - 5')
Clone ID: NM_005931.3-1197s21c1 SEQ ID 90 (3' - CCGGGATGCAGCACAGCTGGGATTTCTCGAGAAATCCCAGCTGTGCTGCATCTTTTTG - 5')

In a preferred embodiment of the invention, the inhibitor is an antagonist of each of CD226, MICA or MICB. One example of an antagonist is an anti-CD226, anti-MICA or anti-MICB antibody (*i.e*. an antibody which specifically binds to human CD226, MICA or MICB peptide). Examples of such antibodies are provided in Table 1 and 2:

**Table 1: Blocking antibodies**

| **Blocking antibodies** | **Clone** | **Reactivity** | **Host** | **Company** | **Cat #** |
|---|---|---|---|---|---|
| **anti-CD226** | 11A8 | Human | Mouse | BioLegend | 338302 |
| **anti-TGFb** | IDII | Human | Mouse | R&D | MAB1835 |
| **anti-NKG2D** | 149810 | Human | Mouse | R&D | MAB139 |
| **anti-MICA** | 159227 | Human | Mouse | R&D | MAB1300 |
| **anti-MICB** | 236511 | Human | Mouse | R&D | MAB1599 |
| **anti-CD111** | R1.302 | Human | Mouse | BioLegend | 340402 |
| **anti**-**CD155** | SKII.4 | Human | Mouse | BioLegend | 337602 |
| **anti-TIGIT** | MBSA43 | Human | Mouse | eBioscience | 16-9500-85 |
| **ant-CD112** | TX31 | Human | Mouse | BioLegend | 337402 |
| **anti-CD96** | NK92.39 | Human | Mouse | BioLegend | 338404 |

**Table 2: Detection Antibodies**

| **Detection antibodies** | **Clone** | **Reactivity** | **Host** | **Company** | **Cat #** |
|---|---|---|---|---|---|
| **anti-CD42b** | HIP1 | Human | Mouse | BioLegend | 303910 |
| **anti-CD3** | UCHT1 | Human | Mouse | BD Pharmigen | 560365 |
| **anti-CD56** | B159 | Human | Mouse | BD Pharmigen | 555517 |
| **anti-CD107a** | H4A3 | Human | Mouse | BD Pharmigen | 560664 |
| **anti-IFNγ** | B27 | Human | Mouse | BD Pharmigen | 557643 |
| **anti-CD112** | TX31 | Human | Mouse | BioLegend | 337412 |
| **anti**-**CD155** | SKII.4 | Human | Mouse | BioLegend | 337614 |
| **anti-CD111** | R1.302 | Human | Mouse | BioLegend | 340404 |
| **anti-CD226** | 11A8 | Human | Mouse | BioLegend | 338312 |
| **anti-TIGIT** | MBSA43 | Human | Mouse | eBioscience | 25-9500-42 |
| **anti-CD96** | NK92.39 | Human | Mouse | eBioscience | 12-0969-42 |
| **anti-MICA** | 159227 | Human | Mouse | R&D | FAB1300A |
| **anti-MICB** | 236511 | Human | Mouse | R&D | FAB1599A |

Antibodies specific for each of CD96, CD112, CD155, CD226, TIGIT, MICA and MICB may be produced using methods which are generally known in the art. In particular, purified CD96, CD112, CD155, CD226, TIGIT, MICA and MICB may be used, respectively, to produce antibodies or to screen libraries of pharmaceutical agents to identify those which specifically bind to CD96, CD112, CD155, CD226, TIGIT, MICA and MICB, respectively. Antibodies to each of CD96, CD112, CD155, CD226, TIGIT, MICA and MICB may also be generated using methods that are well known in the art. Such antibodies may include, but are not limited to, polyclonal, monoclonal, chimeric, and single chain antibodies, Fab fragments, and fragments produced by a Fab expression library. Neutralizing antibodies (*i.e.*, those which inhibit dimer formation) are generally preferred for therapeutic use. Single chain antibodies (*e.g.*, from camels or llamas) may be potent enzyme inhibitors and may have advantages in the design of peptide mimetics, and in the development of immuno-adsorbents and biosensors (Muyldermans, S. (2001) J. Biotechnol. 74:277-302). For the production of antibodies, various hosts including goats, rabbits, rats, mice, camels, dromedaries, llamas, humans, and others may be immunized by injection with CD96, CD112, CD155, CD226, TIGIT, MICA and MICB or with any fragment or oligopeptide thereof which has immunogenic properties. Depending on the host species, various adjuvants may be used to increase immunological response. Such adjuvants include, but are not limited to, Freund's, mineral gels such as aluminum hydroxide, and surface active substances such as lysolecithin, pluronic polyols, polyanions, peptides, oil emulsions, KLH, and dinitrophenol.

It is preferred that the oligopeptides, peptides, or fragments used to induce antibodies to CD96, CD112, CD155, CD226, TIGIT, MICA and MICB, respectively, have an amino acid sequence consisting of at least about 5 amino acids, and generally will consist of at least about 10 amino acids. It is also preferable that these oligopeptides, peptides, or fragments are identical to a portion of the amino acid sequence of the natural protein. Short stretches of CD96, CD112, CD155, CD226, TIGIT, MICA and MICB amino acids may be fused with those of another protein, such as KLH, and antibodies to the chimeric molecule may be produced. Monoclonal antibodies to CD96, CD112, CD155, CD226, TIGIT, MICA and MICB, respectively, may be prepared using any technique which provides for the production of antibody molecules by continuous cell lines in culture. These include, but are not limited to, the hybridoma technique, the human B-cell hybridoma technique, and the EBV-hybridoma technique. (See, *e.g.*, Kohler, G. et al. (1975) Nature 256:495-497; Kozbor, D. et al. (1985) J. Immunol. Methods 81:31-42; Cote, R. J. et al. (1983) Proc. Natl. Acad. Sci. USA 80:2026-2030; and Cole, S.P. et al. (1984) Mol. Cell. Biol. 62:109-120.)

Antibodies are a major class of biopharmaceuticals. Antibodies for therapeutic purposes are often produced from a cell line (e.g. CHO cells, the hamster line BHK21, the human PER.C6 cell line, COS, NIH 3T3, BHK, HEK, 293, L929, MEL, JEG-3, murine lymphoid cells (including NS0 and Sp2/0-Ag 14)), including hybridomas, and are usually a single clone of a specific antibody. Antibodies used for therapeutic purposes are classified as murine, chimeric, humanized or fully human antibodies and are produced by recombinant methods. A "murine antibody" is a full mouse antibody and has only limited use in humane due to its short half-life in circulation and its high immunogenicity. A "chimeric antibody" is a genetically engineered antibody, which contains both mouse and human sequences. The ratio is approximately 33% mouse contribution and 67 % human contribution. Usually the variable domains are murine and the constant region including the Fc fragment is derived from a human IgG.

A "humanized antibody" is a genetically engineered antibody, wherein the mouse content is reduced to about 5-10%. In such cases, the six CDRs of the heavy and light chains and a limited number of structural amino acids of the murine monoclonal antibody are grafted by recombinant technology to the CDR-depleted human IgG scaffold. A fully human antibody or human antibody describes antibodies, which are made in humanized mice resulting in antibodies that do not contain any mouse sequences, or made in vitro using phage libraries or ribosome display or alternatively are obtained from human donors. In certain embodiments, chimeric, humanized or primatized (CDR-grafted) antibodies, comprising portions derived from different species or fully human antibodies, are also encompassed by the present invention. The various portions of these antibodies can be joined together chemically by conventional techniques, or can be prepared as a contiguous protein using genetic engineering techniques. For example, nucleic acids encoding a chimeric or humanized chain can be expressed to produce a contiguous protein. See, e.g., Cabilly et al., U.S. Pat. No. 4,816,567; Cabilly et al., European Patent No. 0,125,023; Boss et al., U.S. Pat. No. 4,816,397; Boss et al., European Patent No. 0,120,694; Neuberger, M. S. et al., WO 86/01533; Neuberger, M. S. et al., European Patent No. 0,194,276 B1; Winter, U.S. Pat. No. 5,225,539; and Winter, European Patent No. 0,239,400 B1. See also, Newman, R. et al., BioTechnology, 10: 1455-1460 (1992), regarding primatized antibody. See, e.g., Ladner et al., U.S. Pat. No. 4,946,778; and Bird, R. E. et al., Science, 242: 423-426 (1988)), regarding single chain antibodies.

In addition, a mixture of antibodies, termed herein as an "antibody preparation", can be used according to the methods of the present invention. Such antibody preparations include polyclonal and monoclonal mixtures of antibodies. A humanized antibody may comprise portions of immunoglobulins of different origin. For example, at least one portion can be of human origin. For example, the humanized antibody can comprise portions derived from an immunoglobulin of nonhuman origin with the requisite specificity, such as a mouse, and from immunoglobulin sequences of human origin (e.g., a chimeric immunoglobulin), joined together chemically by conventional techniques (e.g., synthetic) or prepared as a contiguous polypeptide using genetic engineering techniques (e.g., DNA encoding the protein portions of the chimeric antibody can be expressed to produce a contiguous polypeptide chain). Alternatively, a humanized antibody may be created in a transgenic or humanized animal expressing the human antibody genes (see Lonberg, N. "Transgenic Approaches to Human Monoclonal Antibodies." Handbook of Experimental Pharmacology 113 (1994): 49-101). Another example of a humanized antibody of the present invention is an immunoglobulin containing one or more immunoglobulin chains comprising a CDR of nonhuman origin (e.g., one or more CDRs derived from an antibody of nonhuman origin) and a framework region derived from a light and/or heavy chain of human origin (e.g., CDR-grafted antibodies with or without framework changes). Chimeric or CDR-grafted single chain antibodies are also encompassed by the term "humanized antibody".

In addition, techniques developed for the production of "chimeric antibodies", such as the splicing of mouse antibody genes to human antibody genes to obtain a molecule with appropriate antigen specificity and biological activity, can be used (see, *e.g.*, Morrison, S. L. et al. (1984) Proc. Natl. Acad. Sci. USA 81:6851-6855; Neuberger, M. S. et al. (1984) Nature 312:604-608; and Takeda, S. et al. (1985) Nature 314:452-454). Alternatively, techniques described for the production of single chain antibodies may be adapted, using methods known in the art, to produce CD96, CD112, CD155, CD226, TIGIT, MICA or MICB-specific single chain antibodies. Antibodies with related specificity, but of distinct idiotypic composition, may be generated by chain shuffling from random combinatorial immunoglobulin libraries (see, *e.g.*, Burton, D. R. (1991) Proc. Natl. Acad. Sci. USA 88:10134-10137.). Antibodies may also be produced by inducing *in vivo* production in the lymphocyte population or by screening immunoglobulin libraries or panels of highly specific binding reagents as disclosed in the literature (see, *e.g.*, Orlandi, R. et al. (1989) Proc. Natl. Acad. Sci. USA 86:3833-3837; Winter, G. et al. (1991) Nature 349:293-299).

Antibody fragments which contain specific binding sites for CD96, CD112, CD155, CD226, TIGIT, MICA or MICB may also be generated. For example, such fragments include, but are not limited to, F(ab')₂ fragments produced by pepsin digestion of the antibody molecule and Fab fragments generated by reducing the disulfide bridges of the F(ab')₂ fragments. Alternatively, Fab expression libraries may be constructed to allow rapid and easy identification of monoclonal Fab fragments with the desired specificity (see, *e.g.*, Huse, W. D. et al. (1989) Science 246:1275-1281).

An antibody may have one or more binding sites. If there is more than one binding site, the binding sites may be identical to one another or may be different. For instance, a naturally occurring immunoglobulin has two identical binding sites, a single-chain antibody or Fab fragment has one binding site, while a "bispecific" or "bifunctional" antibody has two different binding sites. An individual antibody's ability to internalize, deliver a payload, and kill a target cell can vary greatly depending on the affinity and the particular target epitope that is engaged by the antibody. The term "human antibody" includes all antibodies that have one or more variable and constant regions derived from human immunoglobulin sequences. In one embodiment, all of the variable and constant domains are derived from human immunoglobulin sequences (a fully human antibody). The term "chimeric antibody" refers to an antibody that contains one or more regions from one antibody and one or more regions from one or more other different antibodies. In one embodiment, one or more of the CDRs are derived from a specific human antibody. In a more preferred embodiment, all of the CDRs are derived from a human antibody. In another preferred embodiment, the CDRs from more than one human antibody are mixed and matched in a chimeric antibody. For instance, a chimeric antibody may comprise a CDR1 from the light chain of a first human antibody combined with CDR2 and CDR3 from the light chain of a second human antibody, and the CDRs from the heavy chain may be derived from a third antibody. Further, the framework regions may be derived from one of the same antibodies, from one or more different antibodies, such as a human antibody, or from a humanized antibody.

Various immunoassays may be used for screening to identify antibodies having the desired specificity. Numerous protocols for competitive binding or immunoradiometric assays using either polyclonal or monoclonal antibodies with established specificities are well known in the art. Such immunoassays typically involve the measurement of complex formation between CD96, CD112, CD155, CD226, TIGIT, MICA or MICB, respectively, and its specific antibody. A two-site, monoclonal-based immunoassay utilizing monoclonal antibodies reactive to two non-interfering CD96, CD112, CD155, CD226, TIGIT, MICA or MICB epitopes is generally used, but a competitive binding assay may also be employed. Various methods such as Scatchard analysis in conjunction with radioimmunoassay techniques may be used to assess the affinity of antibodies for CD96, CD112, CD155, CD226, TIGIT, MICA or MICB. Affinity is expressed as an association constant, Kₐ, which is defined as the molar concentration of CD96-, CD112-, CD155-, CD226-, TIGIT-, MICA-or MICB-antibody complex divided by the molar concentrations of free antigen and free antibody under equilibrium conditions. The Kₐ determined for a preparation of polyclonal antibodies, which are heterogeneous in their affinities for multiple CD96, CD112, CD155, CD226, TIGIT, MICA or MICB epitopes, represents the average affinity, or avidity, of the antibodies for CD226, MICA or MICB. The Kₐ determined for a preparation of monoclonal antibodies, which are monospecific for a particular epitope of CD96, CD112, CD155, CD226, TIGIT, MICA or MICB, represents a true measure of affinity. High-affinity antibody preparations with Kₐ ranging from about 10⁹ to 10¹² L/mole are preferred for use in immunoassays in which the CD96-, CD112-, CD155-, CD226-, TIGIT-, MICA- or MICB-antibody complex must withstand rigorous manipulations.

The titer and avidity of polyclonal antibody preparations may be further evaluated to determine the quality and suitability of such preparations for certain downstream applications. For example, a polyclonal antibody preparation containing at least 1-2 mg specific antibody/ml, preferably 5-10 mg specific antibody/ml, is generally employed in procedures requiring precipitation of CD96-, CD112-, CD155-, CD226-, TIGIT-, MICA- or MICB-antibody complexes. Procedures for evaluating antibody specificity, titer, and avidity, and guidelines for antibody quality and usage in various applications, are generally available.

Methods for assaying a biological sample for the presence of one or more biomarkers selected from CD96, CD112, CD155, CD226, TIGIT, MICA and MICB will be well known to those skilled in the art. Such methods include immunoassays that include, but are not limited to, immunoprecipitation assays, ELISA-based assays, radioimmunoassay, "sandwich" immunoassays, immunodiffusion assays, agglutination assays, and western blot assays. In one example, the selected antibodies against the biomarkers are immobilised on a glass slide in such a manner that an immunological complex is formed with any specific biomarker present in the biological sample reacted with the slide. The glass slide is then brought into contact with the biological sample from an individual, and the antibodies on the slide are allowed to react with the sample for a suitable period of time. The slide is then washed to remove non-reacting protein, and the antibodies that remain on the slide and that have reacted with the biomarkers in the sample are detected using conventional techniques (*i.e.* by reacting with a radioactive anti-IgG antibody). Methods of immobilising proteins on a glass slide, and methods of identifying bound IgG antibodies will be well known to those skilled in the art.

Other methods of detecting biomarkers will be well known to those skilled in the field of proteomics. For example, protein arrays may be generated that are custom-made to assay for the presence of specific biomarkers. The company RZPD (www.rzpd.de) provide a service for the production of customised macro-arrays. Other methods for the detection of the biomarkers of the invention would be the use of ELISA's, the details of which will be well known to those skilled in the field.

The present invention contemplates the use of both monoclonal and polyclonal antibodies in methods of detecting the presence of circulating or tumor antigens. Any suitable method may be used to generate the antibodies used in the methods and kits of the present invention, including but not limited to, those disclosed herein. For example, for preparation of a monoclonal antibody, protein, as such, or together with a suitable carrier or diluent is administered to an animal under conditions that permit the production of antibodies. For enhancing the antibody production capability, complete or incomplete Freund's adjuvant may be administered. Normally, the protein is administered once every 2 weeks to 6 weeks, in total, from about 2 times to about 10 times. Animals suitable for use in such methods include, but are not limited to, primates, rabbits, dogs, guinea pigs, mice, rats, sheep, goats, *etc.*

For preparing monoclonal antibody-producing cells, an individual animal whose antibody titer has been confirmed (*e.g.*, a mouse) is selected, and 2 days to 5 days after the final immunization, its spleen or lymph node is harvested and antibody-producing cells contained therein are fused with myeloma cells to prepare the desired monoclonal antibody producer hybridoma. Measurement of the antibody titer in antiserum can be carried out, for example, by reacting the labelled protein, as described hereinafter and antiserum and then measuring the activity of the labelling agent bound to the antibody. The cell fusion can be carried out according to known methods (Koehler and Milstein (Nature 256:495 [1975])). As a fusion promoter, for example, polyethylene glycol (PEG) or Sendai virus (HVJ), preferably PEG is used.

Polyclonal antibodies may be prepared by any known method or modifications of these methods including obtaining antibodies from patients. For example, a complex of an immunogen (an antigen against the protein) and a carrier protein is prepared and an animal is immunized by the complex according to the same manner as that described with respect to the above monoclonal antibody preparation. A material containing the antibody is recovered from the immunized animal and the antibody is separated and purified. As to the complex of the immunogen and the carrier protein to be used for immunization of an animal, any carrier protein and any mixing proportion of the carrier and a hapten can be employed as long as an antibody against the hapten, which is crosslinked on the carrier and used for immunization, is produced efficiently.

The antibodies may be labelled with a detectable label such as, for example, a fluorescent, luminescent, or radioactive label. Typically, the antibodies will be immobilised to a support, before the support is reacted with a biological sample. The support will then be washed to remove any non-reacting proteins, before any proteins that have formed an immunospecific complex with the antibodies are identified using conventional techniques. Generally, this method is suitable for detecting the presence of autoantigens in biological fluid samples. When the autoantigen is a tumor antigen, in other words, when it is expressed by a tumor cell, the most appropriate method of detection is immunohistochemical detection. Methods of immunohistochemical detection of tumor antigens will be well known to those skilled in the art, and are described previously.

The invention provides a method of treating a cancer, especially metastatic cancer, comprising a step of administering to the individual a therapeutically effective amount of a CD226, MICA or MICB inhibitor, or a combination thereof. The CD226, MICA or MICB inhibitor may be administered together with a therapeutic agent (for example at the same time or as part of a single dose), or it may be administered in advance of or after administration of the therapeutic agent. The therapeutic agent for use with inhibitors of CD226, MICA or MICB can be any standard or experimental agent used in treatment regimens for a specific cancer type. For example, such agents can be selected from Trastuzumab (Herceptin®), Lapatinib (Tykerb®), Neratinib, Afatinib (Tovok®), Pertuzumab, Anastrozol, Exemestane, Fulvestrant, Goserelin, Letrozole, Leuprolide, Megestrol acetate, Tamoxifen, Toremifene, Palbociclib, Gemcitabine, Ixabepilone, Liposomal doxorubicin, Methotrexate, Paclitaxel, Vinorelbine, Capecitabine, Carboplatin, Cisplatin, Cyclophosphamide and Docetaxel.

In this specification, the term "treating" refers to administering an inhibitor of CD226, MICA or MICB, or a combination thereof, to an individual that has a cancer, typically a solid tumour cancer, with the purpose to cure, heal, prevent, alleviate, relieve, alter, remedy, ameliorate, or improve the cancer or symptoms of the cancer. When the term is applied to the use of a CD226, MICA or MICB inhibitor, the respective active agents may be administered together, or separately, and may be administered at the same time or at different times. In one embodiment, the patient may be treated to a course of one active agent, which is then followed by treatment with a course of the second active agent.

The term "therapeutically effective amount" refers to the amount of the CD226, MICA or MICB inhibitor or therapeutic agent/therapy that is required to confer the intended therapeutic effect in the individual, which amount will vary depending on the type of inhibitor, route of administration, status of cancer, and possible inclusion of other therapeutics or excipients. In one embodiment, the therapeutically effective amount is less than 100mg/m2. "mg/m2" means mg per m2 of body surface area, and a method of calculating body surface area is provided at www.halls.md/body-surface-area/bsa.html. In one embodiment, the therapeutically effective amount is less than 50mg/m2. In one embodiment, the therapeutically effective amount is less than 10mg/m2. In one embodiment, the therapeutically effective amount is less than 5mg/m2. In one embodiment, the therapeutically effective amount is less than 1mg/m2. In one embodiment, the therapeutically effective amount is 0.001 to 50mg/m2. In one embodiment the therapeutically effective amount is 0.001 to 10mg/m2. In one embodiment, the therapeutically effective amount is 0.001 to 1.0mg/m2. In one embodiment, the therapeutically effective amount is 0.01 to 10mg/m2. In one embodiment, the therapeutically effective amount is 0.1 to 10mg/m2. In one embodiment, the therapeutically effective amount is 0.001 to 1.0mg/m2. In one embodiment, the therapeutically effective amount is 0.01 to 1mg/m2. In one embodiment, the therapeutically effective amount is 0.1 to 1mg/m2. In one embodiment, the therapeutically effective amount is administered once daily for a period 2 to 14 days. In one embodiment, the therapeutically effective amount is administered once daily for a period 2 to 21 days. In one embodiment, the therapeutically effective amount is administered once daily for a period 2 to 28 days. In another embodiment, the therapeutically effective amount is administered twice daily for a period of 2 to 14 days. In another embodiment, the therapeutically effective amount is administered twice daily for a period of 2 to 21 days. In another embodiment, the therapeutically effective amount is administered twice daily for a period of 2 to 28 days.

The methods of the invention apply to solid tumour cancers (solid tumours), which are cancers of organs and tissue (as opposed to haematological malignancies), and ideally epithelial cancers. Examples of solid tumour cancers include pancreatic cancer, bladder cancer, prostate cancer, ovarian cancer, colorectal cancer (CRC), breast cancer, renal cancer, lung cancer, hepatocellular cancer, cervical cancer, gastric cancer, esophageal cancer, head and neck cancer, carcinoma, sarcoma, melanoma, neuroendocrine cancer. Suitably, the solid tumour cancer suitable for treatment and prognosis according to the methods of the invention are selected from CRC, breast and prostate cancer. In a preferred embodiment of the invention, the invention relates to treatment and prognosis of melanoma, ovarian cancer and chronic myelogenous leukaemia. In another aspect, the methods of the invention apply to treatment and prognosis of outcome of haematological malignancies, including for example multiple myeloma, T-cell lymphoma, B-cell lymphoma, Hodgkin's disease, non-Hodgkin's lymphoma, acute myeloid leukaemia, and chronic myelogenous leukaemia (CML also known as chronic granulocytic leukaemia (CGL)).

Various delivery systems are known and can be used to administer a therapeutic of the invention. Methods of introduction include but are not limited to intradermal, intramuscular, intraperitoneal, intravenous, subcutaneous, intranasal, epidural, intranasal, intracerebral, and oral routes. The compositions may be administered by any convenient route, for example by infusion or bolus injection, by absorption through epithelial or mucocutaneous linings (*e.g.*, oral mucosa, rectal and intestinal mucosa, *etc*.) and may be administered together with other biologically active agents. Administration can be systemic or local. In addition, it may be desirable to introduce the compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g.*, by use of an inhaler or nebulizer, and formulation with an aerosolizing agent.

It may be desirable to administer the compositions of the invention locally to the area in need of treatment; this may be achieved, for example and not by way of limitation, by topical application, by injection, by means of a catheter, by means of a suppository, or by means of an implant, said implant being of a porous, non-porous, or gelatinous material, including membranes, such as sialastic membranes, or fibers.

The present invention also provides pharmaceutical compositions. Such compositions comprise a therapeutically effective amount of the therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. In this specification, the term "therapeutically effective amount" should be taken to mean an amount of therapeutic which results in a clinically significant inhibition, amelioration or reversal of development or occurrence of seizures or, in the case of treatment of stroke, clinically significant inhibition, amelioration or reversal of development of the effects of stroke.

The term "carrier" refers to a diluent, adjuvant, excipient, or vehicle with which the Therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like.

The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, *etc.* Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anaesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachet indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The screening assays of the invention may be performed on any mammalian cell that expresses NeuromedinU (and are ideally HER2-positive), for example SKBR3 and HCC1954 cells (the details of which are provided below). The cells are typically incubated with a test agent, and the expression of NeuromedinU is monitored to detect changes in expression due to the test agent. In one embodiment, the cells are pre-treated or co-treated with a HER2 targeted drug.

### Materials and Methods

### Materials

All materials were sourced from Sigma-Aldrich^{®} unless otherwise stated.

### Cell culture

Ovarian (59M and SKOV3), melanoma (Sk-Mel-28) and CML (K562) cell lines were obtained from ATCC. 59M cells were cultured in DMEM, SKOV3 in McCoy's, Sk-Mel-28 in EMEM and K562 in RPMI 1640 media. All media was supplemented with 10% foetal bovine serum (FBS), 2% penicillin/streptomycin and 2mM L-glutamine. All cell lines were cultured in a humidified atmosphere at 37°C, 5% CO₂.

### PBMC preparation

Whole blood was collected by venipuncture into EDTA. Peripheral blood mononuclear cells (PBMC) were isolated from whole blood by Ficoll density gradient centrifugation (Biosciences). Cells were incubated at 37°C, 5% CO₂ and either left unstimulated or were stimulated for 18 h. Stimulations were performed using 5 × 10⁶ cells/ml in RPMI 1640 containing 10% FBS and 1% penicillin/streptomycin. Cells were either stimulated with IL-2 (500 U/ml) or IFN-α (1000 U/ml).

### Washed Platelet preparation

Whole blood was collected by venipuncture into Acid-Citrate-Dextrose (ACD; 38 mM citric acid, 75 mM sodium citrate, 124 mM D-glucose) as anticoagulant and centrifuged at 170 g for 10 min. Platelet rich plasma (PRP) was acidified to pH 6.5 with ACD, and PGE1 (1 µM) was added to avoid platelet activation during centrifugation. Platelets were pelleted by centrifugation at 720 g for 10 min. The supernatant was removed and the platelet pellet was resuspended in JNL buffer (130 mM NaCl, 10 mM sodium citrate, 9 mM NaHCO3, 6 mM D-glucose, and 0.9 mM MgCl2, 0.81 mM KH2PO4, and 10 mM Tris, pH 7.4) and supplemented with 1.8 mM CaCl₂ prior to commencement of experiments.

### Platelet adhesion assay

Washed suspensions of cancer cells (4 × 10⁶/ml) were incubated with washed platelets (1:1000 cancer cell-platelet ratio) for 1 min under low shear on a rocking table (12 oscillations per minute, opm). Next, samples were washed and labelled with mouse antihuman CD42b-APC (20 µg/ml; BioLegend). Samples were analysed on Cyan flow cytometer (Beckman Coulter).

### Isolation of platelet releasate

Washed platelets (4 x 10⁹/ml) were stimulated with either cancer cells (4 x 10⁶/ml) or Thrombin receptor activating peptide (TRAP; 20 mM; GenScript) at 37 °C for 15 min. The platelet aggregate was centrifuged at 720g for 10 min. The supernatant (platelet releasate) was then aspirated.

### NK cytotoxicity assay

PBMC cells (1x10⁶ cells/well) were stimulated for 18hrs with IL-2 (500 U/ml). For the last 4 h of PBMC stimulation cancer cells (2 x 10⁵ cells/well) or cancer cells cloaked with platelets (2 x 10⁵ cancer cells/ 2x10⁸ platelets/well) were added. In addition, the CD107a-FITC antibody (1.5 µl/well; BD Biosciences) was added to cells. For the final 3 h of stimulation GolgiPlug (2 µl/well; BD Biosciences) and GolgiStop (1 µl/well; BD Biosciences) were added to the assay. The cells were then washed in 1% FBS solution and blocked in a 10% human serum albumin solution at 4°C for 5 min. Next the cells were stained with CD3-PerCP (1 µl/100 µl 1% FBS solution; BD Biosciences) and CD56-PE (1 µl/100 µl 1% FBS solution; BD Biosciences) at 4°C for 20 min. After the extracellular staining was complete the cells were fixed for 20 min, washed in permeabilisation buffer and stained with IFNγPE/Cy7 (1 µl/100 µl permeablisation buffer; BD Biosciences). Samples were analysed on Cyan flow cytometer (Beckman Coulter).

### Ligand/Receptor detection assays

Cancer cells (2 x 10⁵ cells/well) were left untreated or incubated with washed platelets (2 x 10⁸ cells/well) for 24 h and subsequently stained for CD155-FITC (2.5 µg/ml; BioLegend), CD112-APC (5 µg/ml; BioLegend), MICA-APC (30 µg/ml; R&D) or MICB-APC (30 µg/ml; R&D). PBMCs (1 x 10⁶ cells/well) were left untreated or incubated for 24hrs with cancer cells (2 x 10⁵ cells/well) or cancer cells cloaked with platelets (2 x 10⁵ cancer cells/ 2x10⁸ platelets/well) or washed platelets (2 x 10⁸ cells/well) or TRAP activated platelet releasate (from 2 x 10⁸ platelets). NK cells were then stained with CD226-APC (30 µg/ml; BioLegend), CD96-PE (5 µg/ml; eBioscience), TIGIT-PE/Cy7 (25 µg/ml; eBioscience) or NKG2D-APC (30 µg/ml; R&D). All samples were analysed on Cyan flow cytometer (Beckman Coulter).

### Blocking assays

For assay involving NK receptor, PBMC cells (1x10⁶ cells/well) were stimulated for 18hrs with IL-2 (500 U/ml) and blocked with 50 µg/ml of anti-CD96 (BioLegend), CD226 (BioLegend), TIGIT (eBioscience) or NKG2D (R&D) antibody for 30 min at 37°C. For assays involving cancer cell ligands, washed suspensions of cancer cells were blocked with 50 µg/ml of anti-CD 155 (BioLegend), CD112 (BioLegend), MICA (R&D) or MICB (R&D) antibody for 30 min at 37°C. Stimulated PBMCs (1x10⁶ cells/well) and cancer cells (2 x 10⁵ cells/well) were then co-incubated and the NK cytotoxicity assay performed as described above.

For assays involving platelet releasate, platelet releasate from cancer cell activated platelets was prepared and incubated with 50 µg/ml of anti-MICA (R&D) or MICB (R&D) for 30 min at 37°C, prior to the detection of the expression of NKG2D on NK cells.
For assays involving platelet receptors, washed platelets were prepared and incubated with 50 µg/ml of anti-CD226 (BioLegend) for 30 min at 37°C, prior to the platelet adhesion assay being performed.

### Statistical analysis

Statistical analysis of the data generated and graph generation was performed GraphPad Prism 5.0 (Graph Pad Software Inc, La Jolla, USA). P values were generated either using Student's T-tests, two-way ANOVA with Bonferroni post-tests or one-way ANOVA with Tukey's/Dunnett's multiple comparison test as appropriate, with p<0.05 considered as statistically significant.

### Results

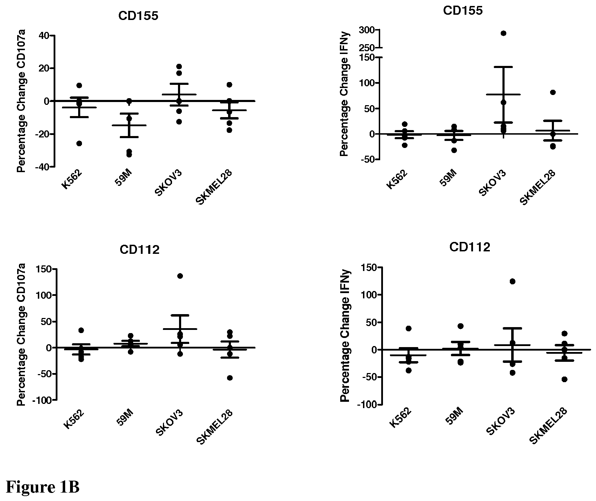

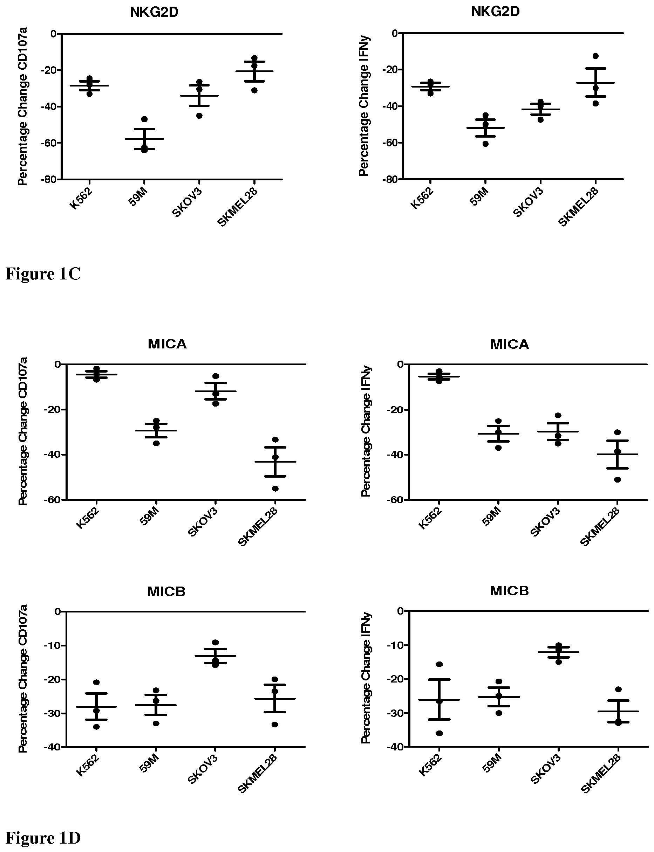

Figure 1 demonstrates the importance of both the NKG2D/MICA/MICB axis and the CD226/CD96/TIGIT/CD155/CD112 axis in the recognition of cancer cells by NK cells and the subsequent destruction of the cancer cells by the NK cells. For example in Figure 1A, blocking CD226 on the NK cells inhibits the ability of the NK cells to translocate CD107a to its surface and also release IFNγ when the NK cells come in contact with ovarian (59M and SKOV3), melanoma (Sk-Mel-28) and CML (K562) *in vitro* cells.

Figure 2 and 3A shows that when platelets adhere and cloak cancer cells there are significant alterations in the CD226/CD96/TIGIT/CD155/CD112 axis. Figure 2 shows that when platelets cloak ovarian (59M and SKOV3), melanoma (Sk-Mel-28) and CML (K562) cancer cells there is a decrease in the level of CD155 and CD112 expression on the cancer cells. Figure 3A demonstrates there is a reciprocal decrease in the expression of both CD226 and CD96 on NK cells when they come in contact with platelet cloaked cancer cells and that in the case of CD96 this is specific to the platelet cloaked cancer cells, while cancer cells alone can induce a similar response to CD226. Figure 3B demonstrates that when NK cells come in contact with platelets there is a significant decrease in the NK receptors CD226, CD96 and TIGIT. In addition, platelet releasate from platelets activated by ovarian (59M and SKOV3), melanoma (Sk-Mel-28) and CML (K562) cancer cells decrease the expression of these three receptors. Overall these graphs illustrates that platelet cloaking of cancer cells influences the expression level of the members of the CD226/CD96/TIGIT/CD155/CD112 axis and thus the ability of NK cells to recognise and kill cancer cells. There is a greater level of down-regulation of CD96 in platelet-cloaked cells compared to uncloaked cancer cells.

Figure 5 shows that platelet cloaked cancer cells to modulate the NKG2D/MICA/MICB NK immune surveillance mechanism. Figure 5A illustrates the ability of cancer cells and cloaked cancer cells to down-regulate the NKG2D receptor on NK cells. While, Figure 5B demonstrates that platelet cloaked cancer cells have a reduced level of MICA and MICB expression on the cancer cell surface in comparison to non-cloaked cancer cells.

Figure 6A demonstrates the ability of platelet cloaking to alter the levels of soluble MICA and MICB released from ovarian (59M and SKOV3), melanoma (Sk-Mel-28) and CML (K562) cancer cells. Figure 6B describes the use of soluble MICA and MICB in platelet releasate from platelets activated by ovarian (59M and SKOV3), melanoma (Sk-Mel-28) and CML (K562) cancer cells to down-regulate the expression of NKG2D on NK cells. Overall these graphs demonstrate the ability of soluble MICA and MICB released from cancer cells to modulate the immune surveillance ability of NK cells towards cancer cells.

Figure 7 demonstrates that blocking the CD226 receptor on platelets prior to exposure to ovarian (59M and SKOV3), melanoma (Sk-Mel-28) and CML (K562) cancer cells significantly inhibits the ability platelets to cloak these cancer cells. This is a highly significant result and it illustrates a potentially important mechanism by which to inhibit the platelet cloaking phenomenon in individuals with cancer and thus, curtailing the formation of cancer metastases.

Figure 8 highlights a mechanism by which to reinstate that immune surveillance ability of NK cells towards platelet cloaked cancer cells. These graphs demonstrate that blocking the soluble MICA and MICB in platelet releasate from platelets activated by ovarian (59M and SKOV3), melanoma (Sk-Mel-28) and CML (K562) cancer cells restores the ability of NK cells to recognize and kill these cancer cells. Again this is another highly significant results as it describes a potentially important mechanism by which the immune evasion of platelet cloaked cancer cells can be inhibited.

In conclusion, the roles of CD 112 and TIGIT as key elements in the inhibition of NK cell activity by platelet-cloaked circulating tumour cells (CTCs) has not been disclosed and their involvement is non-obvious. Furthermore, the reversibility of platelet-cloaking of cancer cells based on the targeting of CD226 and soluble MICA/MICB has never been reported and may be targeted to re-instate the ability of NK cells to target CTCs by reversing platelet-cloaking through the NKG2D signalling mechanism. This targeting of solubilised ligands MICA and MICB to restore NK cell function represents a reverse immune therapy approach.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

### SEQUENCES

SEQ ID 1 (nucleotide) CD96 (Homo sapiens CD96 molecule (CD96)), transcript variant 1 NM_198196) 
TTCCTGTCTACGTTTCATTTCCTGGGGGCTTGCCAAGTGATAAACAGACCCAGGCGTGTGGTAGAGTTCGG
GTTTTTAGCACGAAGTGGGTGGCTGGAGTTTGCTTGAAAACATCAATTGACTTTGTGATCATTACAGAAATG
CTGGTGTAAGGTGTTCAGAAGACAATGGAGAAAAATGGAAATACTGTGCTGTCTATTACATCATCCAGATAC
ATTTTGTCAAGGGAGTTTGGGAAAAACAGTCAACACAGAAGAAAATGTTTATGCTACACTTGGCTCTGATGT
CAACCTGACCTGCCAAACACAGACAGTAGGCTTCTTCGTGCAGATGCAATGGTCCAAGGTCACCAATAAGATA
GACCTGATTGCTGTCTATCATCCCCAATACGGCTTCTACTGTGCCTATGGGAGACCCTGTGAGTCACTTGTGAC
TTTCACAGAAACTCCTGAGAATGGGTCAAAATGGACTCTGCACTTAAGGAATATGTCTTGTTCAGTCAGTGGA
AGGTACGAGTGTATGCTTGTTCTGTATCCAGAGGGCATTCAGACTAAAATCTACAACCTTCTCATTCAGACACA
CGTTACAGCAGATGAATGGAACAGCAACCATACGATAGAAATAGAGATAAATCAGACTCTGGAAATACCATG
CTTTCAAAATAGCTCCTCAAAAATTTCATCTGAGTTCACCTATGCATGGTCGGTGGAAAACAGCAGCACGGATT
CTTGGGTCCTTCTTTCTAAGGGTATAAAGGAGGATAATGGAACTCAGGAAACACTTATCTCCCAAAATCACCTC
ATCAGCAATTCCACATTACTTAAAGATAGAGTCAAGCTTGGTACAGACTACAGACTCCACCTCTCTCCAGTCCA
AATCTTCGATGATGGGCGGAAGTTCTCTTGCCACATTAGAGTCGGTCCTAACAAAATCTTGAGGAGCTCCACC
ACAGTCAAGGTTTTTGCTAAACCAGAAATCCCTGTGATTGTGGAAAATAACTCCACGGATGTCTTGGTAGAGA
GAAGATTTACCTGCTTACTAAAGAATGTATTTCCCAAAGCAAATATCACATGGTTTATAGATGGAAGTTTTCTT
CATGATGAAAAAGAAGGAATATATATTACTAATGAAGAGAGAAAAGGCAAAGATGGATTTTTGGAACTGAAG
TCTGTTTTAACAAGGGTACATAGTAATAAACCAGCCCAATCAGACAACTTGACCATTTGGTGTATGGCTCTGTC
TCCAGTCCCAGGAAATAAAGTGTGGAACATCTCATCAGAAAAGATCACTTTTCTCTTAGGTTCTGAAATTTCCT
CAACAGACCCTCCACTGAGTGTTACAGAATCTACCCTTGACACCCAACCTTCTCCAGCCAGCAGTGTATCTCCT
GCAAGATATCCAGCTACATCTTCAGTGACCCTTGTAGATGTGAGTGCCTTGAGGCCAAACACCACTCCTCAACC
CAGCAATTCCAGTATGACTACCCGAGGCTTCAACTATCCCTGGACCTCCAGTGGGACAGATACCAAAAAATCA
GTTTCACGGATACCTAGTGAAACATACAGTTCATCCCCGTCAGGTGCAGGCTCAACACTTCATGACAATGTCTT
TACCAGCACAGCCAGAGCATTTTCAGAAGTCCCCACAACTGCCAATGGATCTACGAAAACTAATCACGTCCAT
ATCACTGGTATTGTGGTCAATAAGCCCAAAGATGGAATGTCCTGGCCAGTGATTGTAGCAGCTTTACTCTTTTG
CTGCATGATATTGTTTGGTCTTGGAGTGAGAAAATGGTGTCAGTACCAAAAAGAAATAATGGAAAGACCTCCA
CCTTTCAAGCCACCACCACCTCCCATCAAGTACACTTGCATTCAAGAGCCCAACGAAAGTGATCTGCCTTATCA
TGAGATGGAGACCCTCTAGTCTCGTGAGACTTTGCCCCATGGCAGAACTCTGCTGGAATCCTATTGAGAAGGT
AGACATTGTGCTTTATTAATATAGTCGCTCTTCAGCCATGCCTTTGCTGCAGCTGAAATGGAAGTCAGAAGTGA
GTGACCTGTTTTCCCAGCAACTCACCCTCTTCCATCTCCAAACGCCTGAAGCTTAACCAAGAGTGAGAGGATAT
GTCATGTTCACACTCAATGCAATTCGTAGTGGTTTTCTTGCTTATGTAAGAAGTACATATTAGTCTGCCATCTTT
AAAAAAAAATACAGTATTTTCATTTAAATTCTCTGATGGAGGGACAACAATGGTTTCAACTGTATGCCCATGCC
TGATCCTCTTATTTGAACATCTATCAACATTGTAAACTCTTTGCCAAAATCCTGGGGCTTTGCTGCATTCCCTAA
GATAATTACAGGAAAAAGAAAATGTAAAAGTGCTAACAAGGCTGCCAAGTAATGGAGAAGTATGGTTAGTCT
TCATATTGAAATTCTGTTGCTTATTTCATGGAAGGAAACAGAATACTTTGCACAGGAACCACATTTTCAATCCT
CCTTCACTGTCTTCCTACCATGTTCAGCCCAGACTCCTGCCACATGGACCAGGATGAAGAGGGATCAAAGAGA
 
TAATTAGCCAAAAACCCAGTAGCCTAGAAGATACAAAACTCCACTGGCCTCTAAAATTATATTAGCCAAGAGT
GGTTTCATTTGAGTGCCTTCGTGTGTATGTCCATCAAACTGGAACCAAACTGTTTTGTAAGTAAACAGGCAGCC
TAAGCCCAACCCTACTTTCTAATTCCAGTTATTCTCTTTTTCATCTGGGGATTTACCTGTTCATTTAATCTGCCTG
TTTTGATCTGTTTTGAAAAAGATAAAGAGCCTCAAATCAGACCAGCACTGATTAATTAACCCTGCTCCTACCAA
TCTTTTTTAAAGCAGTTGAAGCAGAATGTATAGGTGTCAGAGAAGAAACCTAGTCAGCCAGACGTGCTCTGTA
TTCAGCAATAGTTTGTGAATGAATAAATTACTAATCCTCCTTGTCGCTTGAAACCTTCCCACACTCCCTGCTCCA
GGAGGGAAAAACAGATGTTGTTGACAGATAGAGTGATAGGCAAATTCTGTGTGGACTTTAGTCCCAAAAGGA
AACTTTAGTTCACTTGCAGTATGCTTATCCTTGACTGCACATGAGAATGCCTTGTGCAGAGTTATTTGGAGATT
ATGTCTTTTTCTTAAACACCATGGCTGTCACACTTCAGTTCAATTAAATCAGAATGTCTGAGGAGTGAGACACA
GGCATCAACACTCTCAAATGATTCACATGTTCAGCCAAAGTTGAGAACCATCGAGCCTGTGGAAGTTCTTTCTC
ATGGCTCAGAATCTTAGGTAGGTGCTTAACTCTTGTGGTGGCCAGCCTCCAAGATGAGCCCCAGTGTTCTTGC
CTCCTACTATTCACATCTTTATGTGGTCCCCTCCAATGCTGAATACAGATGATTTGTGTAACCTGAGGCCAGGA
TTAAGGGGAGGCAATCAATGCACCTAGGGAAAAAATTTAAGGAGGTATTCACACTCAGGGTCATGCACTTGC
ACAATGTTGAGAATGAGTACCACTCTCACCATTGGTATAGCCAAAAAAGCTTGGAAGTGACCAAGGCTAGGTC
ACAAAATACACTGTGGCTTCTTCTTTGATCTCTCTTTGACCATACTGACACTGGGAAAAGCCCATTCCCATGCCA
TGAAGACACCAAGGCAGCCCTATTGAGAAATCTACCTGTCGTGGCCGGGCGCAGTGGCTCACGCCTGTAATCC
CAGCACTTTGGGAGGCCGAGGTGGGTGGATCACGAGGTCAGGAGATCGAGACCATCCTGGCTAACACAGTG
AAACCCCGTCTCTACTAAAAATACAAAAAATTAGCCGGGTGTGGTGTCGGGCACCTGTAGTCCCAGCTACTCA
GGAGGCTGAGGCAGGAGAAGGGTGGGAACCCGGGAGGCAGAGCTTGCAGTGAGCCGAGATTGTGCCACTG
CACACTCCAATCTGGGTGAAAGACCGAGACTCCGCCTCAAAAAAAAAAAAAAAAGAAAGAAAGAAAGAAAG
AAAGAAATCTACCTGTCAAGGAACTAAGGTATTTTGCTAACAAGCACCAACTTGCCAGCCATGTAAGGGAGCC
ATCTTGGAAGCAGATCCTCCAGCCTCCAGTCAAGTCTTCAGATAATTGCAACTTCAGTTGATCTTTTGACCAAG
ACCTCAAGAGAGCCAGAACTACCCAGCTAAGCCTTTTACTAAATTTCTGAACTTCTAACACTATTAGATAATAA
GTGCTTATTGTTTAACACCATTAATTTTGAGTATAATTTGTTACATAGCGACAGATAACTATACAGCTCAACAAC
TAGAAAAATAAACTGTTTACCTGCCTTAATTATTTATCTTTAGTTCCTTATTAGTTCTCAAGAAACAAATGCTAG
CTTCATATGTATGGCTGTTGCTTTGCTTCATGTGTATGGCTATTTGTATTTAACAAGACTTAATCATCAGTA
SEQ ID NO. 2 (mRNA) CD96 (Homo sapiens CD96 molecule (CD96)), transcript variant 1 NM_198196) 
ATGGAGAAAAAATGGAAATACTGTGCTGTCTATTACATCATCCAGATACATTTTGTCAAGGGAGTTTGGGAAA
AAACAGTCAACACAGAAGAAAATGTTTATGCTACACTTGGCTCTGATGTCAACCTGACCTGCCAAACACAGAC
AGTAGGCTTCTTCGTGCAGATGCAATGGTCCAAGGTCACCAATAAGATAGACCTGATTGCTGTCTATCATCCCC
AATACGGCTTCTACTGTGCCTATGGGAGACCCTGTGAGTCACTTGTGACTTTCACAGAAACTCCTGAGAATGG
GTCAAAATGGACTCTGCACTTAAGGAATATGTCTTGTTCAGTCAGTGGAAGGTACGAGTGTATGCTTGTTCTG
TATCCAGAGGGCATTCAGACTAAAATCTACAACCTTCTCATTCAGACACACGTTACAGCAGATGAATGGAACA
GCAACCATACGATAGAAATAGAGATAAATCAGACTCTGGAAATACCATGCTTTCAAAATAGCTCCTCAAAAAT
TTCATCTGAGTTCACCTATGCATGGTCGGTGGAAAACAGCAGCACGGATTCTTGGGTCCTTCTTTCTAAGGGTA
TAAAGGAGGATAATGGAACTCAGGAAACACTTATCTCCCAAAATCACCTCATCAGCAATTCCACATTACTTAAA
GATAGAGTCAAGCTTGGTACAGACTACAGACTCCACCTCTCTCCAGTCCAAATCTTCGATGATGGGCGGAAGT
TCTCTTGCCACATTAGAGTCGGTCCTAACAAAATCTTGAGGAGCTCCACCACAGTCAAGGTTTTTGCTAAACCA
GAAATCCCTGTGATTGTGGAAAATAACTCCACGGATGTCTTGGTAGAGAGAAGATTTACCTGCTTACTAAAGA
ATGTATTTCCCAAAGCAAATATCACATGGTTTATAGATGGAAGTTTTCTTCATGATGAAAAAGAAGGAATATAT
ATTACTAATGAAGAGAGAAAAGGCAAAGATGGATTTTTGGAACTGAAGTCTGTTTTAACAAGGGTACATAGT
AATAAACCAGCCCAATCAGACAACTTGACCATTTGGTGTATGGCTCTGTCTCCAGTCCCAGGAAATAAAGTGT
GGAACATCTCATCAGAAAAGATCACTTTTCTCTTAGGTTCTGAAATTTCCTCAACAGACCCTCCACTGAGTGTTA
CAGAATCTACCCTTGACACCCAACCTTCTCCAGCCAGCAGTGTATCTCCTGCAAGATATCCAGCTACATCTTCA
GTGACCCTTGTAGATGTGAGTGCCTTGAGGCCAAACACCACTCCTCAACCCAGCAATTCCAGTATGACTACCC
GAGGCTTCAACTATCCCTGGACCTCCAGTGGGACAGATACCAAAAAATCAGTTTCACGGATACCTAGTGAAAC
ATACAGTTCATCCCCGTCAGGTGCAGGCTCAACACTTCATGACAATGTCTTTACCAGCACAGCCAGAGCATTTT
CAGAAGTCCCCACAACTGCCAATGGATCTACGAAAACTAATCACGTCCATATCACTGGTATTGTGGTCAATAA
GCCCAAAGATGGAATGTCCTGGCCAGTGATTGTAGCAGCTTTACTCTTTTGCTGCATGATATTGTTTGGTCTTG
 
GAGTGAGAAAATGGTGTCAGTACCAAAAAGAAATAATGGAAAGACCTCCACCTTTCAAGCCACCACCACCTCC
CATCAAGTACACTTGCATTCAAGAGCCCAACGAAAGTGATCTGCCTTATCATGAGATGGAGACCCTCTAG
SEQ ID NO. 3 (Protein) CD96 (Homo sapiens CD96 molecule (CD96)), transcript variant 1) NP_937839.1 
MEKKWKYCAVYYIIQIHFVKGVWEKTVNTEENVYATLGSDVNLTCQTQTVGFFVQMQWSKVTNKIDLIAVYHPQ
YGFYCAYGRPCESLVTFTETPENGSKWTLHLRNMSCSVSGRYECMLVLYPEGIQTKIYNLLIQTHVTADEWNSNHTI
EIEINQTLEIPCFQNSSSKISSEFTYAWSVENSSTDSWVLLSKGIKEDNGTQETLISQNHLISNSTLLKDRVKLGTDYRL
HLSPVQIFDDGRKFSCHIRVGPNKILRSSTTVKVFAKPEIPVIVENNSTDVLVERRFTCLLKNVFPKANITWFIDGSFLH
DEKEGIYITNEERKGKDGFLELKSVLTRVHSNKPAQSDNLTIWCMALSPVPGNKVWNISSEKITFLLGSEISSTDPPLS
VTESTLDTQPSPASSVSPARYPATSSVTLVDVSALRPNTTPQPSNSSMTTRGFNYPWTSSGTDTKKSVSRIPSETYSS
SPSGAGSTLHDNVFTSTARAFSEVPTTANGSTKTNHVHITGIVVNKPKDGMSWPVIVAALLFCCMILFGLGVRKWC
QYQKEIMERPPPFKPPPPPIKYTCIQEPNESDLPYHEMETL
SEQ ID NO. 4 Homo sapiens CD96 molecule (CD96), transcript variant 2, NM_005816 (nucleotide) 
TTCCTGTCTACGTTTCATTTCCTGGGGGCTTGCCAAGTGATAAACAGACCCAGGCGTGTGGTAGAGTTCGG
GTTTTTAGCACGAAGTGGGTGGCTGGAGTTTGCTTGAAAACATCAATTGACTTTGTGATCATTACAGAAATG
CTGGTGTAAGGTGTTCAGAAGACAATGGAGAAAAAATGGAAATACTGTGCTGTCTATTACATCATCCAGATAC
ATTTTGTCAAGGGAGTTTGGAAAAAACAGTCAACACAGAAGAAAATGTTTATGCTACACTTGGCTCTGATGT
CAACCTGACCTGCCAAACACAGACAGTAGGCTTCTTCGTGCAGATGCAATGGTCCAAGGTCACCAATAAGATA
GACCTGATTGCTGTCTATCATCCCCAATACGGCTTCTACTGTGCCTATGGGAGACCCTGTGAGTCACTTGTGAC
TTTCACAGAAACTCCTGAGAATGGGTCAAAATGGACTCTGCACTTAAGGAATATGTCTTGTTCAGTCAGTGGA
AGGTACGAGTGTATGCTTGTTCTGTATCCAGAGGGCATTCAGACTAAAATCTACAACCTTCTCATTCAGACACA
CGTTACAGCAGATGAATGGAACAGCAACCATACGATAGAAATAGAGATAAATCAGACTCTGGAAATACCATG
CTTTCAAAATAGCTCCTCAAAAATTTCATCTGAGTTCACCTATGCATGGTCGGTGGAGGATAATGGAACTCAG
GAAACACTTATCTCCCAAAATCACCTCATCAGCAATTCCACATTACTTAAAGATAGAGTCAAGCTTGGTACAGA
CTACAGACTCCACCTCTCTCCAGTCCAAATCTTCGATGATGGGCGGAAGTTCTCTTGCCACATTAGAGTCGGTC
CTAACAAAATCTTGAGGAGCTCCACCACAGTCAAGGTTTTTGCTAAACCAGAAATCCCTGTGATTGTGGAAAA
TAACTCCACGGATGTCTTGGTAGAGAGAAGATTTACCTGCTTACTAAAGAATGTATTTCCCAAAGCAAATATCA
CATGGTTTATAGATGGAAGTTTTCTTCATGATGAAAAAGAAGGAATATATATTACTAATGAAGAGAGAAAAG
GCAAAGATGGATTTTTGGAACTGAAGTCTGTTTTAACAAGGGTACATAGTAATAAACCAGCCCAATCAGACAA
CTTGACCATTTGGTGTATGGCTCTGTCTCCAGTCCCAGGAAATAAAGTGTGGAACATCTCATCAGAAAAGATC
ACTTTTCTCTTAGGTTCTGAAATTTCCTCAACAGACCCTCCACTGAGTGTTACAGAATCTACCCTTGACACCCAA
CCTTCTCCAGCCAGCAGTGTATCTCCTGCAAGATATCCAGCTACATCTTCAGTGACCCTTGTAGATGTGAGTGC
CTTGAGGCCAAACACCACTCCTCAACCCAGCAATTCCAGTATGACTACCCGAGGCTTCAACTATCCCTGGACCT
CCAGTGGGACAGATACCAAAAAATCAGTTTCACGGATACCTAGTGAAACATACAGTTCATCCCCGTCAGGTGC
AGGCTCAACACTTCATGACAATGTCTTTACCAGCACAGCCAGAGCATTTTCAGAAGTCCCCACAACTGCCAATG
GATCTACGAAAACTAATCACGTCCATATCACTGGTATTGTGGTCAATAAGCCCAAAGATGGAATGTCCTGGCC
AGTGATTGTAGCAGCTTTACTCTTTTGCTGCATGATATTGTTTGGTCTTGGAGTGAGAAAATGGTGTCAGTACC
AAAAAGAAATAATGGAAAGACCTCCACCTTTCAAGCCACCACCACCTCCCATCAAGTACACTTGCATTCAAGA
GCCCAACGAAAGTGATCTGCCTTATCATGAGATGGAGACCCTCTAGTCTCGTGAGACTTTGCCCCATGGCAGA
ACTCTGCTGGAATCCTATTGAGAAGGTAGACATTGTGCTTTATTAATATAGTCGCTCTTCAGCCATGCCTTTGCT
GCAGCTGAAATGGAAGTCAGAAGTGAGTGACCTGTTTTCCCAGCAACTCACCCTCTTCCATCTCCAAACGCCTG
AAGCTTAACCAAGAGTGAGAGGATATGTCATGTTCACACTCAATGCAATTCGTAGTGGTTTTCTTGCTTATGTA
AGAAGTACATATTAGTCTGCCATCTTTAAAAAAAAATACAGTATTTTCATTTAAATTCTCTGATGGAGGGACAA
CAATGGTTTCAACTGTATGCCCATGCCTGATCCTCTTATTTGAACATCTATCAACATTGTAAACTCTTTGCCAAA
ATCCTGGGGCTTTGCTGCATTCCCTAAGATAATTACAGGAAAAAGAAAATGTAAAAGTGCTAACAAGGCTGCC
AAGTAATGGAGAAGTATGGTTAGTCTTCATATTGAAATTCTGTTGCTTATTTTCATGGAAGGAAACAGAATACT
TTGCACAGGAACCACATTTTCAATCCTCCTTCACTGTCTTCCTACCATGTTCAGCCCAGACTCCTGCCACATGGA
CCAGGATGAAGAGGGATCAAAGAGATAATTAGCCAAAAACCCAGTAGCCTAGAAGATACAAAACTCCACTGG
CCTCTAAAATTATATTAGCCAAGAGTGGTTTCATTTGAGTGCCTTCGTGTGTATGTCCATCAAACTGGAACCAA
 
ACTGTTTTGTAAGTAAACAGGCAGCCTAAGCCCAACCCTACTTTCTAATTCCAGTTATTCTCTTTTTCATCTGGG
GATTTACCTGTTCATTTAATCTGCCTGTTTTGATCTGTTTTGAAAAAGATAAAGAGCCTCAAATCAGACCAGCA
CTGATTAATTAACCCTGCTCCTACCAATCTTTTTTAAAGCAGTTGAAGCAGAATGTATAGGTGTCAGAGAAGAA
ACCTAGTCAGCCAGACGTGCTCTGTATTCAGCAATAGTTTGTGAATGAATAAATTACTAATCCTCCTTGTCGCTT
GAAACCTTCCCACACTCCCTGCTCCAGGAGGGAAAAACAGATGTTGTTGACAGATAGAGTGATAGGCAAATTC
TGTGTGGACTTTAGTCCCAAAAGGAAACTTTAGTTCACTTGCAGTATGCTTATCCTTGACTGCACATGAGAATG
CCTTGTGCAGAGTTATTTGGAGATTATGTCTTTTTCTTAAACACCATGGCTGTCACACTTCAGTTCAATTAAATC
AGAATGTCTGAGGAGTGAGACACAGGCATCAACACTCTCAAATGATTCACATGTTCAGCCAAAGTTGAGAACC
ATCGAGCCTGTGGAAGTTCTTTCTCATGGCTCAGAATCTTAGGTAGGTGCTTAACTCTTGTGGTGGCCAGCCTC
CAAGATGAGCCCCAGTGTTCTTGCCTCCTACTATTCACATCTTTATGTGGTCCCCTCCAATGCTGAATACAGATG
ATTTGTGTAACCTGAGGCCAGGATTAAGGGGAGGCAATCAATGCACCTAGGGAAAAAATTTAAGGAGGTATT
CACACTCAGGGTCATGCACTTGCACAATGTTGAGAATGAGTACCACTCTCACCATTGGTATAGCCAAAAAAGC
TTGGAAGTGACCAAGGCTAGGTCACAAAATACACTGTGGCTTCTTCTTTGATCTCTCTTTGACCATACTGACAC
TGGGAAAAGCCCATTCCCATGCCATGAAGACACCAAGGCAGCCCTATTGAGAAATCTACCTGTCGTGGCCGG
GCGCAGTGGCTCACGCCTGTAATCCCAGCACTTTGGGAGGCCGAGGTGGGTGGATCACGAGGTCAGGAGAT
CGAGACCATCCTGGCTAACACAGTGAAACCCCGTCTCTACTAAAAATACAAAAAATTAGCCGGGTGTGGTGTC
GGGCACCTGTAGTCCCAGCTACTCAGGAGGCTGAGGCAGGAGAAGGGTGGGAACCCGGGAGGCAGAGCTT
GCAGTGAGCCGAGATTGTGCCACTGCACACTCCAATCTGGGTGAAAGACCGAGACTCCGCCTCAAAAAAAAA
AAAAAAAGAAAGAAAGAAAGAAAGAAAGAAATCTACCTGTCAAGGAACTAAGGTATTTTGCTAACAAGCACC
AACTTGCCAGCCATGTAAGGGAGCCATCTTGGAAGCAGATCCTCCAGCCTCCAGTCAAGTCTTCAGATAATTG
CAACTTCAGTTGATCTTTTGACCAAGACCTCAAGAGAGCCAGAACTACCCAGCTAAGCCTTTTACTAAATTTCT
GAACTTCTAACACTATTAGATAATAAGTGCTTATTGTTTAACACCATTAATTTTGAGTATAATTTGTTACATAGC
GACAGATAACTATACAGCTCAACAACTAGAAAAATAAACTGTTTACCTGCCTTAATTATTTATCTTTAGTTCCTT
ATTAGTTCTCAAGAAACAAATGCTAGCTTCATATGTATGGCTGTTGCTTTGCTTCATGTGTATGGCTATTTGTAT
TTAACAAGACTTAATCATCAGTA
SEQ ID 5 Homo sapiens CD96 molecule (CD96), transcript variant 2, (mRNA) NM_005816.4 
ATGGAGAAAAAATGGAAATACTGTGCTGTCTATTACATCATCCAGATACATTTTGTCAAGGGAGTTTGGGAAA
AAACAGTCAACACAGAAGAAAATGTTTATGCTACACTTGGCTCTGATGTCAACCTGACCTGCCAAACACAGAC
AGTAGGCTTCTTCGTGCAGATGCAATGGTCCAAGGTCACCAATAAGATAGACCTGATTGCTGTCTATCATCCCC
AATACGGCTTCTACTGTGCCTATGGGAGACCCTGTGAGTCACTTGTGACTTTCACAGAAACTCCTGAGAATGG
GTCAAAATGGACTCTGCACTTAAGGAATATGTCTTGTTCAGTCAGTGGAAGGTACGAGTGTATGCTTGTTCTG
TATCCAGAGGGCATTCAGACTAAAATCTACAACCTTCTCATTCAGACACACGTTACAGCAGATGAATGGAACA
GCAACCATACGATAGAAATAGAGATAAATCAGACTCTGGAAATACCATGCTTTCAAAATAGCTCCTCAAAAAT
TTCATCTGAGTTCACCTATGCATGGTCGGTGGAGGATAATGGAACTCAGGAAACACTTATCTCCCAAAATCAC
CTCATCAGCAATTCCACATTACTTAAAGATAGAGTCAAGCTTGGTACAGACTACAGACTCCACCTCTCTCCAGT
CCAAATCTTCGATGATGGGCGGAAGTTCTCTTGCCACATTAGAGTCGGTCCTAACAAAATCTTGAGGAGCTCC
ACCACAGTCAAGGTTTTTGCTAAACCAGAAATCCCTGTGATTGTGGAAAATAACTCCACGGATGTCTTGGTAG
AGAGAAGATTTACCTGCTTACTAAAGAATGTATTTCCCAAAGCAAATATCACATGGTTTATAGATGGAAGTTTT
CTTCATGATGAAAAAGAAGGAATATATATTACTAATGAAGAGAGAAAAGGCAAAGATGGATTTTTGGAACTG
AAGTCTGTTTTAACAAGGGTACATAGTAATAAACCAGCCCAATCAGACAACTTGACCATTTGGTGTATGGCTCT
GTCTCCAGTCCCAGGAAATAAAGTGTGGAACATCTCATCAGAAAAGATCACTTTTCTCTTAGGTTCTGAAATTT
CCTCAACAGACCCTCCACTGAGTGTTACAGAATCTACCCTTGACACCCAACCTTCTCCAGCCAGCAGTGTATCT
CCTGCAAGATATCCAGCTACATCTTCAGTGACCCTTGTAGATGTGAGTGCCTTGAGGCCAAACACCACTCCTCA
ACCCAGCAATTCCAGTATGACTACCCGAGGCTTCAACTATCCCTGGACCTCCAGTGGGACAGATACCAAAAAA
TCAGTTTCACGGATACCTAGTGAAACATACAGTTCATCCCCGTCAGGTGCAGGCTCAACACTTCATGACAATGT
CTTTACCAGCACAGCCAGAGCATTTTCAGAAGTCCCCACAACTGCCAATGGATCTACGAAAACTAATCACGTCC
ATATCACTGGTATTGTGGTCAATAAGCCCAAAGATGGAATGTCCTGGCCAGTGATTGTAGCAGCTTTACTCTTT
TGCTGCATGATATTGTTTGGTCTTGGAGTGAGAAAATGGTGTCAGTACCAAAAAGAAATAATGGAAAGACCTC
CACCTTTCAAGCCACCACCACCTCCCATCAAGTACACTTGCATTCAAGAGCCCAACGAAAGTGATCTGCCTTAT
CATGAGATGGAGACCCTCTAG
SEQ ID NO. 6 Homo sapiens CD96 molecule (CD96), transcript variant 2, (Protein) NP_005807.1 
MEKKWKYCAVYYIIQIHFVKGVWEKTVNTEENVYATLGSDVNLTCQTQTVGFFVQMQWSKVTNKIDLIAVYHPQ
YGFYCAYGRPCESLVTFTETPENGSKWTLHLRNMSCSVSGRYECMLVLYPEGIQTKIYNLLIQTHVTADEWNSNHTI
EIEINQTLEIPCFQNSSSKISSEFTYAWSVENSSTDSWVLLSKGIKEDNGTQETLISQNHLISNSTLLKDRVKLGTDYRL
HLSPVQIFDDGRKFSCHIRVGPNKILRSSTTVKVFAKPEIPVIVENNSTDVLVERRFTCLLKNVFPKANITWFIDGSFLH
DEKEGIYITNEERKGKDGFLELKSVLTRVHSNKPAQSDNLTIWCMALSPVPGNKVWNISSEKITFLLGSEISSTDPPLS
VTESTLDTQPSPASSVSPARYPATSSVTLVDVSALRPNTTPQPSNSSMTTRGFNYPWTSSGTDTKKSVSRIPSETYSS
SPSGAGSTLHDNVFTSTARAFSEVPTTANGSTKTNHVHITGIVVNKPKDGMSWPVIVAALLFCCMILFGLGVRKWC
QYQKEIMERPPPFKPPPPPIKYTCIQEPNESDLPYHEMETL
SEQ ID NO. 7 Homo sapiens CD96 molecule (CD96), transcript variant 3 (nucleotide) NM_001318889 
TTCCTGTCTACGTTTCATTTCCTGGGGGCTTGCCAAGTGATAAACAGACCCAGGCGTGTGGTAGAGTTCGG
GTTTTTAGCACGAAGTGGGTGGCTGGAGTTTGCTTGAAAACATCAATTGACTTTGTGATCATTACAGAAATG
CTGGTGTAAGGTGTTCAGAAGACAATGGAGAAAAATGGAAATACTGTGCTGTCTATTACATCATCCAGATAC
ATTTTGTCAAGGGAGTTTGGGAAAAACAGTCAACACAGAAGAAAATGTTTATGCTACACTTGGCTCTGATGT
CAACCTGACCTGCCAAACACAGACAGTAGGCTTCTTCGTGCAGATGCAATGGTCCAAGGTCACCAATAAGATA
GACCTGATTGCTGTCTATCATCCCCAATACGGCTTCTACTGTGCCTATGGGAGACCCTGTGAGTCACTTGTGAC
TTTCACAGAAACTCCTGAGAATGGGTCAAAATGGACTCTGCACTTAAGGAATATGTCTTGTTCAGTCAGTGGA
AGGTACGAGTGTATGCTTGTTCTGTATCCAGAGGGCATTCAGACTAAAATCTACAACCTTCTCATTCAGACACA
CGTTACAGCAGATGAATGGAACAGCAACCATACGATAGAAATAGAGATAAATCAGACTCTGGAAATACCATG
CTTTCAAAATAGCTCCTCAAAAATTTCATCTGAGTTCACCTATGCATGGTCGGTGGAGGATAATGGAACTCAG
GAAACACTTATCTCCCAAAATCACCTCATCAGCAATTCCACATTACTTAAAGATAGAGTCAAGCTTGGTACAGA
CTACAGACTCCACCTCTCTCCAGTCCAAATCTTCGATGATGGGCGGAAGTTCTCTTGCCACATTAGAGTCGGTC
CTAACAAAATCTTGAGGAGCTCCACCACAGTCAAGGTTTTTGCTAAACCAGAAATCCCTGTGATTGTGGAAAA
TAACTCCACGGATGTCTTGGTAGAGAGAAGATTTACCTGCTTACTAAAGAATGTATTTCCCAAAGCAAATATCA
CATGGTTTATAGATGGAAGTTTTCTTCATGATGAAAAAGAAGGAATATATATTACTAATGAAGAGAGAAAAG
GCAAAGATGGATTTTTGGAACTGAAGTCTGTTTTAACAAGGGTACATAGTAATAAACCAGCCCAATCAGACAA
CTTGACCATTTGGTGTATGGCTCTGTCTCCAGTCCCAGGAAATAAAGTGTGGAACATCTCATCAGAAAAGATC
ACTTTTCTCTTAGGTTCTGAAATTTCCTCAACAGACCCTCCACTGAGTGTTACAGAATCTACCCTTGACACCCAA
CCTTCTCCAGCCAGCAGTGTATCTCCTGCAAGTAAGAATGTTTTCACACTGAGCTATTGATTTAACCAAGCAGA
TTGATAACGATAAAATTTCAGCAAACTTGCATCATTCATGCCTGTTTCTTAGCTATGACTTTTTGGGGCTGAAA
TTGGGTTTTATTTTTTAACAGCTTTATTGAGATATAATTCACGTCACATTAACTTGCCCATTTAAAATGTATGATC
TAATGGTTTTTAGTATGTTCACAGACTTGTACAACCATGACTACAGCCAATTTCAGAACATTTTCATCATCTAAT
AAAGAAACTCCTAAACAAAAAAAAAAAAAAAA
SEQ ID NO. 8 Homo sapiens CD96 molecule (CD96), transcript variant 3 (mRNA) NM_001318889.1 
ATGGAGAAAAAATGGAAATACTGTGCTGTCTATTACATCATCCAGATACATTTTGTCAAGGGAGTTTGGGAAA
AAACAGTCAACACAGAAGAAAATGTTTATGCTACACTTGGCTCTGATGTCAACCTGACCTGCCAAACACAGAC
AGTAGGCTTCTTCGTGCAGATGCAATGGTCCAAGGTCACCAATAAGATAGACCTGATTGCTGTCTATCATCCCC
AATACGGCTTCTACTGTGCCTATGGGAGACCCTGTGAGTCACTTGTGACTTTCACAGAAACTCCTGAGAATGG
GTCAAAATGGACTCTGCACTTAAGGAATATGTCTTGTTCAGTCAGTGGAAGGTACGAGTGTATGCTTGTTCTG
TATCCAGAGGGCATTCAGACTAAAATCTACAACCTTCTCATTCAGACACACGTTACAGCAGATGAATGGAACA
GCAACCATACGATAGAAATAGAGATAAATCAGACTCTGGAAATACCATGCTTTCAAAATAGCTCCTCAAAAAT
TTCATCTGAGTTCACCTATGCATGGTCGGTGGAGGATAATGGAACTCAGGAAACACTTATCTCCCAAAATCAC
CTCATCAGCAATTCCACATTACTTAAAGATAGAGTCAAGCTTGGTACAGACTACAGACTCCACCTCTCTCCAGT
CCAAATCTTCGATGATGGGCGGAAGTTCTCTTGCCACATTAGAGTCGGTCCTAACAAAATCTTGAGGAGCTCC
ACCACAGTCAAGGTTTTTGCTAAACCAGAAATCCCTGTGATTGTGGAAAATAACTCCACGGATGTCTTGGTAG
AGAGAAGATTTACCTGCTTACTAAAGAATGTATTTCCCAAAGCAAATATCACATGGTTTATAGATGGAAGTTTT
CTTCATGATGAAAAAGAAGGAATATATATTACTAATGAAGAGAGAAAAGGCAAAGATGGATTTTTGGAACTG
AAGTCTGTTTTAACAAGGGTACATAGTAATAAACCAGCCCAATCAGACAACTTGACCATTTGGTGTATGGCTCT
 
GTCTCCAGTCCCAGGAAATAAAGTGTGGAACATCTCATCAGAAAAGATCACTTTTCTCTTAGGTTCTGAAATTT
CCTCAACAGACCCTCCACTGAGTGTTACAGAATCTACCCTTGACACCCAACCTTCTCCAGCCAGCAGTGTATCT
CCTGCAAGTAAGAATGTTTTCACACTGAGCTATTGA
SEQ ID NO. 9 Homo sapiens CD96 molecule (CD96), transcript variant 3 (protein) NP_001305818.1 
MEKKWKYCAVYYIIQIHFVKGVWEKTVNTEENVYATLGSDVNLTCQTQTVGFFVQMQWSKVTNKIDLIAVYHPQ
YGFYCAYGRPCESLVTFTETPENGSKWTLHLRNMSCSVSGRYECMLVLYPEGIQTKIYNLLIQTHVTADEWNSNHTI
EIEINQTLEIPCFQNSSSKISSEFTYAWSVEDNGTQETLISQNHLISNSTLLKDRVKLGTDYRLHLSPVQIFDDGRKFSC
HIRVGPNKILRSSTTVKVFAKPEIPVIVENNSTDVLVERRFTCLLKNVFPKANITWFIDGSFLHDEKEGIYITNEERKGK
DGFLELKSVLTRVHSNKPAQSDNLTIWCMALSPVPGNKVWNISSEKITFLLGSEISSTDPPLSVTESTLDTQPSPASSV
SPASKNVFTLSY
SEQ ID NO. 10 Homo sapiens nectin cell adhesion molecule 2 (CD112/NECTIN2), transcript variant alpha (nucleotide) NM_002856 
AGAATTCTTTGGCAGGGGCGACCTTAGAATCCTGGGGAGGAGCGAGAATGGAATCCCGGGGAGGAACAGG
GGTGGAATCCGGGGGGCGGGGTCAGAACGCCAGGAGGGGGCGGGGCCGGAGCCAGGGTCGGCTTGACTC
GGGGGAGCAGCGGGTGGATCCTGTGACGTCAGCGGGTTCGAACCGCCGGAGCTGAGCGAGAGGCGGGGG
TGCCGAGCCGGGCGGGGAGAGCTGGGCCGGGAGAGCAGAACAGGGAGGCTAGAGCGCAGCGGGAACCGG
CCCGGAGCCGGAGCCGGAGCCCCACAGGCACCTACTAAACCGCCCAGCCGATCGGCCCCCACAGAGTGGCCC
GCGGGCCTCCGGCCGGGCCCAGTCCCCTCCCGGGCCCTCCATGGCCCGGGCCGCTGCCCTCCTGCCGTCGAG
ATCGCCGCCGACGCCGCTGCTGTGGCCGCTGCTGCTGCTGCTGCTCCTGGAAACCGGAGCCCAGGATGTGCG
AGTTCAAGTGCTACCCGAGGTGCGAGGCCAGCTCGGGGGCACCGTGGAGCTGCCGTGCCACCTGCTGCCACC
TGTTCCTGGACTGTACATCTCCCTGGTGACCTGGCAGCGCCCAGATGCACCTGCGAACCACCAGAATGTGGCC
GCCTTCCACCCTAAGATGGGTCCCAGCTTCCCCAGCCCGAAGCCTGGCAGCGAGCGGCTGTCCTTCGTCTCTG
CCAAGCAGAGCACTGGGCAAGACACAGAGGCAGAGCTCCAGGACGCCACGCTGGCCCTCCACGGGCTCACG
GTGGAGGACGAGGGCAACTACACTTGCGAGTTTGCCACCTTCCCCAAGGGGTCCGTCCGAGGGATGACCTGG
CTCAGAGTCATAGCCAAGCCCAAGAACCAAGCTGAGGCCCAGAAGGTCACGTTCAGCCAGGACCCTACGACA
GTGGCCCTCTGCATCTCCAAAGAGGGCCGCCCACCTGCCCGGATCTCCTGGCTCTCATCCCTGGACTGGGAAG
CCAAAGAGACTCAGGTGTCAGGGACCCTGGCCGGAACTGTCACTGTCACCAGCCGCTTCACCTTGGTGCCCTC
GGGCCGAGCAGATGGTGTCACGGTCACCTGCAAAGTGGAGCATGAGAGCTTCGAGGAACCAGCCCTGATAC
CTGTGACCCTCTCTGTACGCTACCCTCCTGAAGTGTCCATCTCCGGCTATGATGACAACTGGTACCTCGGCCGT
ACTGATGCCACCCTGAGCTGTGACGTCCGCAGCAACCCAGAGCCCACGGGCTATGACTGGAGCACGACCTCA
GGCACCTTCCCGACCTCCGCAGTGGCCCAGGGCTCCCAGCTGGTCATCCACGCAGTGGACAGTCTGTTCAATA
CCACCTTCGTCTGCACAGTCACCAATGCCGTGGGCATGGGCCGCGCTGAGCAGGTCATCTTTGTCCGAGAAAC
CCCCAGGGCCTCGCCCCGAGATGTGGGCCCGCTGGTGTGGGGGGCCGTGGGGGGGACACTGCTGGTGCTGC
TGCTTCTGGCTGGGGGGTCCTTGGCCTTCATCCTGCTGAGGGTGAGGAGGAGGAGGAAGAGCCCTGGAGGA
GCAGGAGGAGGAGCCAGTGGCGACGGGGGATTCTACGATCCGAAAGCTCAGGTGTTGGGAAATGGGGACC
CCGTCTTCTGGACACCAGTAGTCCCTGGTCCCATGGAACCAGATGGCAAGGATGAGGAGGAGGAGGAGGAG
GAAGAGAAGGCAGAGAAAGGCCTCATGTTGCCTCCACCCCCAGCACTCGAGGATGACATGGAGTCCCAGCTG
GACGGCTCCCTCATCTCACGGCGGGCAGTTTATGTGTGACCTGGACACAGACAGAGACAGAGCCAGGCCCGG
CCCTCCCGCCCCCGACCTGACCACGCCGGCCTAGGGTTCCAGACTGGTTGGACTTGTTCGTCTGGACGACACT
GGAGTGGAACACTGCCTCCCACTTTCTTGGGACTTGGAGGGAGGTGGAACAGCACACTGGACTTCTCCCGTCT
CTAGGGCTGCATGGGGAGCCCGGGGAGCTGAGTAGTGGGGATCCAGAGAGGACCCCCGCCCCCAGAGACTT
GGTTTTGGCTCCAGCCTTCCCCTGGCCCCGTGACACTCAGGAGTTAATAAATGCCTTGGAGGAAAACATCAAA
AAAAAAAAAAAAA
SEQ ID NO. 11 Homo sapiens nectin cell adhesion molecule 2 (CD112/NECTIN2), transcript variant alpha (mRNA) NM_002856.2 
ATGGCCCGGGCCGCTGCCCTCCTGCCGTCGAGATCGCCGCCGACGCCGCTGCTGTGGCCGCTGCTGCTGCTGC
TGCTCCTGGAAACCGGAGCCCAGGATGTGCGAGTTCAAGTGCTACCCGAGGTGCGAGGCCAGCTCGGGGGC
ACCGTGGAGCTGCCGTGCCACCTGCTGCCACCTGTTCCTGGACTGTACATCTCCCTGGTGACCTGGCAGCGCC
 
CAGATGCACCTGCGAACCACCAGAATGTGGCCGCCTTCCACCCTAAGATGGGTCCCAGCTTCCCCAGCCCGAA
GCCTGGCAGCGAGCGGCTGTCCTTCGTCTCTGCCAAGCAGAGCACTGGGCAAGACACAGAGGCAGAGCTCCA
GGACGCCACGCTGGCCCTCCACGGGCTCACGGTGGAGGACGAGGGCAACTACACTTGCGAGTTTGCCACCTT
CCCCAAGGGGTCCGTCCGAGGGATGACCTGGCTCAGAGTCATAGCCAAGCCCAAGAACCAAGCTGAGGCCCA
GAAGGTCACGTTCAGCCAGGACCCTACGACAGTGGCCCTCTGCATCTCCAAAGAGGGCCGCCCACCTGCCCG
GATCTCCTGGCTCTCATCCCTGGACTGGGAAGCCAAAGAGACTCAGGTGTCAGGGACCCTGGCCGGAACTGT
CACTGTCACCAGCCGCTTCACCTTGGTGCCCTCGGGCCGAGCAGATGGTGTCACGGTCACCTGCAAAGTGGAG
CATGAGAGCTTCGAGGAACCAGCCCTGATACCTGTGACCCTCTCTGTACGCTACCCTCCTGAAGTGTCCATCTC
CGGCTATGATGACAACTGGTACCTCGGCCGTACTGATGCCACCCTGAGCTGTGACGTCCGCAGCAACCCAGAG
CCCACGGGCTATGACTGGAGCACGACCTCAGGCACCTTCCCGACCTCCGCAGTGGCCCAGGGCTCCCAGCTG
GTCATCCACGCAGTGGACAGTCTGTTCAATACCACCTTCGTCTGCACAGTCACCAATGCCGTGGGCATGGGCC
GCGCTGAGCAGGTCATCTTTGTCCGAGAAACCCCCAGGGCCTCGCCCCGAGATGTGGGCCCGCTGGTGTGGG
GGGCCGTGGGGGGGACACTGCTGGTGCTGCTGCTTCTGGCTGGGGGGTCCTTGGCCTTCATCCTGCTGAGGG
TGAGGAGGAGGAGGAAGAGCCCTGGAGGAGCAGGAGGAGGAGCCAGTGGCGACGGGGGATTCTACGATC
CGAAAGCTCAGGTGTTGGGAAATGGGGACCCCGTCTTCTGGACACCAGTAGTCCCTGGTCCCATGGAACCAG
ATGGCAAGGATGAGGAGGAGGAGGAGGAGGAAGAGAAGGCAGAGAAAGGCCTCATGTTGCCTCCACCCCC
AGCACTCGAGGATGACATGGAGTCCCAGCTGGACGGCTCCCTCATCTCACGGCGGGCAGTTTATGTGTGA
SEQ ID NO. 12 Homo sapiens nectin cell adhesion molecule 2 (CD112/NECTIN2), transcript variant alpha (protein) NP_002847.1 
MARAAALLPSRSPPTPLLWPLLLLLLLETGAQDVRVQVLPEVRGQLGGTVELPCHLLPPVPGLYISLVTWQRPDAPA
NHQNVAAFHPKMGPSFPSPKPGSERLSFVSAKQSTGQDTEAELQDATLALHGLTVEDEGNYTCEFATFPKGSVRG
MTWLRVIAKPKNQAEAQKVTFSQDPTTVALCISKEGRPPARISWLSSLDWEAKETQVSGTLAGTVTVTSRFTLVPS
GRADGVTVTCKVEHESFEEPALIPVTLSVRYPPEVSISGYDDNWYLGRTDATLSCDVRSNPEPTGYDWSTTSGTFPT
SAVAQGSQLVIHAVDSLFNTTFVCTVTNAVGMGRAEQVIFVRETPRASPRDVGPLVWGAVGGTLLVLLLLAGGSL
AFILLRVRRRRKSPGGAGGGASGDGGFYDPKAQVLGNGDPVFWTPVVPGPMEPDGKDEEEEEEEEKAEKGLMLP
PPPALEDDMESQLDGSLISRRAVYV
SEQ ID NO. 13 Homo sapiens nectin cell adhesion molecule 2 (CD112/NECTIN2), transcript variant delta (nucleotide) NM_001042724 
AGAATTCTTTGGCAGGGGCGACCTTAGAATCCTGGGGAGGAGCGAGAATGGAATCCCGGGGAGGAACAGG
GGTGGAATCCGGGGGGCGGGGTCAGAACGCCAGGAGGGGGCGGGGCCGGAGCCAGGGTCGGCTTGACTC
GGGGGAGCAGCGGGTGGATCCTGTGACGTCAGCGGGTTCGAACCGCCGGAGCTGAGCGAGAGGCCGGGGG
TGCCGAGCCGGGCGGGGAGAGCTGGGCCGGGAGAGCAGAACAGGGAGGCTAGAGCGCAGCGGGAACCGG
CCGGAGCCGGAGCCGGAGCCCCACAGGCACCTACTAAACCGCCCAGCCGATCGGCCCCCACAGAGTGGCCCG
CGGGCCTCCGGCCGGGCCCAGTCCCCTCCCGGGCCCTCCATGGCCCGGGCCGCTGCCCTCCTGCCGTCGAGAT
CGCCGCCGACGCCGCTGCTGTGGCCGCTGCTGCTGCTGCTCCTGGAAACCGGAGCCCAGGATGTGCGAG
TTCAAGTGCTACCCGAGGTGCGAGGCCAGCTCGGGGGCACCGTGGAGCTGCCGTGCCACCTGCTGCCACCTG
TTCCTGGACTGTACATCTCCCTGGTGACCTGGCAGCGCCCAGATGCACCTGCGAACCACCAGAATGTGGCCGC
CTTCCACCCTAAGATGGGTCCCAGCTTCCCCAGCCCGAAGCCTGGCAGCGAGCGGCTGTCCTTCGTCTCTGCCA
AGCAGAGCACTGGGCAAGACACAGAGGCAGAGCTCCAGGACGCCACGCTGGCCCTCCACGGGCTCACGGTG
GAGGACGAGGGCAACTACACTTGCGAGTTTGCCACCTTCCCCAAGGGGTCCGTCCGAGGGATGACCTGGCTC
AGAGTCATAGCCAAGCCCAAGAACCAAGCTGAGGCCCAGAAGGTCACGTTCAGCCAGGACCCTACGACAGTG
GCCCTCTGCATCTCCAAAGAGGGCCGCCCACCTGCCCGGATCTCCTGGCTCTCATCCCTGGACTGGGAAGCCA
AAGAGACTCAGGTGTCAGGGACCCTGGCCGGAACTGTCACTGTCACCAGCCGCTTCACCTTGGTGCCCTCGGG
CCGAGCAGATGGTGTCACGGTCACCTGCAAAGTGGAGCATGAGAGCTTCGAGGAACCAGCCCTGATACCTGT
GACCCTCTCTGTACGCTACCCTCCTGAAGTGTCCATCTCCGGCTATGATGACAACTGGTACCTCGGCCGTACTG
ATGCCACCCTGAGCTGTGACGTCCGCAGCAACCCAGAGCCCACGGGCTATGACTGGAGCACGACCTCAGGCA
CCTTCCCGACCTCCGCAGTGGCCCAGGGCTCCCAGCTGGTCATCCACGCAGTGGACAGTCTGTTCAATACCAC
CTTCGTCTGCACAGTCACCAATGCCGTGGGCATGGGCCGCGCTGAGCAGGTCATCTTTGTCCGAGAGACCCCC
AACACAGCAGGCGCAGGGGCCACAGGCGGCATCATCGGGGGCATCATCGCCGCCATCATTGCTACTGCTGTG
 
GCTGCCACGGGCATCCTTATCTGCCGGCAGCAGCGGAAGGAGCAGACGCTGCAGGGGGCAGAGGAGGACG
AAGACCTGGAGGGACCTCCCTCCTACAAGCCACCGACCCCAAAAGCGAAGCTGGAGGCACAGGAGATGCCCT
CCCAGCTCTTCACTCTGGGGGCCTCGGAGCACAGCCCACTCAAGACCCCCTACTTTGATGCTGGCGCCTCATGC
ACTGAGCAGGAAATGCCTCGATACCATGAGCTGCCCACCTTGGAAGAACGGTCAGGACCCTTGCACCCTGGA
GCCACAAGCCTGGGGTCCCCCATCCCGGTGCCTCCAGGGCCACCTGCTGTGGAAGACGTTTCCCTGGATCTAG
AGGATGAGGAGGGGGAGGAGGAGGAAGAGTATCTGGACAAGATCAACCCCATCTATGATGCTCTGTCCTAT
AGCAGCCCCTCTGATTCCTACCAGGGCAAAGGCTTTGTCATGTCCCGGGCCATGTATGTGTGAGCTGCCATGC
GCCTGGCGTCTCACATCTCACCTGTTGATCCCTTAGCTTTCTTGCCAAGGATCTAGTGCCCCCTGACCTCTGGCC
AGGCCACTGTCAGTTAACACATATGCATTCCATTTGTGATGTCTACCTTGGTGGCTCCACTATGACCCCTAACCC
ATGAGCCCAGAGAAATTCACCGTGATAATGGAATCCTGGCAACCTTATCTCATGAGGCAGGAGGTGGGGAAG
GTGCTTCTGCACAACCTCTGATCCCAAGGACTCCTCTCCCAGACTGTGACCTTAGACCATACCTCTCACCCCCCA
ATGCCTCGACTCCCCCAAAATCACAAAGAAGACCCTAGACCTATAATTTGTCTTCAGGTAGTAAATTCCCAATA
GGTCTGCTGGAGTGGGCGCTGAGGGCTCCCTGCTGCTCAGACCTGAGCCCTCCAGGCAGCAGGGTCCCACTT
ACCCCCTCCCCACCCTGTTCCCCAAAGGTGGGAAAGAGGGGATTCCCCAGCCCAAGGCAGGGTTTTCCCAGCA
CCCTCCTGTAAGCAGAAGTCTCAGGGTCCAGACCCTTCCCTGAGCCCCCACCCCCACCCCAATTCCTGCCTACC
AAGCAAGCAGCCCCAGCCTAGGGTCAGACAGGGTGAGCCTCATACAGACTGTGCCTTGATGGCCCCAGCCTT
GGGAGAAGAATTTACTGTTAACCTGGAAGACTACTGAATCATTTTACCCTTGCCCAGTGGAATAGGACCTAAA
CATCCCCCTTCCGGGGAAAGTGGGTCATCTGAATTGGGGGTAGCAATTGATACTGTTTTGTAAACTACATTTCC
TACAAAATATGAATTTATACTTTGACCAGGAAAAAAAAAAAAA
Homo sapiens nectin cell adhesion molecule 2 (CD112/NECTIN2), transcript variant delta (mRNA) NM_001042724.1 
ATGGCCCGGGCCGCTGCCCTCCTGCCGTCGAGATCGCCGCCGACGCCGCTGCTGTGGCCGCTGCTGCTGCTGC
TGCTCCTGGAAACCGGAGCCCAGGATGTGCGAGTTCAAGTGCTACCCGAGGTGCGAGGCCAGCTCGGGGGC
ACCGTGGAGCTGCCGTGCCACCTGCTGCCACCTGTTCCTGGACTGTACATCTCCCTGGTGACCTGGCAGCGCC
CAGATGCACCTGCGAACCACCAGAATGTGGCCGCCTTCCACCCTAAGATGGGTCCCAGCTTCCCCAGCCCGAA
GCCTGGCAGCGAGCGGCTGTCCTTCGTCTCTGCCAAGCAGAGCACTGGGCAAGACACAGAGGCAGAGCTCCA
GGACGCCACGCTGGCCCTCCACGGGCTCACGGTGGAGGACGAGGGCAACTACACTTGCGAGTTTGCCACCTT
CCCCAAGGGGTCCGTCCGAGGGATGACCTGGCTCAGAGTCATAGCCAAGCCCAAGAACCAAGCTGAGGCCCA
GAAGGTCACGTTCAGCCAGGACCCTACGACAGTGGCCCTCTGCATCTCCAAAGAGGGCCGCCCACCTGCCCG
GATCTCCTGGCTCTCATCCCTGGACTGGGAAGCCAAAGAGACTCAGGTGTCAGGGACCCTGGCCGGAACTGT
CACTGTCACCAGCCGCTTCACCTTGGTGCCCTCGGGCCGAGCAGATGGTGTCACGGTCACCTGCAAAGTGGAG
CATGAGAGCTTCGAGGAACCAGCCCTGATACCTGTGACCCTCTCTGTACGCTACCCTCCTGAAGTGTCCATCTC
CGGCTATGATGACAACTGGTACCTCGGCCGTACTGATGCCACCCTGAGCTGTGACGTCCGCAGCAACCCAGAG
CCCACGGGCTATGACTGGAGCACGACCTCAGGCACCTTCCCGACCTCCGCAGTGGCCCAGGGCTCCCAGCTG
GTCATCCACGCAGTGGACAGTCTGTTCAATACCACCTTCGTCTGCACAGTCACCAATGCCGTGGGCATGGGCC
GCGCTGAGCAGGTCATCTTTGTCCGAGAGACCCCCAACACAGCAGGCGCAGGGGCCACAGGCGGCATCATCG
GGGGCATCATCGCCGCCATCATTGCTACTGCTGTGGCTGCCACGGGCATCCTTATCTGCCGGCAGCAGCGGAA
GGAGCAGACGCTGCAGGGGGCAGAGGAGGACGAAGACCTGGAGGGACCTCCCTCCTACAAGCCACCGACCC
CAAAAGCGAAGCTGGAGGCACAGGAGATGCCCTCCCAGCTCTTCACTCTGGGGGCCTCGGAGCACAGCCCAC
TCAAGACCCCCTACTTTGATGCTGGCGCCTCATGCACTGAGCAGGAAATGCCTCGATACCATGAGCTGCCCAC
CTTGGAAGAACGGTCAGGACCCTTGCACCCTGGAGCCACAAGCCTGGGGTCCCCCATCCCGGTGCCTCCAGG
GCCACCTGCTGTGGAAGACGTTTCCCTGGATCTAGAGGATGAGGAGGGGGAGGAGGAGGAAGAGTATCTGG
ACAAGATCAACCCCATCTATGATGCTCTGTCCTATAGCAGCCCCTCTGATTCCTACCAGGGCAAAGGCTTTGTC
ATGTCCCGGGCCATGTATGTGTGA
SEQ ID NO. 15 Homo sapiens nectin cell adhesion molecule 2 (CD112/NECTIN2), transcript variant delta (protein) NP_001036189 
MARAAALLPSRSPPTPLLWPLLLLLLLETGAQDVRVQVLPEVRGQLGGTVELPCHLLPPVPGLYISLVTWQRPDAPA
NHQNVAAFHPKMGPSFPSPKPGSERLSFVSAKQSTGQDTEAELQDATLALHGLTVEDEGNYTCEFATFPKGSVRG
MTWLRVIAKPKNQAEAQKVTFSQDPTTVALCISKEGRPPARISWLSSLDWEAKETQVSGTLAGTVTVTSRFTLVPS
 
GRADGVTVTCKVEHESFEEPALIPVTLSVRYPPEVSISGYDDNWYLGRTDATLSCDVRSNPEPTGYDWSTTSGTFPT
SAVAQGSQLVIHAVDSLFNTTFVCTVTNAVGMGRAEQVIFVRETPNTAGAGATGGIIGGIIAAIIATAVAATGILICR
QQRKEQTLQGAEEDEDLEGPPSYKPPTPKAKLEAQEMPSQLFTLGASEHSPLKTPYFDAGASCTEQEMPRYHELPT
LEERSGPLHPGATSLGSPIPVPPGPPAVEDVSLDLEDEEGEEEEEYLDKINPIYDALSYSSPSDSYQGKGFVMSRAMY
V
SEQ ID NO. 16 Homo sapiens poliovirus receptor (CD155/PVR), transcript variant 1 (nucleotide) NM_006505 
AGCGGGACTGAGCGCCGGGAGAGACCTGCGCAGGCGCAGGCGCGGGGAGGGCCAGCCTGGTGGCCCA
CCCCGCGCCTGGCGGGACTGGCCGCCAACTCCCCTCCGCTCCAGTCACTTGTCTGGAGCTTGAAGAAGTGGGT
ATTCCCCTTCCCACCCCAGGCACTGGAGGAGCGGCCCCCGGGGATTCCAGGACCTGAGCTCCGGGAGCTGG
ACTCGCAGCGACCGCGGCAGAGCGAGCGGGCGCCGGGAAGCGAGGAGACGCCCGCGGGAGGCCCAGCTGC
TCGGAGCAACTGGCATGGCCCGAGCCATGGCCGCCGTGGCCGCTGCTGCTGGTGGCGCTACTGGTGCTGT
CCTGGCCACCCCCAGGAACCGGGGACGTCGTCGTGCAGGCGCCCACCCAGGTGCCCGGCTTCTTGGGCGACT
CCGTGACGCTGCCCTGCTACCTACAGGTGCCCAACATGGAGGTGACGCATGTGTCACAGCTGACTTGGGCGC
GGCATGGTGAATCTGGCAGCATGGCCGTCTTCCACCAAACGCAGGGCCCCAGCTATTCGGAGTCCAAACGGC
TGGAATTCGTGGCAGCCAGACTGGGCGCGGAGCTGCGGAATGCCTCGCTGAGGATGTTCGGGTTGCGCGTA
GAGGATGAAGGCAACTACACCTGCCTGTTCGTCACGTTCCCGCAGGGCAGCAGGAGCGTGGATATCTGGCTC
CGAGTGCTTGCCAAGCCCCAGAACACAGCTGAGGTTCAGAAGGTCCAGCTCACTGGAGAGCCAGTGCCCATG
GCCCGCTGCGTCTCCACAGGGGGTCGCCCGCCAGCCCAAATCACCTGGCACTCAGACCTGGGCGGGATGCCC
AATACGAGCCAGGTGCCAGGGTTCCTGTCTGGCACAGTCACTGTCACCAGCCTCTGGATATTGGTGCCCTCAA
GCCAGGTGGACGGCAAGAATGTGACCTGCAAGGTGGAGCACGAGAGCTTTGAGAAGCCTCAGCTGCTGACT
GTGAACCTCACCGTGTACTACCCCCCAGAGGTATCCATCTCTGGCTATGATAACAACTGGTACCTTGGCCAGAA
TGAGGCCACCCTGACCTGCGATGCTCGCAGCAACCCAGAGCCCACAGGCTATAATTGGAGCACGACCATGGG
TCCCCTGCCACCCTTTGCTGTGGCCCAGGGCGCCCAGCTCCTGATCCGTCCTGTGGACAAACCAATCAACACAA
CTTTAATCTGCAACGTCACCAATGCCCTAGGAGCTCGCCAGGCAGAACTGACCGTCCAGGTCAAAGAGGGAC
CTCCCAGTGAGCACTCAGGCATGTCCCGTAACGCCATCATCTTCCTGGTTCTGGGAATCCTGGTTTTTCTGATC
CTGCTGGGGATCGGGATTTATTTCTATTGGTCCAAATGTTCCCGTGAGGTCCTTTGGCACTGTCATCTGTGTCC
CTCGAGTACAGAGCATGCCAGCGCCTCAGCTAATGGGCATGTCTCCTATTCAGCTGTGAGCAGAGAGAACAG
CTCTTCCCAGGATCCACAGACAGAGGGCACAAGGTGACAGCGTCGGGACTGAGAGGGGAGAGAGACTGGA
GCTGGCAAGGACGTGGGCCTCCAGAGTTGGACCCGACCCCAATGGATGAAGACCCCCTCCAAAGAGACCAGC
CTCCCTCCCTGTGCCAGACCTCAAAACGACGGGGGCAGGTGCAAGTTCATAGGTCTCCAAGACCACCCTCCTT
TCATTTGCTAGAAGGACTCACTAGACTCAGGAAAGCTGTTAGGCTCACAGTTACAGTTTATTACAGTAAAAGG
ACAGAGATTAAGATCAGCAAAGGGAGGAGGTGCACAGCACACGTTCCACGACAGATGAGGCGACGGCTTCC
ATCTGCCCTCTCCCAGTGGAGCCATATAGGCAGCACCTGATTCTCACAGCAACATGTGACAACATGCAAGAAG
TACTGCCAATACTGCCAACCAGAGCAGCTCACTCGAGATCTTTGTGTCCAGAGTTTTTTGTTTGTCTTGAGACA
GGGTCTGGCTCTGTTGGCAGACTAGAGTACAGTGGTGAGATCACAGTTCATTGCAGCCTTGACTTCTCAACGC
CAAGTCATCCTCCCACCTCAGCCTCCTGAGTAGCTATGACTACAGGTATGTGCCACCACGTCTGGCTAATCTTTT
TATTATTTGTAAAGTCGAGGTTTCCCTGTGTTGCCCAGGCTGGTCTTGAACTCTTGGCTCCAAGTGATACTTCT
GCCTTGGCCTCCCAAAGTGCTGAATTAAGCAGCTCACCATCCACACGGCTGACCTCATACATCAAGCCAATACC
GTGTGGCCCAAGACCCCCACCATAAATCACATCATTAGCATGAACCACCCAGAGTGGCCCAAGACTCCAAGAT
CAGCTACCAGGCAGGATATTCCAAGGGCTTAGAGATGAATGCCCAGGAGCTGAGGATAAAGGGCCCGATCTT
TCTTTGGGCAAGGTTAAGCCTTTACTGCATAGCAGACCACACAGAAGGGTGTGGGCCACCAGAGAATTTTGGT
AAAAATTTGGCCTCTGGCCTTGAGCTTCTAAATCTCTGTATCCGTCAGATCTCTGTGGTTACAAGAAACAGCCA
CTGACCCTGGTCACCAGAGGCTGCAATTCAGGCCGCAAGCAGCTGCCTGGGGGGTGTCCAAGGAGCAGAGA
AAACTACTAGATGTGAACTTGAAGAAGGTTGTCAGCTGCAGCCACTTTCTGCCAGCATCTGCAGCCACTTTCTG
CCAGCATCTGCAGCCAGCAAGCTGGGACTGGCAGGAAATAACCCACAAAAGAAGCAAATGCAATTTCCAACA
CAAGGGGGAAGGGATGCAGGGGGAGGCAGCGCTGCAGTTGCTCAGGACACGCTCCTATAGGACCAAGATG
GATGCGACCCAAGACCCAGGAGGCCCAGCTGCTCAGTGCAACTGACAAGTTAAAAAGGTCTATGATCTTGAG
GGCAGACAGCAGAATTCCTCTTATAAAGAAAACTGTTTGGGAAAATACGTTGAGGGAGAGAAGACCTTGGGC
CAAGATGCTAAATGGGAATGCAAAGCTTGAGCTGCTCTGCAAGAGAAAATAAGCAGGACAGAGGATTTGCTC
 
TGGACAGAGATGGAAGAGCCGGGAACAGAGAAGTGTGGGGAAGAGATAGGAACCAGCAGGATGGCAGGG
GCAAAGGGCTCAAGGGTGAGGAGGCCAGTGGACCCCACAGAGTTGGGGAGATAAAGGAACATTGGTTGCT
TTGGTGGCACGTAAGCTCCTTGTCTGTCTCCAGCACCCAGAATCTCATTAAAGCTTATTTATTGTACCTCCAGCG
GCTGTGTGCAATGGGGTCTTTGTGGAAATCAAGGAGCAGACAGGTTTCATGTGTACTGTCACCACGTGGGA
TGGAACCAGAGGCATGGAAGCAAGACGCTAAATGAAGAGGGCCATAAGGGCTGGGATTCCCAGGCACCTTA
GGAACAGCTTGTCTTTTTTTTTTTCCTCTCCAAAAAAAATGTTTAAGGGACGGTGTCTCCTGTCACCCAGGCTG
GAGTGCAATGGCACGATCATAGCTCATTGCAGCCTCTAACTCCGGGGCTCAAGCAATCCTCCCACCTCAGCCT
ACCAAGTAGCTGTGACCACAGCTGCCCCTCACCATGCTAAGCTAATTTTTTTAATTAGATAGTACATAAACGTC
CCAAAATTAGAAGATAAAAAGACATGAGGGATCCATTCTAATTTGTGTTTGGAGTGTAATGGTCCAGCTCCAT
TCTTCTGCACATGGATATCCAGTTTTACACAACACTGTGAATGTAATGAATGCCACTGAATCATACACTCAAAA
ATAGCTAAAATGGCAAATTGTCTGTTATCTCTTTTTAACCACCATTTTTGAAAATTAATTATACCAAAAAACCAT
TGAATAGTGCACTTTATTTATTTATTTATTTGTTTATTTATTTATTTATTTTAGAAATAAGAGTCTCACTTTGTTGC
CCAGGCTGGAGTGCAGTGGCGTGATCATGGCTCATTGCAGCCTCGACCTGCTGGGCTCGGGCTATCCTTCCAT
CTCAGCCTCCCGAGTAGCTGGGACTATAGGTGGGCGCCACCCCACCTGGCTAAATCTCTTTTTAACTTTTGTAG
AGATAGGCATCTCGCTATGTTGCCTAGGCTGGGCTGGAACTCCTGGGCTCAAGTGCTCCTCCTGCCTTGGCCT
CCCAAAGCGCTAGGATTACAGATGTGAGCCACCGCGCCCACCCTGAACCTTACTTTTTTTGCTCAGTTTCTGGT
AATTCAGAGAATGCCTCCTGAGTTGTTCTACACCCACCTCATATTCCATGGGAGGGCTGTACAGGGCTTTTTTA
ACGAGGCCTCTAAGGACAGGCATTTGTATCCTTTCCAGCCTTTCACTATTACAATGTTGTAGTGAATAACTTTAC
ACACTGTCATTTATTTTACTTTTTTTTTTTTTTTATTTTAGAGAAAGGAATCTTGCCATCTTGCCCAGGCTGGTCTC
AAATTCCTGGGCCCAAACAATCCTCCCGCCTTGGCCTCCTAAAGTACTGGGATTTATAGGCATAAGCCACCGTG
CCTGGCCAATGCACACTGTCATTTAGCTCATGTTAACACCTGAGTGTAGGACACACTCCTGGAGGTGGAATTG
CTGGGCCAAAGAGTATGTTTCTTGTCATTGTGATAGATATTGACAAATGAACCCTCACAGAAGTTGTGCTGAG
TTCTGTTCCCACCAGCGACGTAGGCGATGACCTTTTTCTGGAGGGAGGGGGCATCCTTGGAGTCCACAGAGCC
AGGAATGGAGAGTGGGCCCAGAATTTTGGTATAGGTGTTGTATAAACTTATAGTAAGGTTAAGAAAACCGCA
ACTATCCTTATCAGAGACTTGGCGGGGGGCAGGGTATGATGGAGATCATAAGGAGGCTAAAACACTCCACAC
CCTCCCTCTGCATTGCTCCTGCACGGGAGTCGGGAATCTTTTCAGGTTGATACGATCTCACCTTGAGGAGCTGT
GAGGTCCCAGAAGCCTCTGGGTTGCAGATTGCTTGGGGTGAAAATGTCTGTGCTACTGAAATCTAACTTTTTA
CAAAAAATTACGGGCTGGGCGCAGTGGCTCACGCCTGTAATCCCAGCACTTTGGGAGGCTGCAGCGGGTGGA
TCACTTGAGGTAAGGAGTTCAAGACCAGACCATAGTGAAACCGTGTCTCTACAAAAAAAATTAGCCAGGTGT
GGTGGTGCATGCTTGTAATCCCAGCTACTCAGAAGGCTGAGGTGGGAGAATCCCTTGAACCCGGGAAGTGGA
GGCTGGAGTAAACCATGATCGAGTTACTGCACTCCAGCCTGGGTGACAAGAGTGAGACTCTGTCTCCAAAAA
AAAAAAAAAAAAAAAAAAAAACTGGATTGCCTGGCTCTACTCCGGGCACAGCATGCAGGCCCAGTTCTGCTGCT
CTGCTGTTTGTTCTGCTTTCCTCCACATATTGGCATCACCCTCTGGTGCCAAGATGGCTGCTGCATTCCAGGCAT
CACATCCAGACTCAGACCCAGAGAAGCTGCCCATCCCTACCTGGGTGAGCCTTTGTAGGAACGAGAAACCGC
ATCCAGCAGCAGAAACCTCACCCAGCAGCGTCTTTTCCGGTCTCATTCACCAGCGCCGCCCACCGCTCAACCAA
TCCCTGGCCAAAAGAATGGACCGCCTGGAAGGCTGGACCAAACAGGACCTGCCCTCTGGGGCTGGGGAGA
GGCCCAGATGAAGGCTGCAGGACAGGATGGACTCCTAGACCTCTGTTACCAGCAGTGACTACCTCTGTCTGG
GTGGTTGGAACATGTTTGAATTTTATTCTAAGTACTGTCTACAAGTTCTGCAATAAACCTTGACTCTTCTTTTAA
TAATGCAAA
SEQ ID NO. 17 Homo sapiens poliovirus receptor (CD155/PVR), transcript variant 1 (mRNA) NM_006505.4 
ATGGCCCGAGCCATGGCCGCCGCGTGGCCGCTGCTGCTGGTGGCGCTACTGGTGCTGTCCTGGCCACCCCCA
GGAACCGGGGACGTCGTCGTGCAGGCGCCCACCCAGGTGCCCGGCTTCTTGGGCGACTCCGTGACGCTGCCC
TGCTACCTACAGGTGCCCAACATGGAGGTGACGCATGTGTCACAGCTGACTTGGGCGCGGCATGGTGAATCT
GGCAGCATGGCCGTCTTCCACCAAACGCAGGGCCCCAGCTATTCGGAGTCCAAACGGCTGGAATTCGTGGCA
GCCAGACTGGGCGCGGAGCTGCGGAATGCCTCGCTGAGGATGTTCGGGTTGCGCGTAGAGGATGAAGGCAA
CTACACCTGCCTGTTCGTCACGTTCCCGCAGGGCAGCAGGAGCGTGGATATCTGGCTCCGAGTGCTTGCCAAG
CCCCAGAACACAGCTGAGGTTCAGAAGGTCCAGCTCACTGGAGAGCCAGTGCCCATGGCCCGCTGCGTCTCC
ACAGGGGGTCGCCCGCCAGCCCAAATCACCTGGCACTCAGACCTGGGCGGGATGCCCAATACGAGCCAGGTG
CCAGGGTTCCTGTCTGGCACAGTCACTGTCACCAGCCTCTGGATATTGGTGCCCTCAAGCCAGGTGGACGGCA
 
AGAATGTGACCTGCAAGGTGGAGCACGAGAGCTTTGAGAAGCCTCAGCTGCTGACTGTGAACCTCACCGTGT
ACTACCCCCCAGAGGTATCCATCTCTGGCTATGATAACAACTGGTACCTTGGCCAGAATGAGGCCACCCTGAC
CTGCGATGCTCGCAGCAACCCAGAGCCCACAGGCTATAATTGGAGCACGACCATGGGTCCCCTGCCACCCTTT
GCTGTGGCCCAGGGCGCCCAGCTCCTGATCCGTCCTGTGGACAAACCAATCAACACAACTTTAATCTGCAACG
TCACCAATGCCCTAGGAGCTCGCCAGGCAGAACTGACCGTCCAGGTCAAAGAGGGACCTCCCAGTGAGCACT
CAGGCATGTCCCGTAACGCCATCATCTTCCTGGTTCTGGGAATCCTGGTTTTTCTGATCCTGCTGGGGATCGGG
ATTTATTTCTATTGGTCCAAATGTTCCCGTGAGGTCCTTTGGCACTGTCATCTGTGTCCCTCGAGTACAGAGCAT
GCCAGCGCCTCAGCTAATGGGCATGTCTCCTATTCAGCTGTGAGCAGAGAGAACAGCTCTTCCCAGGATCCAC
AGACAGAGGGCACAAGGTGA
SEQ ID NO. 18 Homo sapiens poliovirus receptor (CD155/PVR), transcript variant 1 (protein) NP_006496.4 
MARAMAAAWPLLLVALLVLSWPPPGTGDVVVQAPTQVPGFLGDSVTLPCYLQVPNMEVTHVSQLTWARHGES
GSMAVFHQTQGPSYSESKRLEFVAARLGAELRNASLRMFGLRVEDEGNYTCLFVTFPQGSRSVDIWLRVLAKPQN
TAEVQKVQLTGEPVPMARCVSTGGRPPAQITWHSDLGGMPNTSQVPGFLSGTVTVTSLWILVPSSQVDGKNVTC
KVEHESFEKPQLLTVNLTVYYPPEVSISGYDNNWYLGQNEATLTCDARSNPEPTGYNWSTTMGPLPPFAVAQGAQ
LLIRPVDKPINTTLICNVTNALGARQAELTVQVKEGPPSEHSGMSRNAIIFLVLGILVFLILLGIGIYFYWSKCSREVLW
HCHLCPSSTEHASASANGHVSYSAVSRENSSSQDPQTEGTR
SEQ ID NO. 19 Homo sapiens poliovirus receptor (CD155/PVR), transcript variant 2 (nucleotide) NM_001135768 
AGCGGGACTGAGCGCCGGGAGAGACCTGCGCAGGCGCAGGCGCGCGGGGAGGGCCAGCCTGGGTGGCCCA
CCCCGCGCCTGGCGGGACTGGCCGCCAACTCCCCTCCGCTCCAGTCACTTGTCTGGAGCTTGAAGAAGTGGGT
ATTCCCCTTCCCACCCCAGGCACTGGAGGAGCGGCCCCCGGGGATTCCAGGACCTGAGCTCCGGGAGCTGG
ACTCGCAGCGACCGCGGCAGAGCGAGCGGGCGCCGGGAAGCGAGGAGACGCCCGCGGAGGCCCAGCTGC
TCGGAGCAACTGGCATGGCCCGAGCCATGGCCGCCGTGGCCGCTGCTGCTGGTGGCGCTACTGGTGCTGT
CCTGGCCACCCCCAGGAACCGGGGACGTCGTCGTGCAGGCGCCCACCCAGGTGCCCGGCTTCTTGGGCGACT
CCGTGACGCTGCCCTGCTACCTACAGGTGCCCAACATGGAGGTGACGCATGTGTCACAGCTGACTTGGGCGC
GGCATGGTGAATCTGGCAGCATGGCCGTCTTCCACCAAACGCAGGGCCCCAGCTATTCGGAGTCCAAACGGC
TGGAATTCGTGGCAGCCAGACTGGGCGCGGAGCTGCGGAATGCCTCGCTGAGGATGTTCGGGTTGCGCGTA
GAGGATGAAGGCAACTACACCTGCCTGTTCGTCACGTTCCCGCAGGGCAGCAGGAGCGTGGATATCTGGCTC
CGAGTGCTTGCCAAGCCCCAGAACACAGCTGAGGTTCAGAAGGTCCAGCTCACTGGAGAGCCAGTGCCCATG
GCCCGCTGCGTCTCCACAGGGGGTCGCCCGCCAGCCCAAATCACCTGGCACTCAGACCTGGGCGGGATGCCC
AATACGAGCCAGGTGCCAGGGTTCCTGTCTGGCACAGTCACTGTCACCAGCCTCTGGATATTGGTGCCCTCAA
GCCAGGTGGACGGCAAGAATGTGACCTGCAAGGTGGAGCACGAGAGCTTTGAGAAGCCTCAGCTGCTGACT
GTGAACCTCACCGTGTACTACCCCCCAGAGGTATCCATCTCTGGCTATGATAACAACTGGTACCTTGGCCAGAA
TGAGGCCACCCTGACCTGCGATGCTCGCAGCAACCCAGAGCCCACAGGCTATAATTGGAGCACGACCATGGG
TCCCCTGCCACCCTTTGCTGTGGCCCAGGGCGCCCAGCTCCTGATCCGTCCTGTGGACAAACCAATCAACACAA
CTTTAATCTGCAACGTCACCAATGCCCTAGGAGCTCGCCAGGCAGAACTGACCGTCCAGGTCAAAGAGGGAC
CTCCCAGTGAGCACTCAGGTACAGAGCATGCCAGCGCCTCAGCTAATGGGCATGTCTCCTATTCAGCTGTGAG
CAGAGAGAACAGCTCTTCCCAGGATCCACAGACAGAGGGCACAAGGTGACAGCGTCGGGACTGAGAGGGGA
GAGAGACTGGAGCTGGCAAGGACGTGGGCCTCCAGAGTTGGACCCGACCCCAATGGATGAAGACCCCCTCC
AAAGAGACCAGCCTCCCTCCCTGTGCCAGACCTCAAAACGACGGGGGCAGGTGCAAGTTCATAGGTCTCCAA
GACCACCCTCCTTTCATTTGCTAGAAGGACTCACTAGACTCAGGAAAGCTGTTAGGCTCACAGTTACAGTTTAT
TACAGTAAAAGGACAGAGATTAAGATCAGCAAAGGGAGGAGGTGCACAGCACACGTTCCACGACAGATGAG
GCGACGGCTTCCATCTGCCCTCTCCCAGTGGAGCCATATAGGCAGCACCTGATTCTCACAGCAACATGTGACA
ACATGCAAGAAGTACTGCCAATACTGCCAACCAGAGCAGCTCACTCGAGATCTTTGTGTCCAGAGTTTTTTGTT
TGTCTTGAGACAGGGTCTGGCTCTGTTGGCAGACTAGAGTACAGTGGTGAGATCACAGTTCATTGCAGCCTTG
ACTTCTCAACGCCAAGTCATCCTCCCACCTCAGCCTCCTGAGTAGCTATGACTACAGGTATGTGCCACCACGTC
TGGCTAATCTTTTTATTATTTGTAAAGTCGAGGTTTCCCTGTGTTGCCCAGGCTGGTCTTGAACTCTTGGCTCCA
AGTGATACTTCTGCCTTGGCCTCCCAAAGTGCTGAATTAAGCAGCTCACCATCCACACGGCTGACCTCATACAT
 
CAAGCCAATACCGTGTGGCCCAAGACCCCCACCATAAATCACATCATTAGCATGAACCACCCAGAGTGGCCCA
AGACTCCAAGATCAGCTACCAGGCAGGATATTCCAAGGGCTTAGAGATGAATGCCCAGGAGCTGAGGATAAA
GGGCCCGATCTTTCTTTGGGCAAGGTTAAGCCTTTACTGCATAGCAGACCACACAGAAGGGTGTGGGCCACCA
GAGAATTTTGGTAAAAATTTGGCCTCTGGCCTTGAGCTTCTAAATCTCTGTATCCGTCAGATCTCTGTGGTTAC
AAGAAACAGCCACTGACCCTGGTCACCAGAGGCTGCAATTCAGGCCGCAAGCAGCTGCCTGGGGGGTGTCCA
AGGAGCAGAGAAAACTACTAGATGTGAACTTGAAGAAGGTTGTCAGCTGCAGCCACTTTCTGCCAGCATCTG
CAGCCACTTTCTGCCAGCATCTGCAGCCAGCAAGCTGGGACTGGCAGGAAATAACCCACAAAAGAAGCAAAT
GCAATTTCCAACACAAGGGGGAAGGGATGCAGGGGGAGGCAGCGCTGCAGTTGCTCAGGACACGCTCCTAT
AGGACCAAGATGGATGCGACCCAAGACCCAGGAGGCCCAGCTGCTCAGTGCAACTGACAAGTTAAAAAGGTC
TATGATCTTGAGGGCAGACAGCAGAATTCCTCTTATAAAGAAAACTGTTTGGGAAAATACGTTGAGGGAGAG
AAGACCTTGGGCCAAGATGCTAAATGGGAATGCAAAGCTTGAGCTGCTCTGCAAGAGAAAATAAGCAGGACA
GAGGATTTGCTCTGGACAGAGATGGAAGAGCCGGGAACAGAGAAGTGTGGGGAAGAGATAGGAACCAGCA
GGATGGCAGGGGCAAAGGGCTCAAGGGTGAGGAGGCCAGTGGGACCCCACAGAGTTGGGGAGATAAAGG
AACATTGGTTGCTTTGGTGGCACGTAAGCTCCTTGTCTGTCTCCAGCACCCAGAATCTCATTAAAGCTTATTTAT
TGTACCTCCAGCGGCTGTGTGCAATGGGGTCTTTTGTGGAAATCAAGGAGCAGACAGGTTTCATGTGTACTGT
CACCACGTGGGATGGAACCAGAGGCATGGAAGCAAGACGCTAAATGAAGAGGGCCATAAGGGCTGGGATTC
CCAGGCACCTTAGGAACAGCTTGTCTTTTTTTTTTTCCTCTCCAAAAAAAATGTTTAAGGGACGGTGTCTCCTGT
CACCCAGGCTGGAGTGCAATGGCACGATCATAGCTCATTGCAGCCTCTAACTCCGGGGCTCAAGCAATCCTCC
CACCTCAGCCTACCAAGTAGCTGTGACCACAGCTGCCCCTCACCATGCTAAGCTAATTTTTTTAATTAGATAGT
ACATAAACGTCCCAAAATTAGAAGATAAAAAGACATGAGGGATCCATTCTAATTTGTGTTTGGAGTGTAATGG
TCCAGCTCCATTCTTCTGCACATGGATATCCAGTTTTACACAACACTGTGAATGTAATGAATGCCACTGAATCAT
ACACTCAAAAATAGCTAAAATGGCAAATTGTCTGTTATCTCTTTTTAACCACCATTTTTGAAAATTAATTATACC
AAAAAACCATTGAATAGTGCACTTTATTTATTTATTTATTTGTTTATTTATTTATTTATTTTAGAAATAAGAGTCT
CACTTTGTTGCCCAGGCTGGAGTGCAGTGGCGTGATCATGGCTCATTGCAGCCTCGACCTGCTGGGCTCGGGC
TATCCTTCCATCTCAGCCTCCCGAGTAGCTGGGACTATAGGTGGGCGCCACCCCACCTGGCTAAATCTCTTTTT
AACTTTTGTAGAGATAGGCATCTCGCTATGTTGCCTAGGCTGGGCTGGAACTCCTGGGCTCAAGTGCTCCTCCT
GCCTTGGCCTCCCAAAGCGCTAGGATTACAGATGTGAGCCACCGCGCCCACCCTGAACCTTACTTTTTTTGCTC
AGTTTCTGGTAATTCAGAGAATGCCTCCTGAGTTGTTCTACACCCACCTCATATTCCATGGGAGGGCTGTACAG
GGCTTTTTTAACGAGGCCTCTAAGGACAGGCATTTGTATCCTTTCCAGCCTTTCACTATTACAATGTTGTAGTGA
ATAACTTTACACACTGTCATTTATTTTACTTTTTTTTTTTTTTATTTTAGAGAAAGGAATCTTGCCATCTTGCCCAG
GCTGGTCTCAAATTCCTGGGCCCAAACAATCCTCCCGCCTTGGCCTCCTAAAGTACTGGGATTTATAGGCATAA
GCCACCGTGCCTGGCCAATGCACACTGTCATTTAGCTCATGTTAACACCTGAGTGTAGGACACACTCCTGGAG
GTGGAATTGCTGGGCCAAAGAGTATGTTTCTTGTCATTGTGATAGATATTGACAAATGAACCCTCACAGAAGT
TGTGCTGAGTTCTGTTCCCACCAGCGACGTAGGCGATGACCTTTTTCTGGAGGGAGGGGGCATCCTTGGAGTC
CACAGAGCCAGGAATGGAGAGTGGGCCCAGAATTTTGGTATAGGTGTTGTATAAACTTATAGTAAGGTTAAG
AAAACCGCAACTATCCTTATCAGAGACTTGGCGGGGGGCAGGGTATGATGGAGATCATAAGGAGGCTAAAA
CACTCCACACCCTCCCTCTGCATTGCTCCTGCACGGGAGTCGGGAATCTTTTCAGGTTGATACGATCTCACCTT
GAGGAGCTGTGAGGTCCCAGAAGCCTCTGGGTTGCAGATTGCTTGGGGTGAAAATGTCTGTGCTACTGAAAT
CTAACTTTTTACAAAAAATTACGGGCTGGGCGCAGTGGCTCACGCCTGTAATCCCAGCACTTTGGGAGGCTGC
AGCGGGTGGATCACTTGAGGTAAGGAGTTCAAGACCAGACCATAGTGAAACCGTGTCTCTACAAAAAAAATT
AGCCAGGTGTGGTGGTGCATGCTTGTAATCCCAGCTACTCAGAAGGCTGAGGTGGGAGAATCCCTTGAACCC
GGGAAGTGGAGGCTGGAGTAAACCATGATCGAGTTACTGCACTCCAGCCTGGGTGACAAGAGTGAGACTCT
GTCTCCAAAAAAAAAAAAAAAAAAAAAAAAACTGGATTGCCTGGCTCTACTCCGGGCACAGCATGCAGGCCC
AGTTCTGCTGCTCTGCTGTTTGTTCTGCTTTCCTCCACATATTGGCATCACCCTCTGGTGCCAAGATGGCTGCTG
CATTCCAGGCATCACATCCAGACTCAGACCCAGAGAAGCTGCCCATCCCTACCTGGGTGAGCCTTTGTAGGAA
CGAGAAACCGCATCCAGCAGCAGAAACCTCACCCAGCAGCGTCTTTTCCGGTCTCATTCACCAGCGCCGCCCA
CCGCTCAACCAATCCCTGGCCAAAAGAATGGGACCGCCTGGAAGGCTGGACCAAACAGGACCTGCCCTCTGG
GGCTGGGGAGAGGCCCAGATGAAGGCTGCAGGACAGGATGGACTCCTAGACCTCTGTTACCAGCAGTGACT
ACCTCTGTCTGGGTGGTTGGAACATGTTTGAATTTTATTCTAAGTACTGTCTACAAGTTCTGCAATAAACCTTG
ACTCTTCTTTTAATAATGCAAA
SEQ ID NO. 20 Homo sapiens poliovirus receptor (CD155/PVR), transcript variant 2 (mRNA) NM_01135768.2 
ATGGCCCGAGCCATGGCCGCCGCGTGGCCGCTGCTGCTGGTGGCGCTACTGGTGCTGTCCTGGCCACCCCCA
GGAACCGGGGACGTCGTCGTGCAGGCGCCCACCCAGGTGCCCGGCTTCTTGGGCGACTCCGTGACGCTGCCC
TGCTACCTACAGGTGCCCAACATGGAGGTGACGCATGTGTCACAGCTGACTTGGGCGCGGCATGGTGAATCT
GGCAGCATGGCCGTCTTCCACCAAACGCAGGGCCCCAGCTATTCGGAGTCCAAACGGCTGGAATTCGTGGCA
GCCAGACTGGGCGCGGAGCTGCGGAATGCCTCGCTGAGGATGTTCGGGTTGCGCGTAGAGGATGAAGGCAA
CTACACCTGCCTGTTCGTCACGTTCCCGCAGGGCAGCAGGAGCGTGGATATCTGGCTCCGAGTGCTTGCCAAG
CCCCAGAACACAGCTGAGGTTCAGAAGGTCCAGCTCACTGGAGAGCCAGTGCCCATGGCCCGCTGCGTCTCC
ACAGGGGGTCGCCCGCCAGCCCAAATCACCTGGCACTCAGACCTGGGCGGGATGCCCAATACGAGCCAGGTG
CCAGGGTTCCTGTCTGGCACAGTCACTGTCACCAGCCTCTGGATATTGGTGCCCTCAAGCCAGGTGGACGGCA
AGAATGTGACCTGCAAGGTGGAGCACGAGAGCTTTGAGAAGCCTCAGCTGCTGACTGTGAACCTCACCGTGT
ACTACCCCCCAGAGGTATCCATCTCTGGCTATGATAACAACTGGTACCTTGGCCAGAATGAGGCCACCCTGAC
CTGCGATGCTCGCAGCAACCCAGAGCCCACAGGCTATAATTGGAGCACGACCATGGGTCCCCTGCCACCCTTT
GCTGTGGCCCAGGGCGCCCAGCTCCTGATCCGTCCTGTGGACAAACCAATCAACACAACTTTAATCTGCAACG
TCACCAATGCCCTAGGAGCTCGCCAGGCAGAACTGACCGTCCAGGTCAAAGAGGGACCTCCCAGTGAGCACT
CAGGTACAGAGCATGCCAGCGCCTCAGCTAATGGGCATGTCTCCTATTCAGCTGTGAGCAGAGAGAACAGCT
CTTCCCAGGATCCACAGACAGAGGGCACAAGGTGA
SEQ ID NO. 21 Homo sapiens poliovirus receptor (CD155/PVR), transcript variant 2 (protein) NP_001129240.1 
MARAMAAAWPLLLVALLVLSWPPPGTGDVVVQAPTQVPGFLGDSVTLPCYLQVPNMEVTHVSQLTWARHGES
GSMAVFHQTQGPSYSESKRLEFVAARLGAELRNASLRMFGLRVEDEGNYTCLFVTFPQGSRSVDIWLRVLAKPQN
TAEVQKVQLTGEPVPMARCVSTGGRPPAQITWHSDLGGMPNTSQVPGFLSGTVTVTSLWILVPSSQVDGKNVTC
KVEHESFEKPQLLTVNLTVYYPPEVSISGYDNNWYLGQNEATLTCDARSNPEPTGYNWSTTMGPLPPFAVAQGAQ
LLIRPVDKPINTTLICNVTNALGARQAELTVQVKEGPPSEHSGTEHASASANGHVSYSAVSRENSSSQDPQTEGTR
SEQ ID NO. 22 Homo sapiens poliovirus receptor (CD155/PVR), transcript variant 3 (nucleotide) NM_001135769 
AGCGGGACTGAGCGCCGGGAGAGACCTGCGCAGGCGCAGGCGCGGGGAGGGCCAGCCTGGGTGGCCCA
CCCCGCGCCTGGCGGGACTGGCCGCCAACTCCCCTCCGCTCCAGTCACTTGTCTGGAGCTTGAAGAAGTGGGT
ATTCCCCTTCCCACCCCAGGCACTGGAGGAGCGGCCCCCGGGGATTCCAGGACCTGAGCTCCGGGAGCTGG
ACTCGCAGCGACCGCGGCAGAGCGAGCGGGCGCCGGGAAGCGAGGAGACGCCCGCGGGAGGCCCAGCTGC
TCGGAGCAACTGGCATGGCCCGAGCCATGGCCGCCGTGGCCGCTGCTGCTGGTGGCGCTACTGGTGCTGT
CCTGGCCACCCCCAGGAACCGGGGACGTCGTCGTGCAGGCGCCCACCCAGGTGCCCGGCTTCTTGGGCGACT
CCGTGACGCTGCCCTGCTACCTACAGGTGCCCAACATGGAGGTGACGCATGTGTCACAGCTGACTTGGGCGC
GGCATGGTGAATCTGGCAGCATGGCCGTCTTCCACCAAACGCAGGGCCCCAGCTATTCGGAGTCCAAACGGC
TGGAATTCGTGGCAGCCAGACTGGGCGCGGAGCTGCGGAATGCCTCGCTGAGGATGTTCGGGTTGCGCGTA
GAGGATGAAGGCAACTACACCTGCCTGTTCGTCACGTTCCCGCAGGGCAGCAGGAGCGTGGATATCTGGCTC
CGAGTGCTTGCCAAGCCCCAGAACACAGCTGAGGTTCAGAAGGTCCAGCTCACTGGAGAGCCAGTGCCCATG
GCCCGCTGCGTCTCCACAGGGGGTCGCCCGCCAGCCCAAATCACCTGGCACTCAGACCTGGGCGGGATGCCC
AATACGAGCCAGGTGCCAGGGTTCCTGTCTGGCACAGTCACTGTCACCAGCCTCTGGATATTGGTGCCCTCAA
GCCAGGTGGACGGCAAGAATGTGACCTGCAAGGTGGAGCACGAGAGCTTTGAGAAGCCTCAGCTGCTGACT
GTGAACCTCACCGTGTACTACCCCCCAGAGGTATCCATCTCTGGCTATGATAACAACTGGTACCTTGGCCAGAA
TGAGGCCACCCTGACCTGCGATGCTCGCAGCAACCCAGAGCCCACAGGCTATAATTGGAGCACGACCATGGG
TCCCCTGCCACCCTTTGCTGTGGCCCAGGGCGCCCAGCTCCTGATCCGTCCTGTGGACAAACCAATCAACACAA
CTTTAATCTGCAACGTCACCAATGCCCTAGGAGCTCGCCAGGCAGAACTGACCGTCCAGGTCAAAGGTACAGA
GCATGCCAGCGCCTCAGCTAATGGGCATGTCTCCTATTCAGCTGTGAGCAGAGAGAACAGCTCTTCCCAGGAT
CCACAGACAGAGGGCACAAGGTGACAGCGTCGGGACTGAGAGGGGAGAGAGACTGGAGCTGGCAAGGACG
TGGGCCTCCAGAGTTGGACCCGACCCCAATGGATGAAGACCCCCTCCAAAGAGACCAGCCTCCCTCCCTGTGC
CAGACCTCAAAACGACGGGGGCAGGTGCAAGTTCATAGGTCTCCAAGACCACCCTCCTTTCATTTGCTAGAAG
 
GACTCACTAGACTCAGGAAAGCTGTTAGGCTCACAGTTACAGTTTATTACAGTAAAGGACAGAGATTAAGAT
CAGCAAAGGGAGGAGGTGCACAGCACACGTTCCACGACAGATGAGGCGACGGCTTCCATCTGCCCTCTCCCA
GTGGAGCCATATAGGCAGCACCTGATTCTCACAGCAACATGTGACAACATGCAAGAAGTACTGCCAATACTGC
CAACCAGAGCAGCTCACTCGAGATCTTTGTGTCCAGAGTTTTTTGTTTGTCTTGAGACAGGGTCTGGCTCTGTT
GGCAGACTAGAGTACAGTGGTGAGATCACAGTTCATTGCAGCCTTGACTTCTCAACGCCAAGTCATCCTCCCA
CCTCAGCCTCCTGAGTAGCTATGACTACAGGTATGTGCCACCACGTCTGGCTAATCTTTTTATTATTTGTAAAGT
CGAGGTTTCCCTGTGTTGCCCAGGCTGGTCTTGAACTCTTGGCTCCAAGTGATACTTCTGCCTTGGCCTCCCAA
AGTGCTGAATTAAGCAGCTCACCATCCACACGGCTGACCTCATACATCAAGCCAATACCGTGTGGCCCAAGAC
CCCCACCATAAATCACATCATTAGCATGAACCACCCAGAGTGGCCCAAGACTCCAAGATCAGCTACCAGGCAG
GATATTCCAAGGGCTTAGAGATGAATGCCCAGGAGCTGAGGATAAAGGGCCCGATCTTTCTTTGGGCAAGGT
TAAGCCTTTACTGCATAGCAGACCACACAGAAGGGTGTGGGCCACCAGAGAATTTTGGTAAAAATTTGGCCTC
TGGCCTTGAGCTTCTAAATCTCTGTATCCGTCAGATCTCTGTGGTTACAAGAAACAGCCACTGACCCTGGTCAC
CAGAGGCTGCAATTCAGGCCGCAAGCAGCTGCCTGGGGGGTGTCCAAGGAGCAGAGAAAACTACTAGATGT
GAACTTGAAGAAGGTTGTCAGCTGCAGCCACTTTCTGCCAGCATCTGCAGCCACTTTCTGCCAGCATCTGCAGC
CAGCAAGCTGGGACTGGCAGGAAATAACCCACAAAAGAAGCAAATGCAATTTCCAACACAAGGGGGAAGGG
ATGCAGGGGGAGGCAGCGCTGCAGTTGCTCAGGACACGCTCCTATAGGACCAAGATGGATGCGACCCAAGA
CCCAGGAGGCCCAGCTGCTCAGTGCAACTGACAAGTTAAAAAGGTCTATGATCTTGAGGGCAGACAGCAGAA
TTCCTCTTATAAAGAAAACTGTTTGGGAAAATACGTTGAGGGAGAGAAGACCTTGGGCCAAGATGCTAAATG
GGAATGCAAAGCTTGAGCTGCTCTGCAAGAGAAAATAAGCAGGACAGAGGATTTGCTCTGGACAGAGATGG
AAGAGCCGGGAACAGAGAAGTGTGGGGAAGAGATAGGAACCAGCAGGATGGCAGGGGCAAAGGGCTCAA
GGGTGAGGAGGCCAGTGGGACCCCACAGAGTTGGGGAGATAAAGGAACATTGGTTGCTTTGGTGGCACGTA
AGCTCCTTGTCTGTCTCCAGCACCCAGAATCTCATTAAAGCTTATTTATTGTACCTCCAGCGGCTGTGTGCAATG
GGGTCTTTTGTGGAAATCAAGGAGCAGACAGGTTTCATGTGTACTGTCACCACGTGGGATGGAACCAGAGGC
ATGGAAGCAAGACGCTAAATGAAGAGGGCCATAAGGGCTGGGATTCCCAGGCACCTTAGGAACAGCTTGTCT
TTTTTTTTTTCCTCTCCAAAAAAAATGTTTAAGGACGGTGTCTCCTGTCACCCAGGCTGGAGTGCAATGGCAC
GATCATAGCTCATTGCAGCCTCTAACTCCGGGGCTCAAGCAATCCTCCCACCTCAGCCTACCAAGTAGCTGTGA
CCACAGCTGCCCCTCACCATGCTAAGCTAATTTTTTTAATTAGATAGTACATAAACGTCCCAAAATTAGAAGAT
AAAAAGACATGAGGGATCCATTCTAATTTGTGTTTGGAGTGTAATGGTCCAGCTCCATTCTTCTGCACATGGAT
ATCCAGTTTTACACAACACTGTGAATGTAATGAATGCCACTGAATCATACACTCAAAAATAGCTAAAATGGCAA
ATTGTCTGTTATCTCTTTTTAACCACCATTTTTGAAAATTAATTATACCAAAAAACCATTGAATAGTGCACTTTAT
TTATTTATTTATTTGTTTATTTATTTATTTATTTTAGAAATAAGAGTCTCACTTTGTTGCCCAGGCTGGAGTGCAG
TGGCGTGATCATGGCTCATTGCAGCCTCGACCTGCTGGGCTCGGGCTATCCTTCCATCTCAGCCTCCCGAGTAG
CTGGGACTATAGGTGGGCGCCACCCCACCTGGCTAAATCTCTTTTTAACTTTTGTAGAGATAGGCATCTCGCTA
TGTTGCCTAGGCTGGGCTGGAACTCCTGGGCTCAAGTGCTCCTCCTGCCTTGGCCTCCCAAAGCGCTAGGATT
ACAGATGTGAGCCACCGCGCCCACCCTGAACCTTACTTTTTTTGCTCAGTTTCTGGTAATTCAGAGAATGCCTC
CTGAGTTGTTCTACACCCACCTCATATTCCATGGGAGGGCTGTACAGGGCTTTTTTAACGAGGCCTCTAAGGAC
AGGCATTTGTATCCTTTCCAGCCTTTCACTATTACAATGTTGTAGTGAATAACTTTACACACTGTCATTTATTTTA
CTTTTTTTTTTTTTTTATTTAGAGAAAGGAATCTTGCCATCTTGCCCAGGCTGGTCTCAAATTCCTGGGCCCAAA
CAATCCTCCCGCCTTGGCCTCCTAAAGTACTGGGATTTATAGGCATAAGCCACCGTGCCTGGCCAATGCACACT
GTCATTTAGCTCATGTTAACACCTGAGTGTAGGACACACTCCTGGAGGTGGAATTGCTGGGCCAAAGAGTATG
TTTCTTGTCATTGTGATAGATATTGACAAATGAACCCTCACAGAAGTTGTGCTGAGTTCTGTTCCCACCAGCGA
CGTAGGCGATGACCTTTTTCTGGAGGGAGGGGGCATCCTTGGAGTCCACAGAGCCAGGAATGGAGAGTGGG
CCCAGAATTTTGGTATAGGTGTTGTATAAACTTATAGTAAGGTTAAGAAAACCGCAACTATCCTTATCAGAGAC
TTGGCGGGGGGCAGGGTATGATGGAGATCATAAGGAGGCTAAAACACTCCACACCCTCCCTCTGCATTGCTC
CTGCACGGGAGTCGGGAATCTTTTCAGGTTGATACGATCTCACCTTGAGGAGCTGTGAGGTCCCAGAAGCCTC
TGGGTTGCAGATTGCTTGGGGTGAAAATGTCTGTGCTACTGAAATCTAACTTTTTACAAAAAATTACGGGCTG
GGCGCAGTGGCTCACGCCTGTAATCCCAGCACTTTGGGAGGCTGCAGCGGGTGGATCACTTGAGGTAAGGAG
TTCAAGACCAGACCATAGTGAAACCGTGTCTCTACAAAAAAAATTAGCCAGGTGTGGTGGTGCATGCTTGTAA
TCCCAGCTACTCAGAAGGCTGAGGTGGGAGAATCCCTTGAACCCGGGAAGTGGAGGCTGGAGTAAACCATG
ATCGAGTTACTGCACTCCAGCCTGGGTGACAAGAGTGAGACTCTGTCTCCAAAAAAAAAAAAAAAAAAAAAA
AAACTGGATTGCCTGGCTCTACTCCGGGCACAGCATGCAGGCCCAGTTCTGCTGCTCTGCTGTTTGTTCTGCTT
 
TCCTCCACATATTGGCATCACCCTCTGGTGCCAAGATGGCTGCTGCATTCCAGGCATCACATCCAGACTCAGAC
CCAGAGAAGCTGCCCATCCCTACCTGGGTGAGCCTTTGTAGGAACGAGAAACCGCATCCAGCAGCAGAAACC
TCACCCAGCAGCGTCTTTTCCGGTCTCATTCACCAGCGCCGCCCACCGCTCAACCAATCCCTGGCCAAAAGAAT
GGGACCGCCTGGAAGGCTGGACCAAACAGGACCTGCCCTCTGGGGCTGGGGAGAGGCCCAGATGAAGGCTG
CAGGACAGGATGGACTCCTAGACCTCTGTTACCAGCAGTGACTACCTCTGTCTGGGTGGTTGGAACATGTTTG
AATTTTATTCTAAGTACTGTCTACAAGTTCTGCAATAAACCTTGACTCTTCTTTAATAATGCAAA
SEQ ID NO. 24 Homo sapiens poliovirus receptor (CD155/PVR), transcript variant 3 (mRNA) NM_001135769.2 
ATGGCCCGAGCCATGGCCGCCGCGTGGCCGCTGCTGCTGGTGGCGCTACTGGTGCTGTCCTGGCCACCCCCA
GGAACCGGGGACGTCGTCGTGCAGGCGCCCACCCAGGTGCCCGGCTTCTTGGGCGACTCCGTGACGCTGCCC
TGCTACCTACAGGTGCCCAACATGGAGGTGACGCATGTGTCACAGCTGACTTGGGCGCGGCATGGTGAATCT
GGCAGCATGGCCGTCTTCCACCAAACGCAGGGCCCCAGCTATTCGGAGTCCAAACGGCTGGAATTCGTGGCA
GCCAGACTGGGCGCGGAGCTGCGGAATGCCTCGCTGAGGATGTTCGGGTTGCGCGTAGAGGATGAAGGCAA
CTACACCTGCCTGTTCGTCACGTTCCCGCAGGGCAGCAGGAGCGTGGATATCTGGCTCCGAGTGCTTGCCAAG
CCCCAGAACACAGCTGAGGTTCAGAAGGTCCAGCTCACTGGAGAGCCAGTGCCCATGGCCCGCTGCGTCTCC
ACAGGGGGTCGCCCGCCAGCCCAAATCACCTGGCACTCAGACCTGGGCGGGATGCCCAATACGAGCCAGGTG
CCAGGGTTCCTGTCTGGCACAGTCACTGTCACCAGCCTCTGGATATTGGTGCCCTCAAGCCAGGTGGACGGCA
AGAATGTGACCTGCAAGGTGGAGCACGAGAGCTTTGAGAAGCCTCAGCTGCTGACTGTGAACCTCACCGTGT
ACTACCCCCCAGAGGTATCCATCTCTGGCTATGATAACAACTGGTACCTTGGCCAGAATGAGGCCACCCTGAC
CTGCGATGCTCGCAGCAACCCAGAGCCCACAGGCTATAATTGGAGCACGACCATGGGTCCCCTGCCACCCTTT
GCTGTGGCCCAGGGCGCCCAGCTCCTGATCCGTCCTGTGGACAAACCAATCAACACAACTTTAATCTGCAACG
TCACCAATGCCCTAGGAGCTCGCCAGGCAGAACTGACCGTCCAGGTCAAAGGTACAGAGCATGCCAGCGCCT
CAGCTAATGGGCATGTCTCCTATTCAGCTGTGAGCAGAGAGAACAGCTCTTCCCAGGATCCACAGACAGAGG
GCACAAGGTGA
SEQ ID NO. 24 Homo sapiens poliovirus receptor (CD155/PVR), transcript variant 3 (protein) NP_001129241.1 
MARAMAAAWPLLLVALLVLSWPPPGTGDVVVQAPTQVPGFLGDSVTLPCYLQVPNMEVTHVSQLTWARHGES
GSMAVFHQTQGPSYSESKRLEFVAARLGAELRNASLRMFGLRVEDEGNYTCLFVTFPQGSRSVDIWLRVLAKPQN
TAEVQKVQLTGEPVPMARCVSTGGRPPAQITWHSDLGGMPNTSQVPGFLSGTVTVTSLWILVPSSQVDGKNVTC
KVEHESFEKPQLLTVNLTVYYPPEVSISGYDNNWYLGQNEATLTCDARSNPEPTGYNWSTTMGPLPPFAVAQGAQ
LLIRPVDKPINTTLICNVTNALGARQAELTVQVKGTEHASASANGHVSYSAVSRENSSSQDPQTEGTR
SEQ ID NO. 25 Homo sapiens poliovirus receptor (CD155/PVR), transcript variant 4 (nucleotide) NM_001135770 
AGCGGGACTGAGCGCCGGGAGAGACCTGCGCAGGCGCAGGCGCGGGGAGGGCCAGCCTGGGTGGCCCA
CCCCGCGCCTGGCGGACTGGCCGCCAACTCCCCTCCGCTCCAGTCACTTGTCTGGAGCTTGAAGAAGTGGGT
ATTCCCCTTCCCACCCCAGGCACTGGAGGAGCGGCCCCCGGGGATTCCAGGACCTGAGCTCCGGGAGCTGG
ACTCGCAGCGACCGCGGCAGAGCGAGCGGGCGCCGGAAGCGAGGAGACGCCCGCGGAGGCCCAGCTGC
TCGGAGCAACTGGCATGGCCCGAGCCATGGCCGCCGTGGCCGCTGCTGCTGGTGGCGCTACTGGTGCTGT
CCTGGCCACCCCCAGGAACCGGGGACGTCGTCGTGCAGGCGCCCACCCAGGTGCCCGGCTTCTTGGGCGACT
CCGTGACGCTGCCCTGCTACCTACAGGTGCCCAACATGGAGGTGACGCATGTGTCACAGCTGACTTGGGCGC
GGCATGGTGAATCTGGCAGCATGGCCGTCTTCCACCAAACGCAGGGCCCCAGCTATTCGGAGTCCAAACGGC
TGGAATTCGTGGCAGCCAGACTGGGCGCGGAGCTGCGGAATGCCTCGCTGAGGATGTTCGGGTTGCGCGTA
GAGGATGAAGGCAACTACACCTGCCTGTTCGTCACGTTCCCGCAGGGCAGCAGGAGCGTGGATATCTGGCTC
CGAGTGCTTGCCAAGCCCCAGAACACAGCTGAGGTTCAGAAGGTCCAGCTCACTGGAGAGCCAGTGCCCATG
GCCCGCTGCGTCTCCACAGGGGGTCGCCCGCCAGCCCAAATCACCTGGCACTCAGACCTGGGCGGGATGCCC
AATACGAGCCAGGTGCCAGGGTTCCTGTCTGGCACAGTCACTGTCACCAGCCTCTGGATATTGGTGCCCTCAA
GCCAGGTGGACGGCAAGAATGTGACCTGCAAGGTGGAGCACGAGAGCTTTGAGAAGCCTCAGCTGCTGACT
GTGAACCTCACCGTGTACTACCCCCCAGAGGTATCCATCTCTGGCTATGATAACAACTGGTACCTTGGCCAGAA
 
TGAGGCCACCCTGACCTGCGATGCTCGCAGCAACCCAGAGCCCACAGGCTATAATTGGAGCACGACCATGGG
TCCCCTGCCACCCTTTGCTGTGGCCCAGGGCGCCCAGCTCCTGATCCGTCCTGTGGACAAACCAATCAACACAA
CTTTAATCTGCAACGTCACCAATGCCCTAGGAGCTCGCCAGGCAGAACTGACCGTCCAGGTCAAAGAGGGAC
CTCCCAGTGAGCACTCAGGCATGTCCCGTAACGCCATCATCTTCCTGGTTCTGGGAATCCTGGTTTTTCTGATC
CTGCTGGGGATCGGGATTTATTTCTATTGGTCCAAATGTTCCCGTGAGGTCCTTTGGCACTGTCATCTGTGTCC
CTCGAGTGAGCATCACCAGAGCTGCCGTAATTGAGCACCTACTACGGGCTCTGTGCTGAGTCCTTCCAGTGTG
CCTCTCACTGAATCCTCACCCCACTGCCATGAGGTTTCCCCCATTTGACTGATGAGGGTGCAGAGCCAGGGAG
CCTTGTTCACTGGTTCATTGATTACATTTACAAATATTATTTACAGAGTGGGA
SEQ ID NO. 26 Homo sapiens poliovirus receptor (CD155/PVR), transcript variant 4 (mRNA) NM_001135770.3 
ATGGCCCGAGCCATGGCCGCCGCGTGGCCGCTGCTGCTGGTGGCGCTACTGGTGCTGTCCTGGCCACCCCCA
GGAACCGGGGACGTCGTCGTGCAGGCGCCCACCCAGGTGCCCGGCTTCTTGGGCGACTCCGTGACGCTGCCC
TGCTACCTACAGGTGCCCAACATGGAGGTGACGCATGTGTCACAGCTGACTTGGGCGCGGCATGGTGAATCT
GGCAGCATGGCCGTCTTCCACCAAACGCAGGGCCCCAGCTATTCGGAGTCCAAACGGCTGGAATTCGTGGCA
GCCAGACTGGGCGCGGAGCTGCGGAATGCCTCGCTGAGGATGTTCGGGTTGCGCGTAGAGGATGAAGGCAA
CTACACCTGCCTGTTCGTCACGTTCCCGCAGGGCAGCAGGAGCGTGGATATCTGGCTCCGAGTGCTTGCCAAG
CCCCAGAACACAGCTGAGGTTCAGAAGGTCCAGCTCACTGGAGAGCCAGTGCCCATGGCCCGCTGCGTCTCC
ACAGGGGGTCGCCCGCCAGCCCAAATCACCTGGCACTCAGACCTGGGCGGGATGCCCAATACGAGCCAGGTG
CCAGGGTTCCTGTCTGGCACAGTCACTGTCACCAGCCTCTGGATATTGGTGCCCTCAAGCCAGGTGGACGGCA
AGAATGTGACCTGCAAGGTGGAGCACGAGAGCTTTGAGAAGCCTCAGCTGCTGACTGTGAACCTCACCGTGT
ACTACCCCCCAGAGGTATCCATCTCTGGCTATGATAACAACTGGTACCTTGGCCAGAATGAGGCCACCCTGAC
CTGCGATGCTCGCAGCAACCCAGAGCCCACAGGCTATAATTGGAGCACGACCATGGGTCCCCTGCCACCCTTT
GCTGTGGCCCAGGGCGCCCAGCTCCTGATCCGTCCTGTGGACAAACCAATCAACACAACTTTAATCTGCAACG
TCACCAATGCCCTAGGAGCTCGCCAGGCAGAACTGACCGTCCAGGTCAAAGAGGGACCTCCCAGTGAGCACT
CAGGCATGTCCCGTAACGCCATCATCTTCCTGGTTCTGGGAATCCTGGTTTTTCTGATCCTGCTGGGGATCGGG
ATTTATTTCTATTGGTCCAAATGTTCCCGTGAGGTCCTTTGGCACTGTCATCTGTGTCCCTCGAGTGAGCATCAC
CAGAGCTGCCGTAATTGA
SEQ ID NO. 27 Homo sapiens poliovirus receptor (CD155/PVR), transcript variant 4 (protein) NP_001129242.2 
MARAMAAAWPLLLVALLVLSWPPPGTGDVVVQAPTQVPGFLGDSVTLPCYLQVPNMEVTHVSQLTWARHGES
GSMAVFHQTQGPSYSESKRLEFVAARLGAELRNASLRMFGLRVEDEGNYTCLFVTFPQGSRSVDIWLRVLAKPQN
TAEVQKVQLTGEPVPMARCVSTGGRPPAQITWHSDLGGMPNTSQVPGFLSGTVTVTSLWILVPSSQVDGKNVTC
KVEHESFEKPQLLTVNLTVYYPPEVSISGYDNNWYLGQNEATLTCDARSNPEPTGYNWSTTMGPLPPFAVAQGAQ
LLIRPVDKPINTTLICNVTNALGARQAELTVQVKEGPPSEHSGMSRNAIIFLVLGILVFLILLGIGIYFYWSKCSREVLW
HCHLCPSSEHHQSCRN
SEQ ID NO. 28 Homo sapiens CD226 molecule (CD226), transcript variant 1 (nucleotide) NM_006566 
TAGAACAAAGGAGAAGTGCGTCCTTTCAAATTATAGATTCTTTGGGGGAAAAGAGGGACAGAACTTTATCTG
AGTTTGGAATGAGTCTGAGTAGCTGCAATAGTAAAGTTGCTTCCAGAAGCAGGTAAACTTGACTTCAAAAAAC
CCCGCTTCATGAAATATTAGTGATTCACTTCAGTTGCTATCTGAGGAAGTTCTGGTAGAGAGAAGAGCTCAAG
AGCATGGGCAGAGTCAGCTCCTGAGTGGGCTGAACGCTCCCCTCAGCTCCTGCAGTGCTAATTAAGGGAGGG
AGCAGCGGGGAGCTTGCAGTGACCAAGAGGGTGTTGAGGCTAGGAGGCCACGATAAACAGGATACGATAAA
AGTCCTTAACCAAGACGCAGATGGGAAGAAGCGTTAGAGCGAGCAGCACTCACATCTCAAGAACCAGCCTTT
CAAACAGTTTCCAGAGATGGATTATCCTACTTTACTTTTGGCTCTTCTTCATGTATACAGAGCTCTATGTGAAGA
GGTGCTTTGGCATACATCAGTTCCCTTTGCCGAGAACATGTCTCTAGAATGTGTGTATCCATCAATGGGCATCT
TAACACAGGTGGAGTGGTTCAAGATCGGGACCCAGCAGGATTCCATAGCCATTTTCAGCCCTACTCATGGCAT
GGTCATAAGGAAGCCCTATGCTGAGAGGGTTTACTTTTTGAATTCAACGATGGCTTCCAATAACATGACTCTTT
TCTTTCGGAATGCCTCTGAAGATGATGTTGGCTACTATTCCTGCTCTCTTTACACTTACCCACAGGGAACTTGGC
AGAAGGTGATACAGGTGGTTCAGTCAGATAGTTTTGAGGCAGCTGTGCCATCAAATAGCCACATTGTTTCGGA
 
ACCTGGAAAGAATGTCACACTCACTTGTCAGCCTCAGATGACGTGGCCTGTGCAGGCAGTGAGGTGGGAAAA
GATCCAGCCCCGTCAGATCGACCTCTTAACTTACTGCAACTTGGTCCATGGCAGAAATTTCACCTCCAAGTTCC
CAAGACAAATAGTGAGCAACTGCAGCCACGGAAGGTGGAGCGTCATCGTCATCCCCGATGTCACAGTCTCAG
ACTCGGGGCTTTACCGCTGCTACTTGCAGGCCAGCGCAGGAGAAAACGAAACCTTCGTGATGAGATTGACTG
TAGCCGAGGGTAAAACCGATAACCAATATACCCTCTTTGTGGCTGGAGGGACAGTTTTATTGTTGTTTGTT
ATCTCAATTACCACCATCATTGTCATTTTCCTTAACAGAAGGAGAAGGAGAGAGAAGAGATCTATTTACAG
AGTCCTGGGATACACAGAAGGCACCCAATAACTATAGAAGTCCCATCTCTACCAGTCAACCTACCAATCAATCC
ATGGATGATACAAGAGAGGATATTTATGTCAACTATCCAACCTTCTCTCGCAGACCAAAGACTAGAGTTTAAG
CTTATTCTTGACATGAGTGCATTAGTAATGACTCTTATGTACTCATGCATGGATCTTTATGCAATTTTTTTCCACT
ACCCAAGGTCTACCTTAGATACTAGTTGTCTGAATTGAGTTACTTTGATAGGAAAAATACTTCATTACCTAAAA
TCATTTTTCATAGAACTGTTTCAGAAAACCTGACTCTAACTGGTTTATATACAAAAGAAAACTTACTGTATCATA
TAACAGAATGATCCAGGGGAGATTAAGCTTTGGGCAAGGGCTATTTACCAGGGCTTAAATGTTGTGTCTAGA
ATTAAGTATGGGCATAAACTGGCTTCTGAATCCCTTTCCAGAGTGTTGGATCCATTTCCCTGGTCTTGGCCTCA
CTCTCATGCAGGCTTTCCTCTTGTGTTGGCAAGATGGCTGCCAACTCTTGGCAATTCATACATCCTTGTTTCTGT
CTGGTAGAGAGTTTGCTTCTCAAATGGAGCAAACAAATTTGATTATTTTTCATTGTTAAATAGGCAACATGAC
CAGAAAGGATGGAATGGCTTAAGTAAACTAAGGGTTCACTTCTAGAGCTGAGAAGCAGGGTCAAAGCACAAT
ACTGGGCAATTCAGAGCATGGTTAGAAGAGGAAAGGGGAGTCTCAAAGCTGGAGAGTTTACCAACAAATATT
GACTGCAGTGATTAACCAAGACATTTTTGTTAACTAAAAAGTGAAATATGGGATGGATTCTAGAAATGGGGTA
TCTCTGTCCATACTTCTAGAATCCACTCTATCAGCATAGTCCAGAAGAATACCTGGCAGTAGAAGAAATGAATA
TTCAAGAGGAAGATAAATGCGAGAGGGCAATCCTTTACTATTCTCATATTTATTTATCTCTCATTCTGTATAGAA
TTCTTGCCGCCATCCCAGGTCTAGCCTTAGGAGCAAATGTAGTAGATAGTCGAATAATAAATAACTTAATGTTT
TGGACATATTTTGTCTACTTTTGAGAATTATTTTTAATATGTAAATTCTCTCAAAAGGGTCAGGCACCTAGTTAT
TATTTTTTAATGATTATGTGAAAGTTGAATATAATATACCACTAAAAGTGACAGTTGAAAGTGGTGGCATAGG
ACGGTAGGGTAGAAATTTGGGAGGGAAAAAAGAAATTGGGAGGGTACAGGCAACAGGAGAAAGGAATCAA
ACCACAGAAAAATACAAAGGGAAACTTCTGCTTCACTATTCAGACAAAGACAGCCCTAATGACATCACCAACA
GTCAAAGCAATTAGAGACCATACCTAATATTGTTTAAATTCTAGATGTAGGCTAACAATGAAAAGTATTTGCCA
AACTGAATAAAACTGTCATGGTTACCTTGAAAAAAAAAAAAAAAAAAAAAAAAAAAAAA
SEQ ID NO. 29 Homo sapiens CD226 molecule (CD226), transcript variant 1 (mRNA) NM_006566.3 
ATGGATTATCCTACTTTACTTTTGGCTCTTCTTCATGTATACAGAGCTCTATGTGAAGAGGTGCTTTGGCATACA
TCAGTTCCCTTTGCCGAGAACATGTCTCTAGAATGTGTGTATCCATCAATGGGCATCTTAACACAGGTGGAGTG
GTTCAAGATCGGGACCCAGCAGGATTCCATAGCCATTTTCAGCCCTACTCATGGCATGGTCATAAGGAAGCCC
TATGCTGAGAGGGTTTACTTTTTGAATTCAACGATGGCTTCCAATAACATGACTCTTTTCTTTCGGAATGCCTCT
GAAGATGATGTTGGCTACTATTCCTGCTCTCTTTACACTTACCCACAGGGAACTTGGCAGAAGGTGATACAGG
TGGTTCAGTCAGATAGTTTTGAGGCAGCTGTGCCATCAAATAGCCACATTGTTTCGGAACCTGGAAAGAATGT
CACACTCACTTGTCAGCCTCAGATGACGTGGCCTGTGCAGGCAGTGAGGTGGGAAAAGATCCAGCCCCGTCA
GATCGACCTCTTAACTTACTGCAACTTGGTCCATGGCAGAAATTTCACCTCCAAGTTCCCAAGACAAATAGTGA
GCAACTGCAGCCACGGAAGGTGGAGCGTCATCGTCATCCCCGATGTCACAGTCTCAGACTCGGGGCTTTACCG
CTGCTACTTGCAGGCCAGCGCAGGAGAAAACGAAACCTTCGTGATGAGATTGACTGTAGCCGAGGGTAAAAC
CGATAACCAATATACCCTCTTTGTGGCTGGAGGGACAGTTTTATTGTTGTTGTTTGTTATCTCAATTACCACCAT
CATTGTCATTTTCCTTAACAGAAGGAGAAGGAGAGAGAGAAGAGATCTATTTACAGAGTCCTGGGATACACA
GAAGGCACCCAATAACTATAGAAGTCCCATCTCTACCAGTCAACCTACCAATCAATCCATGGATGATACAAGA
GAGGATATTTATGTCAACTATCCAACCTTCTCTCGCAGACCAAAGACTAGAGTTTAA
SEQ ID NO. 30 Homo sapiens CD226 molecule (CD226), transcript variant 1 (protein) NP_006557.2 SEQ 
MDYPTLLLALLHVYRALCEEVLWHTSVPFAENMSLECVYPSMGILTQVEWFKIGTQQDSIAIFSPTHGMVIRKPYAE
RVYFLNSTMASNNMTLFFRNASEDDVGYYSCSLYTYPQGTWQKVIQVVQSDSFEAAVPSNSHIVSEPGKNVTLTC
QPQMTWPVQAVRWEKIQPRQIDLLTYCNLVHGRNFTSKFPRQIVSNCSHGRWSVIVIPDVTVSDSGLYRCYLQAS
AGENETFVMRLTVAEGKTDNQYTLFVAGGTVLLLLFVISITTIIVIFLNRRRRRERRDLFTESWDTQKAPNNYRSPIST
SQPTNQSMDDTREDIYVNYPTFSRRPKTRV
SEQ ID NO. 31 Homo sapiens CD226 molecule (CD226), transcript variant 2 (nucleotide) NM_001303618 
ATGCATGCACCACCTTTCACTTATTATTTATATTTTACATCTTTATTTAGTTTCTGCCTCCTCCACATAGAATGT
AAGTTCTATCATGGCAATATTTTTTTTTTTTGTATTATTGTATCCTCATTGCCTACAGCTGAGTTAGGCACAGA
GAATGCATGCAGCACATGTTTGTTGAATGAATGACTCCTTGTAGAAAATGAAAGCCCGAAAATAAGCCGACTG
GTTCCCCGAGAGAGAAGAAACTCAAATCTGCCCATCCTCATAAAGGAGCAAACCTGTCTTTTAAGCTAAGGCC
TTTTTCCTGTGTTCATACTTCAGAAAAAGAAGACACGACTGATAACATCTGAGAGGAAGGAAACTGCATTGTG
CTGGGGATCTGCATGTCACATTATAAAAAGCAATTTTTTTTTTTAATAGAACCAGCCTTTCAAACAGTTTCCAGA
GATGGATTATCCTACTTTACTTTTGGCTCTTCTTCATGTATACAGAGCTCTATGTGAAGAGGTGCTTTGGCATAC
ATCAGTTCCCTTTGCCGAGAACATGTCTCTAGAATGTGTGTATCCATCAATGGGCATCTTAACACAGGTGGAGT
GGTTCAAGATCGGGACCCAGCAGGATTCCATAGCCATTTTCAGCCCTACTCATGGCATGGTCATAAGGAAGCC
CTATGCTGAGAGGGTTTACTTTTTGAATTCAACGATGGCTTCCAATAACATGACTCTTTTCTTTCGGAATGCCTC
TGAAGATGATGTTGGCTACTATTCCTGCTCTCTTTACACTTACCCACAGGGAACTTGGCAGAAGGTGATACAG
GTGGTTCAGTCAGATAGTTTTGAGGCAGCTGTGCCATCAAATAGCCACATTGTTTCGGAACCTGGAAAGAATG
TCACACTCACTTGTCAGCCTCAGATGACGTGGCCTGTGCAGGCAGTGAGGTGGGAAAAGATCCAGCCCCGTC
AGATCGACCTCTTAACTTACTGCAACTTGGTCCATGGCAGAAATTTCACCTCCAAGTTCCCAAGACAAATAGTG
AGCAACTGCAGCCACGGAAGGTGGAGCGTCATCGTCATCCCCGATGTCACAGTCTCAGACTCGGGGCTTTACC
GCTGCTACTTGCAGGCCAGCGCAGGAGAAAACGAAACCTTCGTGATGAGATTGACTGTAGCCGAGGGTAAAA
CCGATAACCAATATACCCTCTTTGTGGCTGGAGGGACAGTTTTATTGTTGTTGTTTGTTATCTCAATTACCACCA
TCATTGTCATTTTCCTTAACAGAAGGAGAAGGAGAGAGAGAAGAGATCTATTTACAGAGTCCTGGGATACAC
AGAAGGCACCCAATAACTATAGAAGTCCCATCTCTACCAGTCAACCTACCAATCAATCCATGGATGATACAAG
AGAGGATATTTATGTCAACTATCCAACCTTCTCTCGCAGACCAAAGACTAGAGTTTAAGCTTATTCTTGACATG
AGTGCATTAGTAATGACTCTTATGTACTCATGCATGGATCTTTATGCAATTTTTTTCCACTACCCAAGGTCTACC
TTAGATACTAGTTGTCTGAATTGAGTTACTTTGATAGGAAAAATACTTCATTACCTAAAATCATTTTTCATAGAA
CTGTTTCAGAAAACCTGACTCTAACTGGTTTATATACAAAAGAAAACTTACTGTATCATATAACAGAATGATCC
AGGGGAGATTAAGCTTTGGGCAAGGGCTATTTACCAGGGCTTAAATGTTGTGTCTAGAATTAAGTATGGGCA
TAAACTGGCTTCTGAATCCCTTTCCAGAGTGTTGGATCCATTTCCCTGGTCTTGGCCTCACTCTCATGCAGGCTT
TCCTCTTGTGTTGGCAAGATGGCTGCCAACTCTTGGCAATTCATACATCCTTGTTTCTGTCTGGTAGAGAGTTT
GCTTCTCAAATGGAGCAAACAAATTTGATTATTTTTTCATTGTTAAATAGGCAACATGACCAGAAAGGATGGA
ATGGCTTAAGTAAACTAAGGGTTCACTTCTAGAGCTGAGAAGCAGGGTCAAAGCACAATACTGGGCAATTCA
GAGCATGGTTAGAAGAGGAAAGGGGAGTCTCAAAGCTGGAGAGTTTACCAACAAATATTGACTGCAGTGATT
AACCAAGACATTTTTGTTAACTAAAAAGTGAAATATGGGATGGATTCTAGAAATGGGGTATCTCTGTCCATAC
TTCTAGAATCCACTCTATCAGCATAGTCCAGAAGAATACCTGGCAGTAGAAGAAATGAATATTCAAGAGGAAG
ATAAATGCGAGAGGGCAATCCTTTACTATTCTCATATTTATTTATCTCTCATTCTGTATAGAATTCTTGCCGCCA
TCCCAGGTCTAGCCTTAGGAGCAAATGTAGTAGATAGTCGAATAATAAATAACTTAATGTTTTGGACATATTTT
GTCTACTTTTGAGAATTATTTTTAATATGTAAATTCTCTCAAAAGGGTCAGGCACCTAGTTATTATTTTTTAATG
ATTATGTGAAAGTTGAATATAATATACCACTAAAAGTGACAGTTGAAAGTGGTGGCATAGGACGGTAGGGTA
GAAATTTGGGAGGGAAAAAAGAAATTGGGAGGGTACAGGCAACAGGAGAAAGGAATCAAACCACAGAAAA
ATACAAAGGGAAACTTCTGCTTCACTATTCAGACAAAGACAGCCCTAATGACATCACCAACAGTCAAAGCAAT
TAGAGACCATACCTAATATTGTTTAAATTCTAGATGTAGGCTAACAATGAAAAGTATTTGCCAAACTGAATAAA
ACTGTCATGGTTACCTTGAAAAAAAAAAAAAAAAAAAAAAAAAAAAA
SEQ ID NO. 32 Homo sapiens CD226 molecule (CD226), transcript variant 2 (mRNA) NM_001303618.1 
ATGGATTATCCTACTTTACTTTTGGCTCTTCTTCATGTATACAGAGCTCTATGTGAAGAGGTGCTTTGGCATACA
TCAGTTCCCTTTGCCGAGAACATGTCTCTAGAATGTGTGTATCCATCAATGGGCATCTTAACACAGGTGGAGTG
GTTCAAGATCGGGACCCAGCAGGATTCCATAGCCATTTTCAGCCCTACTCATGGCATGGTCATAAGGAAGCCC
TATGCTGAGAGGGTTTACTTTTTGAATTCAACGATGGCTTCCAATAACATGACTCTTTTCTTTCGGAATGCCTCT
GAAGATGATGTTGGCTACTATTCCTGCTCTCTTTACACTTACCCACAGGGAACTTGGCAGAAGGTGATACAGG
TGGTTCAGTCAGATAGTTTTGAGGCAGCTGTGCCATCAAATAGCCACATTGTTTCGGAACCTGGAAAGAATGT
 
CACACTCACTTGTCAGCCTCAGATGACGTGGCCTGTGCAGGCAGTGAGGTGGAAAAGATCCAGCCCGTCA
GATCGACCTCTTAACTTACTGCAACTTGGTCCATGGCAGAAATTTCACCTCCAAGTTCCCAAGACAAATAGTGA
GCAACTGCAGCCACGGAAGGTGGAGCGTCATCGTCATCCCCGATGTCACAGTCTCAGACTCGGGGCTTTACCG
CTGCTACTTGCAGGCCAGCGCAGGAGAAAACGAAACCTTCGTGATGAGATTGACTGTAGCCGAGGGTAAAAC
CGATAACCAATATACCCTCTTTGTGGCTGGAGGGACAGTTTTATTGTTGTTGTTTGTTATCTCAATTACCACCAT
CATTGTCATTTTCCTTAACAGAAGGAGAAGGAGAGAGAGAAGAGATCTATTTACAGAGTCCTGGGATACACA
GAAGGCACCCAATAACTATAGAAGTCCCATCTCTACCAGTCAACCTACCAATCAATCCATGGATGATACAAGA
GAGGATATTTATGTCAACTATCCAACCTTCTCTCGCAGACCAAAGACTAGAGTTTAA
SEQ ID NO. 33 Homo sapiens CD226 molecule (CD226), transcript variant 2 (protein) NP_001290547.1 
MDYPTLLLALLHVYRALCEEVLWHTSVPFAENMSLECVYPSMGILTQVEWFKIGTQQDSIAIFSPTHGMVIRKPYAE
RVYFLNSTMASNNMTLFFRNASEDDVGYYSCSLYTYPQGTWQKVIQVVQSDSFEAAVPSNSHIVSEPGKNVTLTC
QPQMTWPVQAVRWEKIQPRQIDLLTYCNLVHGRNFTSKFPRQIVSNCSHGRWSVIVIPDVTVSDSGLYRCYLQAS
AGENETFVMRLTVAEGKTDNQYTLFVAGGTVLLLLFVISITTIIVIFLNRRRRRERRDLFTESWDTQKAPNNYRSPIST
SQPTNQSMDDTREDIYVNYPTFSRRPKTRV
SEQ ID NO. 34 Homo sapiens CD226 molecule (CD226), transcript variant 3 (nucleotide) NM_001303619 
TAGAACAAAGGAGAAGTGCGTCCTTTCAAATTATAGATTCTTGGGGGAAAAGAGGGACAGAACTTTATCTG
AGTTTGGAATGAGTCTGAGTAGCTGCAATAGTAAAGTTGCTTCCAGAAGCAGGTAAACTTGACTTCAAAAAAC
CCCGCTTCATGAAATATTAGTGATTCACTTCAGTTGCTATCTGAGGAAGTTCTGGTAGAGAGAAGAGCTCAAG
AGCATGGGCAGAGTCAGCTCCTGAGTGGGCTGAACGCTCCCCTCAGCTCCTGCAGTGCTAATTAAGGGAGGG
AGCAGCGGGGAGCTTGCAGTGACCAAGAGGGTGTTGAGGCTAGGAGGCCACGATAAACAGGATACGATAAA
AGTCCTTAACCAAGACGCAGATGGGAAGAAGCGTTAGAGCGAGCAGCACTCACATCTCAAGATAGTTTTGAG
GCAGCTGTGCCATCAAATAGCCACATTGTTTCGGAACCTGGAAAGAATGTCACACTCACTTGTCAGCCTCAGA
TGACGTGGCCTGTGCAGGCAGTGAGGTGGGAAAAGATCCAGCCCCGTCAGATCGACCTCTTAACTTACTGCA
ACTTGGTCCATGGCAGAAATTTCACCTCCAAGTTCCCAAGACAAATAGTGAGCAACTGCAGCCACGGAAGGTG
GAGCGTCATCGTCATCCCCGATGTCACAGTCTCAGACTCGGGGCTTTACCGCTGCTACTTGCAGGCCAGCGCA
GGAGAAAACGAAACCTTCGTGATGAGATTGACTGTAGCCGAGGGTAAAACCGATAACCAATATACCCTCTTTG
TGGCTGGAGGGACAGTTTTATTGTTGTTTGTTATCTCAATTACCACCATCATTGTCATTTTCCTTAACAGAA
GGAGAAGGAGAGAGAGAAGAGATCTATTTACAGAGTCCTGGGATACACAGAAGGCACCCAATAACTATAGA
AGTCCCATCTCTACCAGTCAACCTACCAATCAATCCATGGATGATACAAGAGAGGATATTTATGTCAACTATCC
AACCTTCTCTCGCAGACCAAAGACTAGAGTTTAAGCTTATTCTTGACATGAGTGCATTAGTAATGACTCTTATG
TACTCATGCATGGATCTTTATGCAATTTTTTTCCACTACCCAAGGTCTACCTTAGATACTAGTTGTCTGAATTGA
GTTACTTTGATAGGAAAAATACTTCATTACCTAAAATCATTTTTCATAGAACTGTTTCAGAAAACCTGACTCTAA
CTGGTTTATATACAAAAGAAAACTTACTGTATCATATAACAGAATGATCCAGGGGAGATTAAGCTTTGGGCAA
GGGCTATTTACCAGGGCTTAAATGTTGTGTCTAGAATTAAGTATGGGCATAAACTGGCTTCTGAATCCCTTTCC
AGAGTGTTGGATCCATTTCCCTGGTCTTGGCCTCACTCTCATGCAGGCTTTCCTCTTGTGTTGGCAAGATGGCT
GCCAACTCTTGGCAATTCATACATCCTTGTTTCTGTCTGGTAGAGAGTTTGCTTCTCAAATGGAGCAAACAAAT
TTGATTATTTTTTCATTGTTAAATAGGCAACATGACCAGAAAGGATGGAATGGCTTAAGTAAACTAAGGGTTC
ACTTCTAGAGCTGAGAAGCAGGGTCAAAGCACAATACTGGGCAATTCAGAGCATGGTTAGAAGAGGAAAGG
GGAGTCTCAAAGCTGGAGAGTTTACCAACAAATATTGACTGCAGTGATTAACCAAGACATTTTTGTTAACTAA
AAAGTGAAATATGGGATGGATTCTAGAAATGGGGTATCTCTGTCCATACTTCTAGAATCCACTCTATCAGCATA
GTCCAGAAGAATACCTGGCAGTAGAAGAAATGAATATTCAAGAGGAAGATAAATGCGAGAGGGCAATCCTTT
ACTATTCTCATATTTATTTATCTCTCATTCTGTATAGAATTCTTGCCGCCATCCCAGGTCTAGCCTTAGGAGCAA
ATGTAGTAGATAGTCGAATAATAAATAACTTAATGTTTTGGACATATTTTGTCTACTTTTGAGAATTATTTTTAA
TATGTAAATTCTCTCAAAAGGGTCAGGCACCTAGTTATTATTTTTTAATGATTATGTGAAAGTTGAATATAATAT
ACCACTAAAAGTGACAGTTGAAAGTGGTGGCATAGGACGGTAGGGTAGAAATTTGGGAGGGAAAAAAGAAA
TTGGGAGGGTACAGGCAACAGGAGAAAGGAATCAAACCACAGAAAAATACAAAGGGAAACTTCTGCTTCACT
ATTCAGACAAAGACAGCCCTAATGACATCACCAACAGTCAAAGCAATTAGAGACCATACCTAATATTGTTTAA
 
ATTCTAGATGTAGGCTAACAATGAAAAGTATTTGCCAAACTGAATAAAACTGTCATGGTTACCTTGAAAAAAA
AAAAAAAAAAAAAAAAAAAAAA
SEQ ID NO. 35 Homo sapiens CD226 molecule (CD226), transcript variant 3 (mRNA) NM_001303619.1 
ATGACGTGGCCTGTGCAGGCAGTGAGGTGGAAAAGATCCAGCCCCGTCAGATCGACCTCTTAACTTACTGC
AACTTGGTCCATGGCAGAAATTTCACCTCCAAGTTCCCAAGACAAATAGTGAGCAACTGCAGCCACGGAAGGT
GGAGCGTCATCGTCATCCCCGATGTCACAGTCTCAGACTCGGGGCTTTACCGCTGCTACTTGCAGGCCAGCGC
AGGAGAAAACGAAACCTTCGTGATGAGATTGACTGTAGCCGAGGGTAAAACCGATAACCAATATACCCTCTTT
GTGGCTGGAGGGACAGTTTTATTGTTGTTGTTTGTTATCTCAATTACCACCATCATTGTCATTTTCCTTAACAGA
AGGAGAAGGAGAGAGAGAAGAGATCTATTTACAGAGTCCTGGGATACACAGAAGGCACCCAATAACTATAG
AAGTCCCATCTCTACCAGTCAACCTACCAATCAATCCATGGATGATACAAGAGAGGATATTTATGTCAACTATC
CAACCTTCTCTCGCAGACCAAAGACTAGAGTTTAA
SEQ ID NO. 36 Homo sapiens CD226 molecule (CD226), transcript variant 3 (protein) NP_001290548.1 
MTWPVQAVRWEKIQPRQIDLLTYCNLVHGRNFTSKFPRQIVSNCSHGRWSVIVIPDVTVSDSGLYRCYLQASAGE
NETFVMRLTVAEGKTDNQYTLFVAGGTVLLLLFVISITTIIVIFLNRRRRRERRDLFTESWDTQKAPNNYRSPISTSQP
TNQSMDDTREDIYVNYPTFSRRPKTRV
SEQ ID NO. 37 MICA, transcript variant 1*001 (nucleotide) NM_000247 
AAGTTTCCGCGGCGCCTTCTCCCCGGCCACTGCTTGAGCCGCTGAGAGGGTGGCGACGTCGGGGCCATGGGG
CTGGGCCCGGTCTTCCTGCTTCTGGCTGGCATCTTCCCTTTTGCACCTCCGGGAGCTGCTGCTGAGCCCCACAG
TCTTCGTTATAACCTCACGGTGCTGTCCTGGGATGGATCTGTGCAGTCAGGGTTTCTCACTGAGGTACATCTGG
ATGGTCAGCCCTTCCTGCGCTGTGACAGGCAGAAATGCAGGGCAAAGCCCCAGGGACAGTGGGCAGAAGAT
GTCCTGGGAAATAAGACATGGGACAGAGAGACCAGAGACTTGACAGGGAACGGAAAGGACCTCAGGATGAC
CCTGGCTCATATCAAGGACCAGAAAGAAGGCTTGCATTCCCTCCAGGAGATTAGGGTCTGTGAGATCCATGAA
GACAACAGCACCAGGAGCTCCCAGCATTTCTACTACGATGGGGAGCTCTTCCTCTCCCAAAACCTGGAGACTA
AGGAATGGACAATGCCCCAGTCCTCCAGAGCTCAGACCTTGGCCATGAACGTCAGGAATTTCTTGAAGGAAG
ATGCCATGAAGACCAAGACACACTATCACGCTATGCATGCAGACTGCCTGCAGGAACTACGGCGATATCTAAA
ATCCGGCGTAGTCCTGAGGAGAACAGTGCCCCCCATGGTGAATGTCACCCGCAGCGAGGCCTCAGAGGGCAA
CATTACCGTGACATGCAGGGCTTCTGGCTTCTATCCCTGGAATATCACACTGAGCTGGCGTCAGGATGGGGTA
TCTTTGAGCCACGACACCCAGCAGTGGGGGGATGTCCTGCCTGATGGGAATGGAACCTACCAGACCTGGGTG
GCCACCAGGATTTGCCAAGGAGAGGAGCAGAGGTTCACCTGCTACATGGAACACAGCGGGAATCACAGCACT
CACCCTGTGCCCTCTGGGAAAGTGCTGGTGCTTCAGAGTCATTGGCAGACATTCCATGTTTCTGCTGTTGCTGC
TGCTGCTATTTTTGTTATTATTATTTCTATGTCCGTTGTTGTAAGAAGAAAACATCAGCTGCAGAGGGTCCAG
AGCTCGTGAGCCTGCAGGTCCTGGATCAACACCCAGTTGGGACGAGTGACCACAGGGATGCCACACAGCTCG
GATTTCAGCCTCTGATGTCAGATCTTGGGTCCACTGGCTCCACTGAGGGCGCCTAGACTCTACAGCCAGGCAG
CTGGGATTCAATTCCCTGCCTGGACTCACGAGCACTTTCCCTCTTGGTGCCTCAGTTTCCTGACCTATGAAACA
GAGAAAATAAAAGCACTTATTTATTGTTGTTGGAGGCTGCAAAATGTTAGTAGATATGAGGCGTTTGCAGCTG
TACCATATTAAAAAAAAAAAAAAAAAA
SEQ ID NO. 38 MICA, transcript variant 1*001 (mRNA) NM_000247.2 
ATGGGGCTGGGCCCGGTCTTCCTGCTTCTGGCTGGCATCTTCCCTTTTGCACCTCCGGGAGCTGCTGAGCC
CCACAGTCTTCGTTATAACCTCACGGTGCTGTCCTGGGATGGATCTGTGCAGTCAGGGTTTCTCACTGAGGTAC
ATCTGGATGGTCAGCCCTTCCTGCGCTGTGACAGGCAGAAATGCAGGGCAAAGCCCCAGGGACAGTGGGCA
GAAGATGTCCTGGGAAATAAGACATGGGACAGAGAGACCAGAGACTTGACAGGGAACGGAAAGGACCTCA
GGATGACCCTGGCTCATATCAAGGACCAGAAAGAAGGCTTGCATTCCCTCCAGGAGATTAGGGTCTGTGAGA
TCCATGAAGACAACAGCACCAGGAGCTCCCAGCATTTCTACTACGATGGGGAGCTCTTCCTCTCCCAAAACCT
GGAGACTAAGGAATGGACAATGCCCCAGTCCTCCAGAGCTCAGACCTTGGCCATGAACGTCAGGAATTTCTTG
AAGGAAGATGCCATGAAGACCAAGACACACTATCACGCTATGCATGCAGACTGCCTGCAGGAACTACGGCGA
 
TATCTAAAATCCGGCGTAGTCCTGAGGAGAACAGTGCCCCCATGGTGAATGTCACCCGCAGCGAGGCCTCA
GAGGGCAACATTACCGTGACATGCAGGGCTTCTGGCTTCTATCCCTGGAATATCACACTGAGCTGGCGTCAGG
ATGGGGTATCTTTGAGCCACGACACCCAGCAGTGGGGGGATGTCCTGCCTGATGGGAATGGAACCTACCAGA
CCTGGGTGGCCACCAGGATTTGCCAAGGAGAGGAGCAGAGGTTCACCTGCTACATGGAACACAGCGGGAAT
CACAGCACTCACCCTGTGCCCTCTGGGAAAGTGCTGGTGCTTCAGAGTCATTGGCAGACATTCCATGTTTCTGC
TGTTGCTGCTGCTGCTATTTTTGTTATTATTATTTTCTATGTCCGTTGTTGTAAGAAGAAAACATCAGCTGCAGA
GGGTCCAGAGCTCGTGAGCCTGCAGGTCCTGGATCAACACCCAGTTGGGACGAGTGACCACAGGGATGCCAC
ACAGCTCGGATTTCAGCCTCTGATGTCAGATCTTGGGTCCACTGGCTCCACTGAGGGCGCCTAG
SEQ ID NO. 39 MICA, transcript variant 1*001 (protein) NP_000238.1 
MGLGPVFLLLAGIFPFAPPGAAAEPHSLRYNLTVLSWDGSVQSGFLTEVHLDGQPFLRCDRQKCRAKPQGQWAE
DVLGNKTWDRETRDLTGNGKDLRMTLAHIKDQKEGLHSLQEIRVCEIHEDNSTRSSQHFYYDGELFLSQNLETKEW
TMPQSSRAQTLAMNVRNFLKEDAMKTKTHYHAMHADCLQELRRYLKSGVVLRRTVPPMVNVTRSEASEGNITVT
CRASGFYPWNITLSWRQDGVSLSHDTQQWGDVLPDGNGTYQTWVATRICQGEEQRFTCYMEHSGNHSTHPVP
SGKVLVLQSHWQTFHVSAVAAAAIFVIIIFYVRCCKKKTSAAEGPELVSLQVLDQHPVGTSDHRDATQLGFQPLMS
DLGSTGSTEGA
SEQ ID NO. 40 MICA, transcript variant 3 (nucleotide) NM_001289152 
GTATCATTTCAGTGAAGGTCACTCCAGTCTTTCATGGAGGCCAAACTAAGGGTGTAAATTAGGATCCTCACTG
AAGTGGCGGGACCCTAAGAGGCTTTTTCCTGGCCCCTTAGTTGTGGGTTTTCCTGCGGGCGGCGCAGCCGGTT
TCCATCAGAACCGCCCAGAGGCGGACGCTGCCTTCCTGGGGTGACGGAGCAGCAGGAAGCGTTTTCGGATCC
TGGAATACGTGGGCGGCCCGTGGGAGGGGCTGAGGCGCAGTTTCCTACTCACCCGGATCCGAATCCTCCGCG
GTGCTGTTTCAAGAGAGCCGGATTCCAGATCACGCTCCAGCCCGGACTCGGAATTCCTGCCCTGCGGGTCTGC
ATTTTCATAACGGGCAGGTGTGAGTGCCCTGCAGCTGGAGACCAGAAGCCTGAAGGCAGCTCGGCCCTCCCC
AGCCCACAGCGCCGTTATTCCGTTTCTATATCAGTAAACACATTTCATTTTCCGTAGACCAGGGCGGGGTGACG
GGTGATCCCAGTCCTCGCAGTGAATTCCGGGCAGCAAAATTCAAAACACATGCGGCCAAGGCCGGGCACGGT
GGTTCACGCCTGTAATCCCAGCACTTTGGGAGGTCGAGGCGGGCGATCACCTGAGGTCGGGAGCTCGAGACC
AACCTGACCAACATGGGGAAATCCCGTCTCTACTAAAAATATAAAATTAGACGGGCTTGGTGGTGAATGCCTG
TAATCCCAGCTAGTCGGGAGGCTGAGGCAGGAGAATCGCTTAAACCTTGGAGGCGGAGGTTGCGGTGAGCC
GAGATCGCGCCATTGCACTTCAGCCTGGGCAACAAGAGGGAAAACTCCGTCGCAAAAACTTTCGGGGGCGGA
GCGGAGCCCCGCCCTGGGTTATGTAAGCGACCGCGCTGGGCCGTTTCTCTTTCTTTTCCGGACCCTGCAGTGG
CGCCTAAAGTCTGAGAGAGGGAAGTCGCCTCTGTGCTCGTGAGTGCATGGGGTATAAGAGCCCCACAGTCTT
CGTTATAACCTCACGGTGCTGTCCTGGGATGGATCTGTGCAGTCAGGGTTTCTTGCTGAGGTACATCTGGATG
GTCAGCCCTTCCTGCGCTATGACAGGCAGAAATGCAGGGCAAAGCCCCAGGGACAGTGGGCAGAAGATGTC
CTGGGAAATAAGACATGGGACAGAGAGACCAGGGACTTGACAGGGAACGGAAAGGACCTCAGGATGACCCT
GGCTCATATCAAGGACCAGAAAGAAGGCTTGCATTCCCTCCAGGAGATTAGGGTCTGTGAGATCCATGAAGA
CAACAGCACCAGGAGCTCCCAGCATTTCTACTACGATGGGGAGCTCTTCCTCTCCCAAAACCTGGAGACTGAG
GAATGGACAGTGCCCCAGTCCTCCAGAGCTCAGACCTTGGCCATGAACGTCAGGAATTTCTTGAAGGAAGAT
GCCATGAAGACCAAGACACACTATCACGCTATGCATGCAGACTGCCTGCAGGAACTACGGCGATATCTAGAAT
CCGGCGTAGTCCTGAGGAGAACAGTGCCCCCCATGGTGAATGTCACCCGCAGCGAGGCCTCAGAGGGCAACA
TCACCGTGACATGCAGGGCTTCCAGCTTCTATCCCCGGAATATCATACTGACCTGGCGTCAGGATGGGGTATC
TTTGAGCCACGACACCCAGCAGTGGGGGGATGTCCTGCCTGATGGGAATGGAACCTACCAGACCTGGGTGGC
CACCAGGATTTGCCGAGGAGAGGAGCAGAGGTTCACCTGCTACATGGAACACAGCGGGAATCACAGCACTCA
CCCTGTGCCCTCTGGGAAAGTGCTGGTGCTTCAGAGTCATTGGCAGACATTCCATGTTTCTGCTGTTGCTGCTG
GCTGCTGCTATTTTTGTTATTATTATTTTCTATGTCCGTTGTTGTAAGAAGAAAACATCAGCTGCAGAGGGTCC
AGAGCTCGTGAGCCTGCAGGTCCTGGATCAACACCCAGTTGGGACGAGTGACCACAGGGATGCCACACAGCT
CGGATTTCAGCCTCTGATGTCAGCTCTTGGGTCCACTGGCTCCACTGAGGGCACCTAGACTCTACAGCCAGGC
GGCTGGAATTGAATTCCCTGCCTGGATCTCACAAGCACTTTCCCTCTTGGTGCCTCAGTTTCCTGACCTATGAA
ACAGAGAAAATAAAAGCACTTATTTATTGTTGTTGGAGGCTGCAAAATGTTAGTAGATATGAGGCATTTGCAG
CTGTGCCATATTAAAAAAAAAAAAAAAAAA
SEQ ID NO. 41 MICA, transcript variant 3 (mRNA) NM_001289152.1 
ATGACCCTGGCTCATATCAAGGACCAGAAAGAAGGCTTGCATTCCCTCCAGGAGATTAGGGTCTGTGAGATCC
ATGAAGACAACAGCACCAGGAGCTCCCAGCATTTCTACTACGATGGGGAGCTCTTCCTCTCCCAAAACCTGGA
GACTGAGGAATGGACAGTGCCCCAGTCCTCCAGAGCTCAGACCTTGGCCATGAACGTCAGGAATTTCTTGAA
GGAAGATGCCATGAAGACCAAGACACACTATCACGCTATGCATGCAGACTGCCTGCAGGAACTACGGCGATA
TCTAGAATCCGGCGTAGTCCTGAGGAGAACAGTGCCCCCCATGGTGAATGTCACCCGCAGCGAGGCCTCAGA
GGGCAACATCACCGTGACATGCAGGGCTTCCAGCTTCTATCCCCGGAATATCATACTGACCTGGCGTCAGGAT
GGGGTATCTTTGAGCCACGACACCCAGCAGTGGGGGGATGTCCTGCCTGATGGGAATGGAACCTACCAGACC
TGGGTGGCCACCAGGATTTGCCGAGGAGAGGAGCAGAGGTTCACCTGCTACATGGAACACAGCGGGAATCA
CAGCACTCACCCTGTGCCCTCTGGGAAAGTGCTGGTGCTTCAGAGTCATTGGCAGACATTCCATGTTTCTGCTG
TTGCTGCTGGCTGCTGCTATTTTTGTTATTATTATTTTCTATGTCCGTTGTTGTAA
SEQ ID NO. 42 MICA, transcript variant 3 (protein) NP_001276081.1 
MTLAHIKDQKEGLHSLQEIRVCEIHEDNSTRSSQHFYYDGELFLSQNLETEEWTVPQSSRAQTLAMNVRNFLKEDA
MKTKTHYHAMHADCLQELRRYLESGVVLRRTVPPMVNVTRSEASEGNITVTCRASSFYPRNIILTWRQDGVSLSH
DTQQWGDVLPDGNGTYQTWVATRICRGEEQRFTCYMEHSGNHSTHPVPSGKVLVLQSHWQTFHVSAVAAGCC
YFCYYYFLCPLL
SEQ ID NO. 43 MICA, transcript variant 2 (nucleotide) NM_001289153 
GTATCATTTCAGTGAAGGTCACTCCAGTCTTTCATGGAGGCCAAACTAAGGGTGTAAATTAGGATCCTCACTG
AAGTGGCGGGACCCTAAGAGGCTTTTTCCTGGCCCCTTAGTTGTGGGTTTTCCTGCGGGCGGCGCAGCCGGTT
TCCATCAGAACCGCCCAGAGGCGGACGCTGCCTTCCTGGGGTGACGGAGCAGCAGGAAGCGTTTTCGGATCC
TGGAATACGTGGGCGGCCCGTGGGAGGGGCTGAGGCGCAGTTTCCTACTCACCCGGATCCGAATCCTCCGCG
GTGCTGTTTCAAGAGAGCCGGATTCCAGATCACGCTCCAGCCCGGACTCGGAATTCCTGCCCTGCGGGTCTGC
ATTTTCATAACGGGCAGGTGTGAGTGCCCTGCAGCTGGAGACCAGAAGCCTGAAGGCAGCTCGGCCCTCCCC
AGCCCACAGCGCCGTTATTCCGTTTCTATATCAGTAAACACATTTCATTTTCCGTAGACCAGGGCGGGGTGACG
GGTGATCCCAGTCCTCGCAGTGAATTCCGGGCAGCAAAATTCAAAACACATGCGGCCAAGGCCGGGCACGGT
GGTTCACGCCTGTAATCCCAGCACTTTGGGAGGTCGAGGCGGGCGATCACCTGAGGTCGGGAGCTCGAGACC
AACCTGACCAACATGGGGAAATCCCGTCTCTACTAAAAATATAAAATTAGACGGGCTTGGTGGTGAATGCCTG
TAATCCCAGCTAGTCGGGAGGCTGAGGCAGGAGAATCGCTTAAACCTTGGAGGCGGAGGTTGCGGTGAGCC
GAGATCGCGCCATTGCACTTCAGCCTGGGCAACAAGAGGGAAAACTCCGTCGCAAAAACTTTCGGGGGCGGA
GCGGAGCCCCGCCCTGGGTTATGTAAGCGACCGCGCTGGGCCGTTTCTCTTTCTTTTCCGGACCCTGCAGTGG
CGCCTAAAGTCTGAGAGAGGGAAGTCGCCTCTGTGCTCAGCCCCACAGTCTTCGTTATAACCTCACGGTGCTG
TCCTGGGATGGATCTGTGCAGTCAGGGTTTCTTGCTGAGGTACATCTGGATGGTCAGCCCTTCCTGCGCTATG
ACAGGCAGAAATGCAGGGCAAAGCCCCAGGGACAGTGGGCAGAAGATGTCCTGGGAAATAAGACATGGGA
CAGAGAGACCAGGGACTTGACAGGGAACGGAAAGGACCTCAGGATGACCCTGGCTCATATCAAGGACCAGA
AAGAAGGCTTGCATTCCCTCCAGGAGATTAGGGTCTGTGAGATCCATGAAGACAACAGCACCAGGAGCTCCC
AGCATTTCTACTACGATGGGGAGCTCTTCCTCTCCCAAAACCTGGAGACTGAGGAATGGACAGTGCCCCAGTC
CTCCAGAGCTCAGACCTTGGCCATGAACGTCAGGAATTTCTTGAAGGAAGATGCCATGAAGACCAAGACACA
CTATCACGCTATGCATGCAGACTGCCTGCAGGAACTACGGCGATATCTAGAATCCGGCGTAGTCCTGAGGAG
AACAGTGCCCCCCATGGTGAATGTCACCCGCAGCGAGGCCTCAGAGGGCAACATCACCGTGACATGCAGGGC
TTCCAGCTTCTATCCCCGGAATATCATACTGACCTGGCGTCAGGATGGGGTATCTTTGAGCCACGACACCCAGC
AGTGGGGGGATGTCCTGCCTGATGGGAATGGAACCTACCAGACCTGGGTGGCCACCAGGATTTGCCGAGGA
GAGGAGCAGAGGTTCACCTGCTACATGGAACACAGCGGGAATCACAGCACTCACCCTGTGCCCTCTGGGAAA
GTGCTGGTGCTTCAGAGTCATTGGCAGACATTCCATGTTTCTGCTGTTGCTGCTGGCTGCTGCTATTTTTGTTAT
TATTATTTTCTATGTCCGTTGTTGTAAGAAGAAAACATCAGCTGCAGAGGGTCCAGAGCTCGTGAGCCTGCAG
GTCCTGGATCAACACCCAGTTGGGACGAGTGACCACAGGGATGCCACACAGCTCGGATTTCAGCCTCTGATGT
CAGCTCTTGGGTCCACTGGCTCCACTGAGGGCACCTAGACTCTACAGCCAGGCGGCTGGAATTGAATTCCCTG
CCTGGATCTCACAAGCACTTTCCCTCTTGGTGCCTCAGTTTCCTGACCTATGAAACAGAGAAAATAAAAGCACT
TATTTATTGTTGTTGGAGGCTGCAAAATGTTAGTAGATATGAGGCATTTGCAGCTGTGCCATATTAAAAAAAA
AAAAAAAAAA
SEQ ID NO. 44 MICA, transcript variant 2 (mRNA) NM_001289153.1 
ATGACCCTGGCTCATATCAAGGACCAGAAAGAAGGCTTGCATTCCCTCCAGGAGATTAGGGTCTGTGAGATCC
ATGAAGACAACAGCACCAGGAGCTCCCAGCATTTCTACTACGATGGGGAGCTCTTCCTCTCCCAAAACCTGGA
GACTGAGGAATGGACAGTGCCCCAGTCCTCCAGAGCTCAGACCTTGGCCATGAACGTCAGGAATTTCTTGAA
GGAAGATGCCATGAAGACCAAGACACACTATCACGCTATGCATGCAGACTGCCTGCAGGAACTACGGCGATA
TCTAGAATCCGGCGTAGTCCTGAGGAGAACAGTGCCCCCCATGGTGAATGTCACCCGCAGCGAGGCCTCAGA
GGGCAACATCACCGTGACATGCAGGGCTTCCAGCTTCTATCCCCGGAATATCATACTGACCTGGCGTCAGGAT
GGGGTATCTTTGAGCCACGACACCCAGCAGTGGGGGGATGTCCTGCCTGATGGGAATGGAACCTACCAGACC
TGGGTGGCCACCAGGATTTGCCGAGGAGAGGAGCAGAGGTTCACCTGCTACATGGAACACAGCGGGAATCA
CAGCACTCACCCTGTGCCCTCTGGGAAAGTGCTGGTGCTTCAGAGTCATTGGCAGACATTCCATGTTTCTGCTG
TTGCTGCTGGCTGCTGCTATTTTTGTTATTATTATTTTCTATGTCCGTTGTTGTAA
SEQ ID NO. 45 MICA, transcript variant 2 (protein) NP_001276082.1 
MTLAHIKDQKEGLHSLQEIRVCEIHEDNSTRSSQHFYYDGELFLSQNLETEEWTVPQSSRAQTLAMNVRNFLKEDA
MKTKTHYHAMHADCLQELRRYLESGVVLRRTVPPMVNVTRSEASEGNITVTCRASSFYPRNIILTWRQDGVSLSH
DTQQWGDVLPDGNGTYQTWVATRICRGEEQRFTCYMEHSGNHSTHPVPSGKVLVLQSHWQTFHVSAVAAGCC
YFCYYYFLCPLL
SEQ ID NO. 46 MICA, transcript variant 4 (nucleotide) NM_001289154 
GTATCATTTCAGTGAAGGTCACTCCAGTCTTTCATGGAGGCCAAACTAAGGGTGTAAATTAGGATCCTCACTG
AAGTGGCGGGACCCTAAGAGGCTTTTTCCTGGCCCCTTAGTTGTGGGTTTTCCTGCGGGCGGCGCAGCCGGTT
TCCATCAGAACCGCCCAGAGGCGGACGCTGCCTTCCTGGGGTGACGGAGCAGCAGGAAGCGTTTTCGGATCC
TGGAATACGTGGGCGGCCCGTGGGAGGGGCTGAGGCGCAGTTTCCTACTCACCCGGATCCGAATCCTCCGCG
GTGCTGTTTCAAGAGAGCCGGATTCCAGATCACGCTCCAGCCCGGACTCGGAATTCCTGCCCTGCGGGTCTGC
ATTTTCATAACGGGCAGGTGTGAGTGCCCTGCAGCTGGAGACCGAAGCCTGAAGGCAGCTCGGCCCTCCCCA
GCCCACAGCGCCGTTATTCCGTTTCTATATCAGTAAACACATTTCATTTTCCGTAGACCAGGGCGGGGTGACGG
GTGATCCCAGTCCTCGCAGTGAATTCCGGGCAGCAAAATTCAAAACACATGCGGCCAAGGCCGGGCACGGTG
GTTCACGCCTGTAATCCCAGCACTTTGGGAGGTCGAGGCGGGCGATCACCTGAGGTCGGGAGCTCGAGACCA
ACCTGACCAACATGGGGAAATCCCGTCTCTACTAAAAATATAAAATTAGACGGGCTTGGTGGTGAATGCCTGT
AATCCCAGCTAGTCGGGAGGCTGAGGCAGGAGAATCGCTTAAACCTTGGAGGCGGAGGTTGCGGTGAGCCG
AGATCGCGCCATTGCACTTCAGCCTGGGCAACAAGAGGGAAAACTCCGTCGCAAAAACTTTCGGGGGCGGAG
CGGAGCCCCGCCCTGGGTTATGTAAGCGACCGCGCTGGGCCGTTTCTCTTTCTTTTCCGGACCCTGCAGTGGC
GCCTAAAGTCTGAGAGAGGGAAGTCGCCTCTGTGCTCAGCCCCACAGTCTTCGTTATAACCTCACGGTGCTGT
CCTGGGATGGATCTGTGCAGTCAGGGTTTCTTGCGAGGTACATCTGGATGGTCAGCCCTTCCTGCGCTATGAC
AGGCAGAAATGCAGGGCAAAGCCCCAGGGACAGTGGGCAGAAGATGTCCTGGGAAATAAGACATGGGACA
GAGAGACCAGGGACTTGACAGGGAACGGAAAGGACCTCAGGATGACCCTGGCTCATATCAAGGACCAGAAA
GAAGGAATTTCTTGAAGGAAGATGCCATGAAGACCAAGACACACTATCACGCTATGCATGCAGACTGCCTGC
AGGAACTACGGCGATATCTAGAATCCGGCGTAGTCCTGAGGAGAACAGTGCCCCCATGGTGAATGTCACCC
GCAGCGAGGCCTCAGAGGGCAACATCACCGTGACATGCAGGGCTTCCAGCTTCTATCCCCGGAATATCATACT
GACCTGGCGTCAGGATGGGGTATCTTTGAGCCACGACACCCAGCAGTGGGGGGATGTCCTGCCTGATGGGAA
TGGAACCTACCAGACCTGGGTGGCCACCAGGATTTGCCGAGGAGAGGAGCAGAGGTTCACCTGCTACATGGA
ACACAGCGGGAATCACAGCACTCACCCTGTGCCCTCTGGGAAAGTGCTGGTGCTTCAGAGTCATTGGCAGAC
ATTCCATGTTTCTGCTGTTGCTGCTGGCTGCTGCTATTTTTGTTATTATTATTTTCTATGTCCGTTGTTGTAAGAA
GAAAACATCAGCTGCAGAGGGTCCAGAGCTCGTGAGCCTGCAGGTCCTGGATCAACACCCAGTTGGGACGAG
TGACCACAGGGATGCCACACAGCTCGGATTTCAGCCTCTGATGTCAGCTCTTGGGTCCACTGGCTCCACTGAG
GGCACCTAGACTCTACAGCCAGGCGGCTGGAATTGAATTCCCTGCCTGGATCTCACAAGCACTTTCCCTCTTGG
TGCCTCAGTTTCCTGACCTATGAAACAGAGAAAATAAAAGCACTTATTTATTGTTGTTGGAGGCTGCAAAATGT
TAGTAGATATGAGGCATTTGCAGCTGTGCCATATTAAAAAAAAAAAAAAAAAA
SEQ ID NO. 47 MICA, transcript variant 4 (mRNA) NM_001289154.1 
ATGGGACAGAGAGACCAGGGACTTGACAGGGAACGGAAAGGACCTCAGGATGACCCTGGCTCATATCAAGG
ACCAGAAAGAAGGAATTTCTTGAAGGAAGATGCCATGAAGACCAAGACACACTATCACGCTATGCATGCAGA
CTGCCTGCAGGAACTACGGCGATATCTAGAATCCGGCGTAGTCCTGAGGAGAACAGTGCCCCCCATGGTGAA
TGTCACCCGCAGCGAGGCCTCAGAGGGCAACATCACCGTGACATGCAGGGCTTCCAGCTTCTATCCCCGGAAT
ATCATACTGACCTGGCGTCAGGATGGGGTATCTTTGAGCCACGACACCCAGCAGTGGGGGGATGTCCTGCCT
GATGGGAATGGAACCTACCAGACCTGGGTGGCCACCAGGATTTGCCGAGGAGAGGAGCAGAGGTTCACCTG
CTACATGGAACACAGCGGGAATCACAGCACTCACCCTGTGCCCTCTGGGAAAGTGCTGGTGCTTCAGAGTCAT
TGGCAGACATTCCATGTTTCTGCTGTTGCTGCTGGCTGCTGCTATTTTTGTTATTATTATTTTCTATGTCCGTTGT
TGTAA
SEQ ID NO. 48 MICA, transcript variant 4 (protein) NP_001276083.1 
MGQRDQGLDRERKGPQDDPGSYQGPERRNFLKEDAMKTKTHYHAMHADCLQELRRYLESGVVLRRTVPPMVN
VTRSEASEGNITVTCRASSFYPRNIILTWRQDGVSLSHDTQQWGDVLPDGNGTYQTWVATRICRGEEQRFTCYME
HSGNHSTHPVPSGKVLVLQSHWQTFHVSAVAAGCCYFCYYYFLCPLL
SEQ ID NO. 49 MICA, transcript variant 1*00801 (nucleotide) NM_001177519 
AAGTTTCCGCGGCGCCTTCTCCCCGGCCACTGCTTGAGCCGCTGAGAGGGTGGCGACGTCGGGGCCATGGGG
CTGGGCCCGGTCTTTCTGCTTCTGGCTGGCATCTTCCCTTTTGCACCTCCGGGAGCTGCTGCTGAGCCCCACAG
TCTTCGTTATAACCTCACGGTGCTGTCCTGGGATGGATCTGTGCAGTCAGGGTTTCTTGCTGAGGTACATCTGG
ATGGTCAGCCCTTCCTGCGCTATGACAGGCAGAAATGCAGGGCAAAGCCCCAGGGACAGTGGGCAGAAGAT
GTCCTGGGAAATAAGACATGGGACAGAGAGACCAGGGACTTGACAGGGAACGGAAAGGACCTCAGGATGA
CCCTGGCTCATATCAAGGACCAGAAAGAAGGCTTGCATTCCCTCCAGGAGATTAGGGTCTGTGAGATCCATGA
AGACAACAGCACCAGGAGCTCCCAGCATTTCTACTACGATGGGGAGCTCTTCCTCTCCCAAAACCTGGAGACT
GAGGAATGGACAGTGCCCCAGTCCTCCAGAGCTCAGACCTTGGCCATGAACGTCAGGAATTTCTTGAAGGAA
GATGCCATGAAGACCAAGACACACTATCACGCTATGCATGCAGACTGCCTGCAGGAACTACGGCGATATCTA
GAATCCGGCGTAGTCCTGAGGAGAACAGTGCCCCCCATGGTGAATGTCACCCGCAGCGAGGCCTCAGAGGGC
AACATCACCGTGACATGCAGGGCTTCCAGCTTCTATCCCCGGAATATCATACTGACCTGGCGTCAGGATGGGG
TATCTTTGAGCCACGACACCCAGCAGTGGGGGGATGTCCTGCCTGATGGGAATGGAACCTACCAGACCTGGG
TGGCCACCAGGATTTGCCGAGGAGAGGAGCAGAGGTTCACCTGCTACATGGAACACAGCGGGAATCACAGC
ACTCACCCTGTGCCCTCTGGGAAAGTGCTGGTGCTTCAGAGTCATTGGCAGACATTCCATGTTTCTGCTGTTGC
TGCTGGCTGCTGCTATTTTTGTTATTATTATTTTCTATGTCCGTTGTTGTAAGAAGAAAACATCAGCTGCAGAG
GGTCCAGAGCTCGTGAGCCTGCAGGTCCTGGATCAACACCCAGTTGGGACGAGTGACCACAGGGATGCCACA
CAGCTCGGATTTCAGCCTCTGATGTCAGCTCTTGGGTCCACTGGCTCCACTGAGGGCACCTAGACTCTACAGCC
AGGCGGCTGGAATTGAATTCCCTGCCTGGATCTCACAAGCACTTTCCCTCTTGGTGCCTCAGTTTCCTGACCTA
TGAAACAGAGAAAATAAAAGCACTTATTTATTGTTGTTGGAGGCTGCAAAATGTTAGTAGATATGAGGCATTT
GCAGCTGTGCCATATTAAAAAAAAAAAAAAAAAA
SEQ ID NO. 50 MICA, transcript variant 1*00801 (mRNA) NM_001177519.2 
ATGGGGCTGGGCCCGGTCTTTCTGCTTCTGGCTGGCATCTTCCCTTTTGCACCTCCGGGAGCTGCTGAGCC
CCACAGTCTTCGTTATAACCTCACGGTGCTGTCCTGGGATGGATCTGTGCAGTCAGGGTTTCTTGCTGAGGTAC
ATCTGGATGGTCAGCCCTTCCTGCGCTATGACAGGCAGAAATGCAGGGCAAAGCCCCAGGGACAGTGGGCA
GAAGATGTCCTGGGAAATAAGACATGGGACAGAGAGACCAGGGACTTGACAGGGAACGGAAAGGACCTCA
GGATGACCCTGGCTCATATCAAGGACCAGAAAGAAGGCTTGCATTCCCTCCAGGAGATTAGGGTCTGTGAGA
TCCATGAAGACAACAGCACCAGGAGCTCCCAGCATTTCTACTACGATGGGGAGCTCTTCCTCTCCCAAAACCT
GGAGACTGAGGAATGGACAGTGCCCCAGTCCTCCAGAGCTCAGACCTTGGCCATGAACGTCAGGAATTTCTT
GAAGGAAGATGCCATGAAGACCAAGACACACTATCACGCTATGCATGCAGACTGCCTGCAGGAACTACGGCG
ATATCTAGAATCCGGCGTAGTCCTGAGGAGAACAGTGCCCCCCATGGTGAATGTCACCCGCAGCGAGGCCTC
AGAGGGCAACATCACCGTGACATGCAGGGCTTCCAGCTTCTATCCCCGGAATATCATACTGACCTGGCGTCAG
GATGGGGTATCTTTGAGCCACGACACCCAGCAGTGGGGGGATGTCCTGCCTGATGGGAATGGAACCTACCAG
ACCTGGGTGGCCACCAGGATTTGCCGAGGAGAGGAGCAGAGGTTCACCTGCTACATGGAACACAGCGGGAA
 
TCACAGCACTCACCCTGTGCCCTCTGGGAAAGTGCTGGTGCTTCAGAGTCATTGGCAGACATTCCATGTTTCTG
CTGTTGCTGCTGGCTGCTGCTATTTTTGTTATTATTATTTCTATGTCCGTTGTTGTAA
SEQ ID NO. 51 MICA, transcript variant 1*00801 (protein) NP_001170990.1 
MGLGPVFLLLAGIFPFAPPGAAAEPHSLRYNLTVLSWDGSVQSGFLAEVHLDGQPFLRYDRQKCRAKPQGQWAE
DVLGNKTWDRETRDLTGNGKDLRMTLAHIKDQKEGLHSLQEIRVCEIHEDNSTRSSQHFYYDGELFLSQNLETEEW
TVPQSSRAQTLAMNVRNFLKEDAMKTKTHYHAMHADCLQELRRYLESGVVLRRTVPPMVNVTRSEASEGNITVT
CRASSFYPRNIILTWRQDGVSLSHDTQQWGDVLPDGNGTYQTWVATRICRGEEQRFTCYMEHSGNHSTHPVPS
GKVLVLQSHWQTFHVSAVAAGCCYFCYYYFLCPLL
SEQ ID NO. 52 MICB, transcript variant 2 (nucleotide) NM_001289160 
GAATTTTGTGAGCGACCGCGCTGGGCCGTTTCTCTTTCTTTTCCGGACCCTGCAGTGGCGCCTAAAGTCTGCGA
GGAGGAAGTCGCCTCTGTGCTCGTGAGTCCAGGGATCTAAGAGCCCCACAGTCTTCGTTACAACCTCATGGTG
CTGTCCCAGGATGGATCTGTGCAGTCAGGGTTTCTCGCTGAGGGACATCTGGATGGTCAGCCCTTCCTGCGCT
ATGACAGGCAGAAACGCAGGGCAAAGCCCCAGGGACAGTGGGCAGAAAATGTCCTGGGAGCTAAGACCTGG
GACACAGAGACCGAGGACTTGACAGAGAATGGGCAAGACCTCAGGAGGACCCTGACTCATATCAAGGACCA
GAAAGGAGGCTTGCATTCCCTCCAGGAGATTAGGGTCTGTGAGATCCATGAAGACAGCAGCACCAGGGGCTC
CCGGCATTTCTACTACGATGGGGAGCTCTTCCTCTCCCAAAACCTGGAGACTCAAGAATCGACAGTGCCCCAG
TCCTCCAGAGCTCAGACCTTGGCTATGAACGTCACAAATTTCTGGAAGGAAGATGCCATGAAGACCAAGACAC
ACTATCGCGCTATGCAGGCAGACTGCCTGCAGAAACTACAGCGATATCTGAAATCCGGGGTGGCCATCAGGA
GAACAGTGCCCCCCATGGTGAATGTCACCTGCAGCGAGGTCTCAGAGGGCAACATCACCGTGACATGCAGGG
CTTCCAGCTTCTATCCCCGGAATATCACACTGACCTGGCGTCAGGATGGGGTATCTTTGAGCCACAACACCCAG
CAGTGGGGGGATGTCCTGCCTGATGGGAATGGAACCTACCAGACCTGGGTGGCCACCAGGATTCGCCAAGG
AGAGGAGCAGAGGTTCACCTGCTACATGGAACACAGCGGGAATCACGGCACTCACCCTGTGCCCTCTGGGAA
GGCGCTGGTGCTTCAGAGTCAACGGACAGACTTTCCATATGTTTCTGCTGCTATGCCATGTTTTGTTATTATTAT
TATTCTCTGTGTCCCTTGTTGCAAGAAGAAAACATCAGCGGCAGAGGGTCCAGAGCTTGTGAGCCTGCAGGTC
CTGGATCAACACCCAGTTGGGACAGGAGACCACAGGGATGCAGCACAGCTGGGATTTCAGCCTCTGATGTCA
GCTACTGGGTCCACTGGTTCCACTGAGGGCACCTAGACTCTACAGCCAGGCGGCCAGGATTCAACTCCCTGCC
TGGATCTCACCAGCACTTTCCCTCTGTTTCCTGACCTATGAAACAGAGAAAATAACATCACTTATTTATTGTTGT
TGGATGCTGCAAAGTGTTAGTAGGTATGAGGTGTTTGCTGCTCTGCCACGTAGAGAGCCAGCAAAGGGATCA
TGACCAACTCAACATTCCATTGGAGGCTATATGATCAAACAGCAAATTGTTTATCATGAATGCAGGATGTGGG
CAAACTCACGACTGCTCCTGCCAACAGAAGGTTTGCTGAGGGCATTCACTCCATGGTGCTCATTGGAGTTATCT
ACTGGGTCATCTAGAGCCTATTGTTTGAGGAATGCAGTCTTACAAGCCTACTCTGGACCCAGCAGCTGACTCCT
TCTTCCACCCCTCTTCTTGCTATCTCCTATACCAATAAATACGAAGGGCTGTGGAAGATCAGAGCCCTTGTTCAC
GAGAAGCAAGAAGCCCCCTGACCCCTTGTTCCAAATATACTCTTTTGTCTTTCTCTTTATTCCCACGTTCGCCCTT
TGTTCAGTCCAATACAGGGTTGTGGGGCCCTTAACAGTGCCATATTAATTGGTATCATTATTTCTGTTGTTTTTG
TTTTTGTTTTTGTTTTTGTTTTTGAGACAGAGTCTCACTCTGTCACCCAGGCTGCAGTTCACTGGTGTGATCTCA
GCTCACTGCAACCTCTGCCTCCCAGGTTCAAGCACTTCTCGTACCTCAGACTCCCGAATAGCTGGGATTACAGA
CAGGCACCACCACACCCAGCTAATTTTTGTATTTTTGTAGAGACGGGGTTTCGCCAAGTTGACCAGCCCAGTT
TCAAACTCCTGACCTCAGGTGATCTGCCTGCCTTGGCATCCCAAAGTGCTGGGATTACAAGAATGAGCCACCG
TGCCTGGCCTATTTTATTATATTGTAATATATTTTATTATATTAGCCACCATGCCTGTCCTATTTTCTTATGTTTTA
ATATATTTTAATATATTACATGTGCAGTAATTAGATTATCATGGGTGAACTTTATGAGTGAGTATCTTGGTGAT
GACTCCTCCTGACCAGCCCAGGACCAGCTTTCTTGTCACCTTGAGGTCCCCTCGCCCCGTCACACCGTTATGCA
TTACTCTGTGTCTACTATTATGTGTGCATAATTTATACCGTAAATGTTTACTCTTTAAATAGAAAAAAAAAAAAA
AA
SEQ ID NO. 53 MICB, transcript variant 2 (mRNA) NM_001289160.1 
ATGGTGCTGTCCCAGGATGGATCTGTGCAGTCAGGGTTTCTCGCTGAGGGACATCTGGATGGTCAGCCCTTCC
TGCGCTATGACAGGCAGAAACGCAGGGCAAAGCCCCAGGGACAGTGGGCAGAAAATGTCCTGGGAGCTAAG
ACCTGGGACACAGAGACCGAGGACTTGACAGAGAATGGGCAAGACCTCAGGAGGACCCTGACTCATATCAA
GGACCAGAAAGGAGGCTTGCATTCCCTCCAGGAGATTAGGGTCTGTGAGATCCATGAAGACAGCAGCACCAG
 
GGGCTCCCGGCATTTCTACTACGATGGGGAGCTCTTCCTCTCCCAAAACCTGGAGACTCAAGAATCGACAGTG
CCCCAGTCCTCCAGAGCTCAGACCTTGGCTATGAACGTCACAAATTTCTGGAAGGAAGATGCCATGAAGACCA
AGACACACTATCGCGCTATGCAGGCAGACTGCCTGCAGAAACTACAGCGATATCTGAAATCCGGGGTGGCCA
TCAGGAGAACAGTGCCCCCCATGGTGAATGTCACCTGCAGCGAGGTCTCAGAGGGCAACATCACCGTGACAT
GCAGGGCTTCCAGCTTCTATCCCCGGAATATCACACTGACCTGGCGTCAGGATGGGGTATCTTTGAGCCACAA
CACCCAGCAGTGGGGGGATGTCCTGCCTGATGGGAATGGAACCTACCAGACCTGGGTGGCCACCAGGATTCG
CCAAGGAGAGGAGCAGAGGTTCACCTGCTACATGGAACACAGCGGGAATCACGGCACTCACCCTGTGCCCTC
TGGGAAGGCGCTGGTGCTTCAGAGTCAACGGACAGACTTTCCATATGTTTCTGCTGCTATGCCATGTTTTGTTA
TTATTATTATTCTCTGTGTCCCTTGTTGCAAGAAGAAAACATCAGCGGCAGAGGGTCCAGAGCTTGTGAGCCT
GCAGGTCCTGGATCAACACCCAGTTGGGACAGGAGACCACAGGGATGCAGCACAGCTGGGATTTCAGCCTCT
GATGTCAGCTACTGGGTCCACTGGTTCCACTGAGGGCACCTAG
SEQ ID NO. 54 MICB, transcript variant 2 (protein) NP_001276089.1 
MVLSQDGSVQSGFLAEGHLDGQPFLRYDRQKRRAKPQGQWAENVLGAKTWDTETEDLTENGQDLRRTLTHIKD
QKGGLHSLQEIRVCEIHEDSSTRGSRHFYYDGELFLSQNLETQESTVPQSSRAQTLAMNVTNFWKEDAMKTKTHY
RAMQADCLQKLQRYLKSGVAIRRTVPPMVNVTCSEVSEGNITVTCRASSFYPRNITLTWRQDGVSLSHNTQQWG
DVLPDGNGTYQTWVATRIRQGEEQRFTCYMEHSGNHGTHPVPSGKALVLQSQRTDFPYVSAAMPCFVIIIILCVPC
CKKKTSAAEGPELVSLQVLDQHPVGTGDHRDAAQLGFQPLMSATGSTGSTEGT
SEQ ID NO. 55 MICB, transcript variant 3 (nucleotide) 
TGGCCCTAAGTTCCGGGCCTCAGTTTTCACTGGATAAGCGGTCGCTGAGCGGGGCGCAGGTGACTAAATTTC
GACGGGGTCTTCTCACGGGTTTCATTCAGTTGGCCACTGCTGAGCAGCTGAGAAGGTGGCGACGTAGGGGCC
ATGGGGCTGGGCCGGGTCCTGCTGTTTCTGGCCGTCGCCTTCCCTTTTGCACCCCCGGCAGCCGCCGCTGAGC
CCCACAGTCTTCGTTACAACCTCATGGTGCTGTCCCAGGATGGATCTGTGCAGTCAGGGTTTCTCGCTGAGGG
ACATCTGGATGGTCAGCCCTTCCTGCGCTATGACAGGCAGAAACGCAGGGCAAAGCCCCAGGGACAGTGGGC
AGAAAATGTCCTGGGAGCTAAGACCTGGGACACAGAGACCGAGGACTTGACAGAGAATGGGCAAGACCTCA
GGAGGACCCTGACTCATATCAAGGACCAGAAAGGAGTGCCCCAGTCCTCCAGAGCTCAGACCTTGGCTATGA
ACGTCACAAATTTCTGGAAGGAAGATGCCATGAAGACCAAGACACACTATCGCGCTATGCAGGCAGACTGCC
TGCAGAAACTACAGCGATATCTGAAATCCGGGGTGGCCATCAGGAGAACAGTGCCCCCCATGGTGAATGTCA
CCTGCAGCGAGGTCTCAGAGGGCAACATCACCGTGACATGCAGGGCTTCCAGCTTCTATCCCCGGAATATCAC
ACTGACCTGGCGTCAGGATGGGGTATCTTTGAGCCACAACACCCAGCAGTGGGGGGATGTCCTGCCTGATGG
GAATGGAACCTACCAGACCTGGGTGGCCACCAGGATTCGCCAAGGAGAGGAGCAGAGGTTCACCTGCTACAT
GGAACACAGCGGGAATCACGGCACTCACCCTGTGCCCTCTGGGAAGGCGCTGGTGCTTCAGAGTCAACGGAC
AGACTTTCCATATGTTTCTGCTGCTATGCCATGTTTTGTTATTATTATTCTCTGTGTCCCTTGTTGCAAGAAG
AAAACATCAGCGGCAGAGGGTCCAGAGCTTGTGAGCCTGCAGGTCCTGGATCAACACCCAGTTGGGACAGG
AGACCACAGGGATGCAGCACAGCTGGGATTTCAGCCTCTGATGTCAGCTACTGGGTCCACTGGTTCCACTGAG
GGCACCTAGACTCTACAGCCAGGCGGCCAGGATTCAACTCCCTGCCTGGATCTCACCAGCACTTTCCCTCTGTT
TCCTGACCTATGAAACAGAGAAAATAACATCACTTATTTATTGTTGTTGGATGCTGCAAAGTGTTAGTAGGTAT
GAGGTGTTTGCTGCTCTGCCACGTAGAGAGCCAGCAAAGGGATCATGACCAACTCAACATTCCATTGGAGGCT
ATATGATCAAACAGCAAATTGTTTATCATGAATGCAGGATGTGGGCAAACTCACGACTGCTCCTGCCAACAGA
AGGTTTGCTGAGGGCATTCACTCCATGGTGCTCATTGGAGTTATCTACTGGGTCATCTAGAGCCTATTGTTTGA
GGAATGCAGTCTTACAAGCCTACTCTGGACCCAGCAGCTGACTCCTTCTTCCACCCCTCTTCTTGCTATCTCCTA
TACCAATAAATACGAAGGGCTGTGGAAGATCAGAGCCCTTGTTCACGAGAAGCAAGAAGCCCCCTGACCCCT
TGTTCCAAATATACTCTTTTGTCTTTCTCTTTATTCCCACGTTCGCCCTTTGTTCAGTCCAATACAGGGTTGTGGG
GCCCTTAACAGTGCCATATTAATTGGTATCATTATTTCTGTTGTTTTTGTTTTTGTTTTTGTTTTTGTTTTTGAGAC
AGAGTCTCACTCTGTCACCCAGGCTGCAGTTCACTGGTGTGATCTCAGCTCACTGCAACCTCTGCCTCCCAGGT
TCAAGCACTTCTCGTACCTCAGACTCCCGAATAGCTGGGATTACAGACAGGCACCACCACACCCAGCTAATTTT
TGTATTTTTTGTAGAGACGGGGTTTCGCCAAGTTGACCAGCCCAGTTTCAAACTCCTGACCTCAGGTGATCTGC
CTGCCTTGGCATCCCAAAGTGCTGGGATTACAAGAATGAGCCACCGTGCCTGGCCTATTTTATTATATTGTAAT
ATATTTTATTATATTAGCCACCATGCCTGTCCTATTTCTTATGTTTTAATATATTTTAATATATTACATGTGCAGT
AATTAGATTATCATGGGTGAACTTTATGAGTGAGTATCTTGGTGATGACTCCTCCTGACCAGCCCAGGACCAG
 
CTTTCTTGTCACCTTGAGGTCCCCTCGCCCCGTCACACCGTTATGCATTACTCTGTGTCTACTATTATGTGTGCAT
AATTTATACCGTAAATGTTTACTCTTTAAATAGAAAAAAAAAAAAAAA
SEQ ID NO. 56 MICB, transcript variant 3 (mRNA) NM_001289161.1 
ATGGGGCTGGGCCGGGTCCTGCTGTTTCTGGCCGTCGCCTTCCCTTTTGCACCCCCGGCAGCCGCCGCTGAGC
CCCACAGTCTTCGTTACAACCTCATGGTGCTGTCCCAGGATGGATCTGTGCAGTCAGGGTTTCTCGCTGAGGG
ACATCTGGATGGTCAGCCCTTCCTGCGCTATGACAGGCAGAAACGCAGGGCAAAGCCCCAGGGACAGTGGGC
AGAAAATGTCCTGGGAGCTAAGACCTGGGACACAGAGACCGAGGACTTGACAGAGAATGGGCAAGACCTCA
GGAGGACCCTGACTCATATCAAGGACCAGAAAGGAGTGCCCCAGTCCTCCAGAGCTCAGACCTTGGCTATGA
ACGTCACAAATTTCTGGAAGGAAGATGCCATGAAGACCAAGACACACTATCGCGCTATGCAGGCAGACTGCC
TGCAGAAACTACAGCGATATCTGAAATCCGGGGTGGCCATCAGGAGAACAGTGCCCCCCATGGTGAATGTCA
CCTGCAGCGAGGTCTCAGAGGGCAACATCACCGTGACATGCAGGGCTTCCAGCTTCTATCCCCGGAATATCAC
ACTGACCTGGCGTCAGGATGGGGTATCTTTGAGCCACAACACCCAGCAGTGGGGGGATGTCCTGCCTGATGG
GAATGGAACCTACCAGACCTGGGTGGCCACCAGGATTCGCCAAGGAGAGGAGCAGAGGTTCACCTGCTACAT
GGAACACAGCGGGAATCACGGCACTCACCCTGTGCCCTCTGGGAAGGCGCTGGTGCTTCAGAGTCAACGGAC
AGACTTTCCATATGTTTCTGCTGCTATGCCATGTTTTGTTATTATTATTATTCTCTGTGTCCCTTGTTGCAAGAAG
AAAACATCAGCGGCAGAGGGTCCAGAGCTTGTGAGCCTGCAGGTCCTGGATCAACACCCAGTTGGGACAGG
AGACCACAGGGATGCAGCACAGCTGGGATTTCAGCCTCTGATGTCAGCTACTGGGTCCACTGGTTCCACTGAG
GGCACCTAG
SEQ ID NO. 57 MICB, transcript variant 3 (protein) NP_001276090.1 
MGLGRVLLFLAVAFPFAPPAAAAEPHSLRYNLMVLSQDGSVQSGFLAEGHLDGQPFLRYDRQKRRAKPQGQWAE
NVLGAKTWDTETEDLTENGQDLRRTLTHIKDQKGVPQSSRAQTLAMNVTNFWKEDAMKTKTHYRAMQADCLQ
KLQRYLKSGVAIRRTVPPMVNVTCSEVSEGNITVTCRASSFYPRNITLTWRQDGVSLSHNTQQWGDVLPDGNGTY
QTWVATRIRQGEEQRFTCYMEHSGNHGTHPVPSGKALVLQSQRTDFPYVSAAMPCFVIIIILCVPCCKKKTSAAEG
PELVSLQVLDQHPVGTGDHRDAAQLGFQPLMSATGSTGSTEGT
SEQ ID NO. 58 MICB, transcript variant 1 (nucleotide) NM_005931 
TGGCCCTAAGTTCCGGGCCTCAGTTTTCACTGGATAAGCGGTCGCTGAGCGGGGCGCAGGTGACTAAATTTC
GACGGGGTCTTCTCACGGGTTTCATTCAGTTGGCCACTGCTGAGCAGCTGAGAAGGTGGCGACGTAGGGGCC
ATGGGGCTGGGCCGGGTCCTGCTGTTTCTGGCCGTCGCCTTCCCTTTTGCACCCCCGGCAGCCGCCGCTGAGC
CCCACAGTCTTCGTTACAACCTCATGGTGCTGTCCCAGGATGGATCTGTGCAGTCAGGGTTTCTCGCTGAGGG
ACATCTGGATGGTCAGCCCTTCCTGCGCTATGACAGGCAGAAACGCAGGGCAAAGCCCCAGGGACAGTGGGC
AGAAAATGTCCTGGGAGCTAAGACCTGGGACACAGAGACCGAGGACTTGACAGAGAATGGGCAAGACCTCA
GGAGGACCCTGACTCATATCAAGGACCAGAAAGGAGGCTTGCATTCCCTCCAGGAGATTAGGGTCTGTGAGA
TCCATGAAGACAGCAGCACCAGGGGCTCCCGGCATTTCTACTACGATGGGGAGCTCTTCCTCTCCCAAAACCT
GGAGACTCAAGAATCGACAGTGCCCCAGTCCTCCAGAGCTCAGACCTTGGCTATGAACGTCACAAATTTCTGG
AAGGAAGATGCCATGAAGACCAAGACACACTATCGCGCTATGCAGGCAGACTGCCTGCAGAAACTACAGCGA
TATCTGAAATCCGGGGTGGCCATCAGGAGAACAGTGCCCCCCATGGTGAATGTCACCTGCAGCGAGGTCTCA
GAGGGCAACATCACCGTGACATGCAGGGCTTCCAGCTTCTATCCCCGGAATATCACACTGACCTGGCGTCAGG
ATGGGGTATCTTTGAGCCACAACACCCAGCAGTGGGGGGATGTCCTGCCTGATGGGAATGGAACCTACCAGA
CCTGGGTGGCCACCAGGATTCGCCAAGGAGAGGAGCAGAGGTTCACCTGCTACATGGAACACAGCGGGAAT
CACGGCACTCACCCTGTGCCCTCTGGGAAGGCGCTGGTGCTTCAGAGTCAACGGACAGACTTTCCATATGTTT
CTGCTGCTATGCCATGTTTTGTTATTATTATTCTCTGTGTCCCTTGTTGCAAGAAGAAAACATCAGCGGCAG
AGGGTCCAGAGCTTGTGAGCCTGCAGGTCCTGGATCAACACCCAGTTGGGACAGGAGACCACAGGGATGCA
GCACAGCTGGGATTTCAGCCTCTGATGTCAGCTACTGGGTCCACTGGTTCCACTGAGGGCACCTAGACTCTAC
AGCCAGGCGGCCAGGATTCAACTCCCTGCCTGGATCTCACCAGCACTTTCCCTCTGTTTCCTGACCTATGAAAC
AGAGAAAATAACATCACTTATTTATTGTTGTTGGATGCTGCAAAGTGTTAGTAGGTATGAGGTGTTTGCTGCTC
TGCCACGTAGAGAGCCAGCAAAGGGATCATGACCAACTCAACATTCCATTGGAGGCTATATGATCAAACAGC
AAATTGTTTATCATGAATGCAGGATGTGGGCAAACTCACGACTGCTCCTGCCAACAGAAGGTTTGCTGAGGGC
ATTCACTCCATGGTGCTCATTGGAGTTATCTACTGGGTCATCTAGAGCCTATTGTTTGAGGAATGCAGTCTTAC
 
AAGCCTACTCTGGACCCAGCAGCTGACTCCTTCTTCCACCCCTCTTCTTGCTATCTCCTATACCAATAAATACGA
AGGGCTGTGGAAGATCAGAGCCCTTGTTCACGAGAAGCAAGAAGCCCCCTGACCCCTTGTTCCAAATATACTC
TTTTGTCTTTCTCTTTATTCCCACGTTCGCCCTTTGTTCAGTCCAATACAGGGTTGTGGGGCCCTTAACAGTGCC
ATATTAATTGGTATCATTATTTCTGTTGTTTTTGTTTTTGTTTTTGTTTTTGTTTTTGAGACAGAGTCTCACTCTGT
CACCCAGGCTGCAGTTCACTGGTGTGATCTCAGCTCACTGCAACCTCTGCCTCCCAGGTTCAAGCACTTCTCGT
ACCTCAGACTCCCGAATAGCTGGGATTACAGACAGGCACCACCACACCCAGCTAATTTTTGTATTTTTTGTAGA
GACGGGGTTTCGCCAAGTTGACCAGCCCAGTTTCAAACTCCTGACCTCAGGTGATCTGCCTGCCTTGGCATCCC
AAAGTGCTGGGATTACAAGAATGAGCCACCGTGCCTGGCCTATTTTATTATATTGTAATATATTTTATTATATTA
GCCACCATGCCTGTCCTATTTTCTTATGTTTTAATATATTTTAATATATTACATGTGCAGTAATTAGATTATCATG
GGTGAACTTTATGAGTGAGTATCTTGGTGATGACTCCTCCTGACCAGCCCAGGACCAGCTTTCTTGTCACCTTG
AGGTCCCCTCGCCCCGTCACACCGTTATGCATTACTCTGTGTCTACTATTATGTGTGCATAATTTATACCGTAAA
TGTTTACTCTTTAAATAGAAAAAAAAAAAAAAA
SEQ ID NO. 59 MICB, transcript variant 1 (mRNA) NM_005931.4 
ATGGGGCTGGGCCGGGTCCTGCTGTTTCTGGCCGTCGCCTTCCCTTTTGCACCCCCGGCAGCCGCCGCTGAGC
CCCACAGTCTTCGTTACAACCTCATGGTGCTGTCCCAGGATGGATCTGTGCAGTCAGGGTTTCTCGCTGAGGG
ACATCTGGATGGTCAGCCCTTCCTGCGCTATGACAGGCAGAAACGCAGGGCAAAGCCCCAGGGACAGTGGGC
AGAAAATGTCCTGGGAGCTAAGACCTGGGACACAGAGACCGAGGACTTGACAGAGAATGGGCAAGACCTCA
GGAGGACCCTGACTCATATCAAGGACCAGAAAGGAGGCTTGCATTCCCTCCAGGAGATTAGGGTCTGTGAGA
TCCATGAAGACAGCAGCACCAGGGGCTCCCGGCATTTCTACTACGATGGGGAGCTCTTCCTCTCCCAAAACCT
GGAGACTCAAGAATCGACAGTGCCCCAGTCCTCCAGAGCTCAGACCTTGGCTATGAACGTCACAAATTTCTGG
AAGGAAGATGCCATGAAGACCAAGACACACTATCGCGCTATGCAGGCAGACTGCCTGCAGAAACTACAGCGA
TATCTGAAATCCGGGGTGGCCATCAGGAGAACAGTGCCCCCCATGGTGAATGTCACCTGCAGCGAGGTCTCA
GAGGGCAACATCACCGTGACATGCAGGGCTTCCAGCTTCTATCCCCGGAATATCACACTGACCTGGCGTCAGG
ATGGGGTATCTTTGAGCCACAACACCCAGCAGTGGGGGGATGTCCTGCCTGATGGGAATGGAACCTACCAGA
CCTGGGTGGCCACCAGGATTCGCCAAGGAGAGGAGCAGAGGTTCACCTGCTACATGGAACACAGCGGGAAT
CACGGCACTCACCCTGTGCCCTCTGGGAAGGCGCTGGTGCTTCAGAGTCAACGGACAGACTTTCCATATGTTT
CTGCTGCTATGCCATGTTTTGTTATTATTATTCTCTGTGTCCCTTGTTGCAAGAAGAAAACATCAGCGGCAG
AGGGTCCAGAGCTTGTGAGCCTGCAGGTCCTGGATCAACACCCAGTTGGGACAGGAGACCACAGGGATGCA
GCACAGCTGGGATTTCAGCCTCTGATGTCAGCTACTGGGTCCACTGGTTCCACTGAGGGCACCTAG
SEQ ID NO. 60 MICB, transcript variant 1 (protein) NP_005922.2 
MGLGRVLLFLAVAFPFAPPAAAAEPHSLRYNLMVLSQDGSVQSGFLAEGHLDGQPFLRYDRQKRRAKPQGQWAE
NVLGAKTWDTETEDLTENGQDLRRTLTHIKDQKGGLHSLQEIRVCEIHEDSSTRGSRHFYYDGELFLSQNLETQEST
VPQSSRAQTLAMNVTNFWKEDAMKTKTHYRAMQADCLQKLQRYLKSGVAIRRTVPPMVNVTCSEVSEGNITVT
CRASSFYPRNITLTWRQDGVSLSHNTQQWGDVLPDGNGTYQTWVATRIRQGEEQRFTCYMEHSGNHGTHPVPS
GKALVLQSQRTDFPYVSAAMPCFVIIIILCVPCCKKKTSAAEGPELVSLQVLDQHPVGTGDHRDAAQLGFQPLMSA
TGSTGSTEGT
SEQ ID NO. 61 Homo sapiens T-cell immunoreceptor with Ig and ITIM domains (TIGIT) (nucleotide) NM_173799 
CGTCCTATCTGCAGTCGGCTACTTTCAGTGGCAGAAGAGGCCACATCTGCTTCCTGTAGGCCCTCTGGGCAGA
AGCATGCGCTGGTGTCTCCTCCTGATCTGGGCCCAGGGGCTGAGGCAGGCTCCCCTCGCCTCAGGAATGATG
ACAGGCACAATAGAAACAACGGGGAACATTTCTGCAGAGAAAGGTGGCTCTATCATCTTACAATGTCACCTCT
CCTCCACCACGGCACAAGTGACCCAGGTCAACTGGGAGCAGCAGGACCAGCTTCTGGCCATTTGTAATGCTGA
CTTGGGGTGGCACATCTCCCCATCCTTCAAGGATCGAGTGGCCCCAGGTCCCGGCCTGGGCCTCACCCTCCAG
TCGCTGACCGTGAACGATACAGGGGAGTACTTCTGCATCTATCACACCTACCCTGATGGGACGTACACTGGGA
GAATCTTCCTGGAGGTCCTAGAAAGCTCAGTGGCTGAGCACGGTGCCAGGTTCCAGATTCCATTGCTTGGAGC
CATGGCCGCGACGCTGGTGGTCATCTGCACAGCAGTCATCGTGGTGGTCGCGTTGACTAGAAAGAAGAAAGC
CCTCAGAATCCATTCTGTGGAAGGTGACCTCAGGAGAAAATCAGCTGGACAGGAGGAATGGAGCCCCAGTGC
TCCCTCACCCCCAGGAAGCTGTGTCCAGGCAGAAGCTGCACCTGCTGGGCTCTGTGGAGAGCAGCGGGGAGA
 
GGACTGTGCCGAGCTGCATGACTACTTCAATGTCCTGAGTTACAGAAGCCTGGGTAACTGCAGCTTCTTCACA
GAGACTGGTTAGCAACCAGAGGCATCTTCTGGAAGATACACTTTTGTCTTTGCTATTATAGATGAATATATAAG
CAGCTGTACTCTCCATCAGTGCTGCGTGTGTGTGTGTGTGTATGTGTGTGTGTTCAGTTGAGTGAATAA
ATGTCATCCTCTTCTCCATCTTCATTTCCTTGGCCTTTTCGTTCTATTCCATTTGCATTATGGCAGGCCTAGGGT
GAGTAACGTGGATCTTGATCATAAATGCAAAATTAAAAAATATCTTGACCTGGTTTTAAATCTGGCAGTTTGAG
CAGATCCTATGTCTCTGAGAGACATTCCTCATAATGGCCAGCATTTTGGGCTACAAGGTTTTGTGGTTGATG
ATGAGGATGGCATGACTGCAGAGCCATCCTCATCTCATTTTTCACGTCATTTTCAGTAACTTTCACTCATTCAA
AGGCAGGTTATAAGTAAGTCCTGGTAGCAGCCTCTATGGGGAGATTTGAGAGTGACTAAATCTTGGTATCTGC
CCTCAAGAACTTACAGTTAAATGGGGAGACAATGTTGTCATGAAAAGGTATTATAGTAAGGAGAGAAGGAGA
CATACACAGGCCTTCAGGAAGAGACGACAGTTTGGGGTGAGGTAGTTGGCATAGGCTTATCTGTGATGAAGT
GGCCTGGGAGCACCAAGGGGATGTTGAGGCTAGTCTGGGAGGAGCAGGAGTTTTGTCTAGGGAACTTGTAG
GAAATTCTTGGAGCTGAAAGTCCCACAAAGAAGGCCCTGGCACCAAGGGAGTCAGCAAACTTCAGATTTTATT
CTCTGGGCAGGCATTTCAAGTTTCCTTTTGCTGTGACATACTCATCCATTAGACAGCCTGATACAGGCCTGTAG
CCTCTTCCGGCCGTGTGTGCTGGGGAAGCCCCAGGAAACGCACATGCCCACACAGGGAGCCAAGTCGTAGCA
TTTGGGCCTTGATCTACCTTTTCTGCATCAATACACTCTTGAGCCTTTGAAAAAAGAACGTTTCCCACTAAAAAG
AAAATGTGGATTTTTAAAATAGGGACTCTTCCTAGGGGAAAAAGGGGGGCTGGGAGTGATAGAGGGTTTAA
AAAATAAACACCTTCAAACTAACTTCTTCGAACCCTTTTATTCACTCCCTGACGACTTTGTGCTGGGGTTGGGGT
AACTGAACCGCTTATTTCTGTTTAATTGCATTCAGGCTGGATCTTAGAAGACTTTTATCCTTCCACCATCTCTCTC
AGAGGAATGAGCGGGGAGGTTGGATTTACTGGTGACTGATTTTCTTTCATGGGCCAAGGAACTGAAAGAGAA
TGTGAAGCAAGGTTGTGTCTTGCGCATGGTTAAAAATAAAGCATTGTCCTGCTTCCTAAGACTTAGACTGGGG
TTGACAATTGTTTTAGCAACAAGACAATTCAACTATTTCTCCTAGGATTTTTATTATTATTATTTTTCACTTTTCT
ACCAAATGGGTTACATAGGAAGAATGAACTGAAATCTGTCCAGAGCTCCAAGTCCTTTGGAAGAAAGATTAG
ATGAACGTAAAAATGTTGTTGTTTGCTGTGGCAGTTTACAGCATTTTTCTTGCAAAATTAGTGCAAATCTGTTG
GAAATAGAACACAATTCACAAATTGGAAGTGAACTAAAATGTAATGACGAAAAGGGAGTAGTGTTTTGATTT
GGAGGAGGTGTATATTCGGCAGAGGTTGGACTGAGAGTTGGGTGTTATTTAACATAATTATGGTAATTGGGA
AACATTTATAAACACTATTGGGATGGTGATAAAATACAAAAGGGCCTATAGATGTTAGAAATGGGTCAGGTTA
CTGAAATGGGATTCAATTTGAAAAAAATTTTTTTAAATAGAACTCACTGAACTAGATTCTCCTCTGAGAACCAG
AGAAGACCATTTCATAGTTGGATTCCTGGAGACATGCGCTATCCACCACGTAGCCACTTTCCACATGTGGCCAT
CAACCACTTAAGATGGGGTTAGTTTAAATCAAGATGTGCTGTTATAATTGGTATAAGCATAAAATCACACTAG
ATTCTGGAGATTTAATATGAATAATAAGAATACTATTTCAGTAGTTTTGGTATATTGTGTGTCAAAAATGATAA
TATTTTGGATGTATTGGGTGAAATAAAATATTAACATTAAAAAAAAAAA
SEQ ID NO. 62 Homo sapiens T-cell immunoreceptor with Ig and ITIM domains (TIGIT) (mRNA) NM_173799.3 
ATGCGCTGGTGTCTCCTCCTGATCTGGGCCCAGGGGCTGAGGCAGGCTCCCCTCGCCTCAGGAATGATGACA
GGCACAATAGAAACAACGGGGAACATTTCTGCAGAGAAAGGTGGCTCTATCATCTTACAATGTCACCTCTCCT
CCACCACGGCACAAGTGACCCAGGTCAACTGGGAGCAGCAGGACCAGCTTCTGGCCATTTGTAATGCTGACTT
GGGGTGGCACATCTCCCCATCCTTCAAGGATCGAGTGGCCCCAGGTCCCGGCCTGGGCCTCACCCTCCAGTCG
CTGACCGTGAACGATACAGGGGAGTACTTCTGCATCTATCACACCTACCCTGATGGGACGTACACTGGGAGAA
TCTTCCTGGAGGTCCTAGAAAGCTCAGTGGCTGAGCACGGTGCCAGGTTCCAGATTCCATTGCTTGGAGCCAT
GGCCGCGACGCTGGTGGTCATCTGCACAGCAGTCATCGTGGTGGTCGCGTTGACTAGAAAGAAGAAAGCCCT
CAGAATCCATTCTGTGGAAGGTGACCTCAGGAGAAAATCAGCTGGACAGGAGGAATGGAGCCCCAGTGCTCC
CTCACCCCCAGGAAGCTGTGTCCAGGCAGAAGCTGCACCTGCTGGGCTCTGTGGAGAGCAGCGGGGAGAGG
ACTGTGCCGAGCTGCATGACTACTTCAATGTCCTGAGTTACAGAAGCCTGGGTAACTGCAGCTTCTTCACAGA
GACTGGTTAG
SEQ ID NO. 63 Homo sapiens T-cell immunoreceptor with Ig and ITIM domains (TIGIT) (protein) NP_776160.2 
MRWCLLLIWAQGLRQAPLASGMMTGTIETTGNISAEKGGSIILQCHLSSTTAQVTQVNWEQQDQLLAICNADLG
WHISPSFKDRVAPGPGLGLTLQSLTVNDTGEYFCIYHTYPDGTYTGRIFLEVLESSVAEHGARFQIPLLGAMAATLVV
 
ICTAVIVVVALTRKKKALRIHSVEGDLRRKSAGQEEWSPSAPSPPGSCVQAEAAPAGLCGEQRGEDCAELHDYFNV
LSYRSLGNCSFFTETG
SEQ ID NO. 64 Homo sapiens killer cell lectin like receptor K1 (KLRK1) (nucleotide) NM_007360 
ACTAAGTATCTCCACTTTCAATTCTAGATCAGGAACTGAGGACATATCTAAATTTTCTAGTTTATAGAAGGCTT
TTATCCACAAGAATCAAGATCTTCCCTCTCTGAGCAGGAATCCTTTGTGCATTGAAGACTTTAGATTCCTCTCTG
CGGTAGACGTGCACTTATAAGTATTTGATGGGGTGGATTCGTGGTCGGAGGTCTCGACACAGCTGGGAGATG
AGTGAATTTCATAATTATAACTTGGATCTGAAGAAGAGTGATTTTCAACACGATGGCAAAAGCAAAGATGTC
CAGTAGTCAAAAGCAAATGTAGAGAAAATGCATCTCCATTTTTTTCTGCTGCTTCATCGCTGTAGCCATGGGA
ATCCGTTTCATTATTATGGTAACAATATGGAGTGCTGTATTCCTAAACTCATTATTCAACCAAGAAGTTCAAATT
CCCTTGACCGAAAGTTACTGTGGCCCATGTCCTAAAAACTGGATATGTTACAAAAATAACTGCTACCAATTTTT
TGATGAGAGTAAAAACTGGTATGAGAGCCAGGCTTCTTGTATGTCTCAAAATGCCAGCCTTCTGAAAGTATAC
AGCAAAGAGGACCAGGATTTACTTAAACTGGTGAAGTCATATCATTGGATGGGACTAGTACACATTCCAACAA
ATGGATCTTGGCAGTGGGAAGATGGCTCCATTCTCTCACCCAACCTACTAACAATAATTGAAATGCAGAAGGG
AGACTGTGCACTCTATGCCTCGAGCTTTAAAGGCTATATAGAAAACTGTTCAACTCCAAATACGTACATCTGCA
TGCAAAGGACTGTGTAAAGATGATCAACCATCTCAATAAAAGCCAGGAACAGAGAAGAGATTACACCAGCGG
TAACACTGCCAACTGAGACTAAAGGAAACAAACAAAAACAGGACAAAATGACCAAAGACTGTCAGATTTCTT
AGACTCCACAGGACCAAACCATAGAACAATTTCACTGCAAACATGCATGATTCTCCAAGACAAAAGAAGAGA
GATCCTAAAGGCAATTCAGATATCCCCAAGGCTGCCTCTCCCACCACAAGCCCAGAGTGGATGGGCTGGGGG
AGGGGTGCTGTTTTAATTTCTAAAGGTAGGACCAACACCCAGGGGATCAGTGAAGGAAGAGAAGGCCAGCA
GATCACTGAGAGTGCAACCCCACCCTCCACAGGAAATTGCCTCATGGGCAGGGCCACAGCAGAGAGACACAG
CATGGGCAGTGCCTTCCCTGCCTGTGGGGGTCATGCTGCCACTTTTAATGGGTCCTCCACCCAACGGGGTCAG
GGAGGTGGTGCTGCCCCAGTGGGCCATGATTATCTTAAAGGCATTATTCTCCAGCCTTAAGTAAGATCTTAGG
ACGTTTCCTTTGCTATGATTTGTACTTGCTTGAGTCCCATGACTGTTTCTCTTCCTCTCTTTCTTCCTTTTGGAATA
GTAATATCCATCCTATGTTTGTCCCACTATTGTATTTTGGAAGCACATAACTTGTTTGGTTTCACAGGTTCACAG
TTAAGAAGGAATTTTGCCTCTGAATAAATAGAATCTTGAGTCTCATGCAAAAAAAAAAAAAAAAAA
SEQ ID NO. 65 Homo sapiens killer cell lectin like receptor K1 (KLRK1) (mRNA) NM_007360.3 
ATGGGGTGGATTCGTGGTCGGAGGTCTCGACACAGCTGGGAGATGAGTGAATTTCATAATTATAACTTGGAT
CTGAAGAAGAGTGATTTTTCAACACGATGGCAAAAGCAAAGATGTCCAGTAGTCAAAAGCAAATGTAGAGAA
AATGCATCTCCATTTTTTTTCTGCTGCTTCATCGCTGTAGCCATGGGAATCCGTTTCATTATTATGGTAACAATA
TGGAGTGCTGTATTCCTAAACTCATTATTCAACCAAGAAGTTCAAATTCCCTTGACCGAAAGTTACTGTGGCCC
ATGTCCTAAAAACTGGATATGTTACAAAAATAACTGCTACCAATTTTTTGATGAGAGTAAAAACTGGTATGAG
AGCCAGGCTTCTTGTATGTCTCAAAATGCCAGCCTTCTGAAAGTATACAGCAAAGAGGACCAGGATTTACTTA
AACTGGTGAAGTCATATCATTGGATGGGACTAGTACACATTCCAACAAATGGATCTTGGCAGTGGGAAGATG
GCTCCATTCTCTCACCCAACCTACTAACAATAATTGAAATGCAGAAGGGAGACTGTGCACTCTATGCCTCGAGC
TTTAAAGGCTATATAGAAAACTGTTCAACTCCAAATACGTACATCTGCATGCAAAGGACTGTGTAA
SEQ ID NO. 66 Homo sapiens killer cell lectin like receptor K1 (KLRK1) (protein) NP_031386.2

MGWIRGRRSRHSWEMSEFHNYNLDLKKSDFSTRWQKQRCPVVKSKCRENASPFFFCCFIAVAMGIRFIIMVTIWS
AVFLNSLFNQEVQIPLTESYCGPCPKNWICYKNNCYQFFDESKNWYESQASCMSQNASLLKVYSKEDQDLLKLVKS
YHWMGLVIPTNGSWQWEDGSILSPNLLTIIEMQKGDCALYASSFKGYIENCSTPNTYICMQRTV

## Claims

1. A method of assessing metastatic potential of a cancer, or poor outcome, in an individual diagnosed with cancer, the method comprising a step of comparing a level of expression of either CD112 or TIGIT, or both, in a biological sample obtained from the individual to a reference level of CD112 or TIGIT, or both, wherein decreased expression levels of either CD112 or TIGIT, or both, when compared to the reference level correlates with increased potential for metastasis of the cancer and/or poor outcome.

2. A method according to Claim 1, further comprising the step of comparing the expression levels of one or more of CD96, CD155, CD226, circulating MICA and circulating MICB in the biological sample from the individual with cancer to a reference level of CD96, CD155, CD226, circulating MICA and circulating MICB, wherein decreased expression of CD96, CD155 or CD226, or increased expression of circulating MICA or circulating MICB, when compared to the reference level correlates with increased potential for metastasis of the cancer and/or poor outcome for the individual with cancer.

3. A method as claimed in any one of the preceding claims in which the biological sample is selected from tumour cells, tumour tissue, conditioned media, blood or blood derivatives, urine, or cerebrospinal fluid.

4. An inhibitor of CD226, circulating MICA or circulating MICB, for use in a method of treatment or prevention of metastatic cancer in a patient, whereby inhibition of CD226 inhibits platelet cloaked cancer cell formation, and inhibition of circulating MICA or circulating MICB increases the ability of NK cells to target cancer cells.

5. An inhibitor of CD226, circulating MICA or circulating MICB, for use of Claim 4, wherein the inhibitor of CD226, circulating MICA or circulating MICB comprises an antibody or antibody fragment having specific binding affinity to a CD226, circulating MICA or circulating MICB peptide.

6. An inhibitor of CD226, circulating MICA or circulating MICB, for use of Claim 4, wherein the inhibitor is a nucleic acid capable of inhibiting or reducing the expression of CD226, circulating MICA or circulating MICB.

7. An inhibitor of CD226, circulating MICA or circulating MICB, for use of Claim 4, wherein the inhibitor is selected from the group consisting of: siRNA; miRNA; ribozyme, anti-sense oligonucleotide.

8. An inhibitor of CD226, circulating MICA or circulating MICB, for use of Claim 4, wherein the cancer is selected from the list consisting of: breast; non-small cell lung cancer (NSCLC); pancreas; ovarian; colon; kidney; head and neck; stomach; prostate; gliomas; carcinoma; sarcoma; leukaemia; lymphoma; and biologically aggressive forms of uterine cancer.

9. An inhibitor of CD226, circulating MICA or circulating MICB, for use of Claim 4, wherein the inhibition of CD226, circulating MICA or circulating MICB, or a combination thereof, is administered in combination with a therapeutic drug.

10. An inhibitor of CD226, circulating MICA or circulating MICB, for use of Claim 4, wherein the inhibitor of CD226, circulating MICA or circulating MICB, or a combination thereof, and the therapeutic drug are administered together or separately.

11. An inhibitor of CD226, circulating MICA or circulating MICB, for use of Claim 4, wherein the therapeutic drug is selected from the group consisting of: Trastuzumab; Lapatinib; Neratinib; Afatinib; Pertuzumab, Anastrozol, Exemestane, Fulvestrant, Goserelin, Letrozole, Leuprolide, Megestrol acetate, Tamoxifen, Toremifene, Palbociclib, Gemcitabine, Ixabepilone, Liposomal doxorubicin, Methotrexate, Paclitaxel, Vinorelbine, Capecitabine, Carboplatin, Cisplatin, Cyclophosphamide and Docetaxel and variants thereof.

12. An inhibitor of CD226, circulating MICA or circulating MICB, or a combination thereof, for use as a medicament.

13. A pharmaceutical composition comprising an inhibitor of CD226, circulating MICA or circulating MICB, or a combination thereof, and a pharmaceutically acceptable carrier.

14. A pharmaceutical composition according to Claim 13 further including a therapeutic drug.

15. A method for the treatment or prevention of a metastatic cancer in a patient, the method comprising a step of comparing a level of expression of either CD112 or TIGIT, or both, in a biological sample obtained from the individual to a reference level of CD 112 or TIGIT, or both, obtained from a biological sample from an individual who does not have a metastatic cancer, wherein decreased expression levels of either CD 112 or TIGIT, or both, when compared to the reference level indicates that the patient is suitable for treatment with a therapeutically effective amount of an inhibitor of CD226, circulating MICA or circulating MICB, or a combination thereof.
